# EUROPEAN PATENT SPECIFICATION

(11) **EP 2 658 835 B1**
(45) Date of publication and mention of the grant of the patent: **17.08.2016**
(21) Application number: 11811629.2
(22) Date of filing: 29.12.2011
(51) Int. Cl.: C07C 2/36, C07C 9/15, C07F 9/46, C07F 11/00, B01J 31/14, B01J 31/18

(54) **OLEFIN OLIGOMERIZATION CATALYSTS AND METHODS OF MAKING AND USING SAME**
OLEFIN-OLIGOMERISIERUNGSKATALYSATOREN SOWIE VERFAHREN ZU IHRER HERSTELLUNG UND VERWENDUNG
CATALYSEURS DE L'OLIGOMÉRISATION D'OLÉFINES ET LEURS PROCÉDÉS DE FABRICATION ET D'UTILISATION

(30) Priority: 29.12.2010 US 980457
(43) Date of publication of application: 06.11.2013
(73) Proprietor: Chevron Phillips Chemical Company LP, The Woodlands, Texas 77380 (US)
(72) Inventor: SYDORA, Orson L, Houston Texas 77004 (US)
(74) Representative: Nash, David Allan
(86) International application number: PCT/US2011/067709
(87) International publication number: WO 2012/092415

(56) References cited:
- US-A1- 2006 020 091
- US-A1- 2007 232 481
- BHASKAR REDDY ALURI ET AL: "Coordination chemistry of new selective ethylene trimerisation ligand Ph2PN(iPr)P(Ph)NH(R) (R = iPr, Et) and tests in catalysis", DALTON TRANSACTIONS, vol. 39, no. 34, 1 January 2010 (2010-01-01), page 7911, XP55023528, ISSN: 1477-9226, DOI: 10.1039/c0dt00440e
- JABRI AMIR ET AL: "Isolation of a Cationic Chromium(II) Species in a Catalytic System for Ethylene Tri- and Tetramerization", ORGANOMETALLICS, ACS, WASHINGTON, DC, US, vol. 25, no. 3, 1 June 2006 (2006-06-01), pages 715-718, XP002457472, ISSN: 0276-7333, DOI: 10.1021/OM050886L
- KUHLMANN ET AL: "N-substituted diphosphinoamines: Toward rational ligand design for the efficient tetramerization of ethylene", JOURNAL OF CATALYSIS, ACADEMIC PRESS, DULUTH, MN, US, vol. 245, no. 2, 18 January 2007 (2007-01-18), pages 279-284, XP005755888, ISSN: 0021-9517, DOI: 10.1016/J.JCAT.2006.10.020

## Description

### FIELD OF THE INVENTION

The present invention relates to metal complexes and catalyst systems for producing olefin oligomers. More particularly, the present invention relates to the improved methods for preparation of olefin oligomerization catalyst systems.

### BACKGROUND OF THE INVENTION

Olefin oligomerization catalyst systems are known in the art, but sometimes lack selectivity to a desired product, have a low product yield, produce unexceptable quantities of polymer, and have a tendency to degrade at elevated temperatures. Enhancements in preparation methods for olefin oligomerization catalyst systems to improve productivity, improve selectivity, decrease polymer production, and improve temperature stability can reduce olefin oligomerization process cost and improve process performance.
US 2006/0020091 A1 describes polymerisation of olefinic componds in the presence of a polymerisation catalyst to produce polymers, including waxes.
US 2007/0232481 A1 describes a catalyst composition for ethylene oligomerization, the catalyst composition including a chromium compound, ligand containing P and N, an activator, and an accelerator.
Kuhlmann et al., "N-substituted diphosphinoamines: Toward rational ligand design for the efficient tetramerization of ethylene" describes diphosphino aminyl ligands with different alkyl and cycloalkyl substituents attached to the N atom of the ligand backbone and testing of these ligands with chromium as ethylene tetramerization catalysts.

### SUMMARY OF THE INVENTION

In an aspect, the present disclosure relates to an olefin oligomerization process comprising: a) contacting i) a metal compound, ii) a diphosphino aminyl ligand, and iii) a metal alkyl comprising an aluminoxane to form a mixture; b) aging the mixture in the substantial absence of an olefin monomer to form an aged mixture for at least 20 minutes; c) contacting the aged mixture with an olefin monomer; and d) forming an olefin oligomer product, wherein "substantial absence of an olefin monomer" means a molar ratio of olefin monomer to the metal compound up to 5:1, and wherein the mixture consists essentially of the metal compound, the diphosphino aminyl ligand, the metal alkyl comprising an aluminoxane and optionally a hydrocarbon solvent. In an embodiment, the mixture is aged at a temperature from 10 °C to 130 °C. In an embodiment, the oligomer product has a polymer amount that is reduced when compared to an otherwise similar process wherein the mixture is aged in the presence of an olefin monomer. In other embodiments, the oligomer product has a polymer amount that is reduced when compared to an otherwise similar process wherein the mixture is not aged before contact with the olefin monomer.

Described herein is a process for reducing an amount of polymer produced in an olefin oligomerization process comprising: a) contacting a metal compound, a diphosphino aminyl ligand metal complex, and a metal alkyl to for a time period to form a mixture; b) aging the mixture in the substantial absence of an olefin monomer for at least 20 minutes to form an aged mixture; c) contacting the aged mixture with an olefin monomer; and d) forming an olefin oligomer product wherein the olefin oligomer product has a reduced polymer formation when compared to an otherwise similar process wherein the mixture is not aged before contact with the olefin monomer.
In another aspect, the present disclosure relates to a process for reducing an amount of polymer produced in an olefin oligomerization process comprising: a) contacting a metal compound, a diphosphino aminyl ligand, and a metal alkyl comprising an aluminoxane for a time period to form a mixture; b) aging the mixture in the substantial absence of an olefin monomer for at least 20 minutes to form an aged mixture; c) contacting the aged mixture with an olefin monomer; and d) forming an olefin oligomer product wherein the olefin oligomer product has a reduced polymer formation when compared to an otherwise similar process wherein the mixture is aged in the presence of the olefin monomer wherein "substantial absence of an olefin monomer" means a molar ratio of olefin monomer to the metal compound up to 5:1; and wherein the mixture consists essentially of the metal compound, the diphosphino aminyl ligand, the metal alkyl comprising an aluminoxane and optionally a hydrocarbon solvent. In an embodiment, the mixture is aged at a temperature from 10 °C to 130 °C.

In another aspect, the present disclosure relates to a process for preparing a catalyst system comprising: a) forming a catalyst system mixture consisting essentially of i) a metal compound, ii) a diphosphino aminyl ligand, iii) a metal alkyl comprising an aluminoxane, and optionally (iv) a hydrocarbon solvent; b) aging the catalyst system mixture for at least 20 minutes in the substantial absence of an olefin monomer, wherein "substantial absence of an olefin monomer" means a molar ratio of olefin monomer to the metal compound up to 5:1. In an embodiment, the mixture is aged at a temperature from 10 °C to 130 °C. In some embodiments, the mixture is aged for at least 20 minutes.

### BRIEF DESCRIPTION OF THE FIGURES

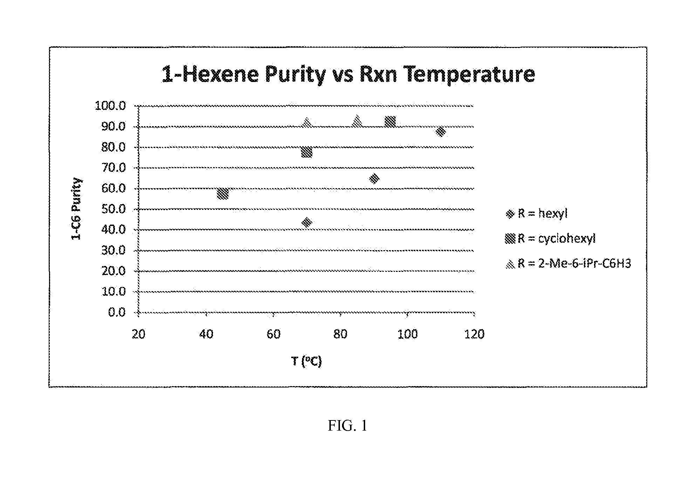

Figure 1 is a graph of oligomer purity versus reaction temperature for oligomerization runs 1, 2, 3, 12, 13, 21, 22, and 23.

### DETAILED DESCRIPTION

Disclosed herein are catalyst systems comprising 1) a metal complex comprising a metal compound complexed to a heteroatomic ligand and 2) a metal alkyl; or alternatively, catalyst systems comprising 1) a metal compound, 2) a heteroatomic ligand, and 3) a metal alkyl. The present disclosure also provides methods for preparing the catalyst systems and methods for oligomerizing olefins using the disclosed catalyst systems and methods of preparing the catalyst systems. Additional details of the metal complex, heteroatomic ligand, metal compound, metal alkyl, catalyst system, catalyst system preparation are disclosed herein.

Further disclosed herein are methods of oligomerizing olefins using the catalyst systems disclosed herein and/or or methods of preparing the catalyst systems disclosed herein. The olefin oligomerization catalyst systems can be used to oligomerize an olefin such as ethylene to 1-hexene, 1-octene, or combinations thereof. The disclosed olefin oligomerization methods can display desirable properties such as enhanced productivity, enhanced selectivity, and/or reduced polymer formation.

Also disclosed herein are methods of preparing catalyst systems comprising a metal complex comprising a metal compound complexed to a diphosphino aminyl ligand; or alternatively, a catalyst system that comprises a metal compound and a ligand comprising a diphosphino aminyl moiety.

Also disclosed herein is an oligomerization process comprising 1) contacting an olefin, a metal complex, and a metal alkyl, and 2) forming an olefin oligomer product; wherein the metal complex comprises a metal compound complexed to a heteroatomic ligand comprising a diphosphino aminyl moiety. Additionally, there is disclosed an oligomerization process comprising 1) contacting an olefin, a metal compound, a heteroatomic ligand comprising a diphosphino aminyl moiety, and a metal alkyl, and 2) forming an olefin oligomer product.

Also disclosed herein is an oligomerization process comprising 1) contacting an olefin, a metal complex, a metal alkyl, and hydrogen, and 2) forming an olefin oligomer product; wherein the metal complex comprises a metal compound complexed to a heteroatomic ligand comprising a diphosphino aminyl moiety. Additionally, there is disclosed an oligomerization process comprising 1) contacting an olefin, a metal compound, a heteroatomic ligand comprising a diphosphino aminyl moiety, a metal alkyl, and hydrogen, and 2) forming an olefin oligomer product.

Also disclosed herein is a process for reducing an amount of polymer produced in an olefin production process comprising 1) contacting an olefin, a diphosphino aminyl ligand metal complex, a metal alkyl, and hydrogen, 2) providing an olefin production process parameter selected from the group consisting of i) a concentration of diphosphino aminyl complexed metal compound greater than or equal to 2 x 10⁻⁵ equivalents/liter, ii) a metal of the metal alkyl to metal of the metal complex molar ratio greater than or equal to 400:1, iii) a diphosphino aminyl moiety to metal compound molar ratio greater than or equal to 2:1, iv) a hydrogen partial pressure ranging from 1 psi (6.9 kPa) to 40 psi (275 kPa), or any combination of i, ii, iii, and iv thereof, and 3) forming an olefin oligomer product.

Also disclosed herein is an olefin oligomerization process comprising: a) preparing a catalyst system; b) allowing the catalyst sytem to age for a period of time; and c) contacting the aged catalyst system with an olefin; and d) forming an olefin oligomer product. Additionally, there is disclosed a process for reducing an amount of polymer produced in an olefin oligomerization process comprising: a) preparing a catalyst system; b) aging the catalyst system to age for a period of time; c) contacting the aged catalyst system with an olefin monomer; and d) forming an olefin oligomer product wherein the oligomer product has a reduced polymer formation when compared to an otherwise similar process wherein the catalyst system in not aged.

To define more clearly the terms used herein, the following definitions are provided. Unless otherwise indicated, the following definitions are applicable to this disclosure. If a term is used in this disclosure but is not specifically defined herein, the definition from the IUPAC Compendium of Chemical Terminology, 2^{nd} Ed (1997) can be applied, as long as that definition does not conflict with any other disclosure or definition applied herein, or render indefinite or non-enabled any claim to which that definition is applied. To the extent that any definition or usage provided by any document cited herein conflicts with the definition or usage provided herein, the definition or usage provided herein controls.

The present disclosure provides for various aspects and/or embodiments. These aspects and/or embodiments can be combined in any manner.

Regarding claim transitional terms or phrases, the transitional term "comprising", which is synonymous with "including," "containing," "having" or "characterized by," is inclusive or open-ended and does not exclude additional, unrecited elements or method steps. The transitional phrase "consisting of" excludes any element, step, or ingredient not specified in the claim. The transitional phrase "consisting essentially of" limits the scope of a claim to the specified materials or steps and those that do not materially affect the basic and novel characteristic(s) of the claimed invention. A "consisting essentially of" claim occupies a middle ground between closed claims that are written in a "consisting of" format and fully open claims that are drafted in a "comprising" format. Absent an indication to the contrary, when describing a compound or composition "consisting essentially of" is not to be construed as "comprising," but is intended to describe the recited component that includes materials which do not significantly alter composition or method to which the term is applied. For example, a feedstock consisting of a material A can include impurities typically present in a commercially produced or commercially available sample of material A. When a claim includes different features and/or feature classes (for example, a method step, feedstock features, and/or product features, among other possibilities), the transitional terms comprising, consisting essentially of, and consisting of apply only to the feature class that is utilized and it is possible to have different transitional terms or phrases utilized with different features within a claim. For example a method can comprises several recited steps (and other non-recited steps) but utilize a catalyst system preparation consisting of specific steps but utilize a catalyst system comprising recited components and other non-recited components.

Within this specification, use of "comprising" or an equivalent expression contemplates the use of the phrase "consisting essentially of," "consists essentially of," or equivalent expressions as alternative embodiments to the open-ended expression. Additionally, use of "comprising" or an equivalent expression or use of "consisting essentially of" in the specification contemplates the use of the phrase "consisting of," "consists of," or equivalent expressions as an alternative to the open-ended expression or middle ground expression, respectively. For example, "comprising" should be understood to include "consisting essentially of," and "consisting of" as alternative embodiments for the aspect, features, and/or elements presented in the specification unless specifically indicated otherwise.

While compositions and methods are described in terms of "comprising" various components or steps, the compositions and methods can also "consist essentially of" or "consist of" the various components or steps.

The terms "a," "an," and "the" are intended, unless specifically indicated otherwise, to include plural alternatives, e.g., at least one. For instance, the disclosure of "a trialkylaluminum compound" is meant to encompass one trialkylaluminum compound, or mixtures or combinations of more than one trialkylaluminum compound unless otherwise specified.

For any particular compound disclosed herein, the general structure or name presented is also intended to encompass all structural isomers, conformational isomers, and stereoisomers that may arise from a particular set of substituents, unless indicated otherwise. Thus, a general reference to a compound includes all structural isomers unless explicitly indicated otherwise; e.g., a general reference to pentane includes n-pentane, 2-methyl-butane, and 2,2-dimethylpropane while a general reference to a butyl group includes an n-butyl group, a sec-butyl group, an iso-butyl group, and a tert-butyl group. Additionally, the reference to a general structure or name encompasses all enantiomers, diastereomers, and other optical isomers whether in enantiomeric or racemic forms, as well as mixtures of stereoisomers, as the context permits or requires. For any particular formula or name that is presented, any general formula or name presented also encompasses all conformational isomers, regioisomers, and stereoisomers that may arise from a particular set of substituents.

Within this disclosure the normal rules of organic nomenclature prevail. For instance, when referencing substituted compounds or groups, references to substitution patterns are taken to indicate that the indicated group(s) is (are) located at the indicated position and that all other non-indicated positions are hydrogen. For example, reference to a 4-substituted phenyl group indicates that there is a non-hydrogen substituent located at the 4 position and hydrogens located at the 2, 3, 5, and 6 positions. By way of another example, reference to a 3-substituted naphth-2-yl indicates that there is a non-hydrogen substituent located at the 3 position and hydrogens located at the 1, 4, 5, 6, 7, and 8 positions. References to compounds or groups having substitution at positions in addition to the indicated position will be referenced using comprising or some other alternative language. For example a reference to a phenyl group comprising a substituent at the 4 position refers to a group having a group at the 4 position and hydrogen or any non-hydrogen group at the 2, 3, 5, and 6 positions.

A chemical "group" is described according to how that group is formally derived from a reference or "parent" compound, for example, by the number of hydrogen atoms formally removed from the parent compound to generate the group, even if that group is not literally synthesized in this manner. These groups can be utilized as substituents or coordinated or bonded to metal atoms. By way of example, an "alkyl group" formally can be derived by removing one hydrogen atom from an alkane, while an "alkylene group" formally can be derived by removing two hydrogen atoms from an alkane. Moreover, a more general term can be used to encompass a variety of groups that formally are derived by removing any number ("one or more") hydrogen atoms from a parent compound, which in this example can be described as an "alkane group," and which encompasses an "alkyl group," an "alkylene group," and materials have three or more hydrogens atoms, as necessary for the situation, removed from the alkane. Throughout, the disclosure that a substituent, ligand, or other chemical moiety may constitute a particular "group" implies that the well-known rules of chemical structure and bonding are followed when that group is employed as described. When describing a group as being "derived by," "derived from," "formed by," or "formed from," such terms are used in a formal sense and are not intended to reflect any specific synthetic methods or procedure, unless specified otherwise or the context requires otherwise.

The term "substituted" when used to describe a group, for example, when referring to a substituted analog of a particular group, is intended to describe any non-hydrogen moiety that formally replaces a hydrogen in that group, and is intended to be non-limiting. A group or group may also be referred to herein as "unsubstituted" or by equivalent terms such as "non-substituted," which refers to the original group in which a non-hydrogen moiety does not replace a hydrogen within that group. "Substituted" is intended to be non-limiting and include inorganic substituents or organic substituents.

The term "organyl group" is used herein in accordance with the definition specified by IUPAC: an organic substituent group, regardless of functional type, having one free valence at a carbon atom. Similarly, an "organylene group" refers to an organic group, regardless of functional type, derived by removing two hydrogen atoms from an organic compound, either two hydrogen atoms from one carbon atom or one hydrogen atom from each of two different carbon atoms. An "organic group" refers to a generalized group formed by removing one or more hydrogen atoms from carbon atoms of an organic compound. Thus, an "organyl group," an "organylene group," and an "organic group" can contain organic functional group(s) and/or atom(s) other than carbon and hydrogen, that is, an organic group that can comprise functional groups and/or atoms in addition to carbon and hydrogen. For instance, non-limiting examples of atoms other than carbon and hydrogen include halogens, oxygen, nitrogen, phosphorus, and the like. Non-limiting examples of functional groups include ethers, aldehydes, ketones, esters, sulfides, amines, and phosphines, and so forth. In one aspect, the hydrogen atom(s) removed to form the "organyl group," "organylene group," or "organic group" can be attached to a carbon atom belonging to a functional group, for example, an acyl group (-C(O)R), a formyl group (-C(O)H), a carboxy group (-C(O)OH), a hydrocarboxycarbonyl group (-C(O)OR), a cyano group (-C=N), a carbamoyl group (-C(O)NH₂), a *N-*hydrocarbylcarbamoyl group (-C(O)NHR), or *N*,*N'*-dihydrocarbylcarbamoyl group (-C(O)NR₂), among other possibilities. In another aspect, the hydrogen atom(s) removed to form the "organyl group," "organylene group," or "organic group" can be attached to a carbon atom not belonging to, and remote from, a functional group, for example, -CH₂C(O)CH₃, -CH₂NR₂, and the like. An "organyl group," "organylene group," or "organic group" can be aliphatic, inclusive of being cyclic or acyclic, or can be aromatic. "Organyl groups," "organylene groups," and "organic groups" also encompass heteroatom-containing rings, heteroatom-containing ring systems, heteroaromatic rings, and heteroaromatic ring systems. "Organyl groups," "organylene groups," and "organic groups" can be linear or branched unless otherwise specified. Finally, it is noted that the "organyl group," "organylene group," or "organic group" definitions include "hydrocarbyl group," "hydrocarbylene group," "hydrocarbon group," respectively, and "alkyl group," "alkylene group," and "alkane group," respectively, as members.

For the purposes of this application, the term or variations of the term "organyl group consisting of inert functional groups" refers to an organyl group wherein the organic functional group(s) and/or atom(s) other than carbon and hydrogen present in the functional group are restricted to those functional group(s) and/or atom(s) other than carbon and hydrogen which do not complex with a metal compound and/or are inert under the process conditions defined herein. Thus, the term or variation of the term "organyl group consisting of inert functional groups" further defines the particular organyl groups that can be present within the organyl group consisting of inert functional groups. Additionally, the term "organyl group consisting of inert functional groups" can refer to the presence of one or more inert functional groups within the organyl group. The term or variation of the term "organyl group consisting of inert functional groups" definition includes the hydrocarbyl group as a member (among other groups). Similarly, an "organylene group consisting of inert functional groups" refers to an organic group formed by removing two hydrogen atoms from one or two carbon atoms of an organic compound consisting of inert functional groups and an "organic group consisting of inert functional groups" refers to a generalized organic group consisting of inert functional groups formed by removing one or more hydrogen atoms from one or more carbon atoms of an organic compound consisting of inert functional groups.

For purposes of this application, an "inert functional group" is a group which does not substantially interfere with the process described herein or in which the material having an inert functional group takes part and/or does not complex with the metal compound of the metal complex. The term "does not complex with the metal compound" can include groups that could complex with a metal compound but in particular molecules described herein cannot complex with a metal compound due to its positional relationship within a ligand. For example, while an ether group can complex with a metal compound, an ether group located at a para position of a substituted phenyl phosphino aminyl group is an inert functional group because a single metal compound cannot complex with both the para ether group and diphosphino aminyl ligand within the same metal complex molecule. Thus, the inertness of a particular functional group is not only related to its functional group's inherent inability to complex the metal compound but can also be related to the functional group's position within the metal complex. Non-limiting examples of inert functional groups which due not substantially interfere with processes described herein can include halo (fluoro, chloro, bromo and/or iodo), nitro, hydrocarboxy groups (e.g, alkoxy, and/or aroxy, among others), sulfidyl groups, and/or hydrocarbyl groups, among others.

The term "hydrocarbon" whenever used in this specification and claims refers to a compound containing only carbon and hydrogen. Other identifiers can be utilized to indicate the presence of particular groups in the hydrocarbon (e.g. halogenated hydrocarbon indicates that the presence of one or more halogen atoms replacing an equivalent number of hydrogen atoms in the hydrocarbon). The term "hydrocarbyl group" is used herein in accordance with the definition specified by IUPAC: a univalent group formed by removing a hydrogen atom from a hydrocarbon. Non-limiting examples of hydrocarbyl groups include ethyl, phenyl, tolyl, propenyl, and the like. Similarly, a "hydrocarbylene group" refers to a group formed by removing two hydrogen atoms from a hydrocarbon, either two hydrogen atoms from one carbon atom or one hydrogen atom from each of two different carbon atoms. Therefore, in accordance with the terminology used herein, a "hydrocarbon group" refers to a generalized group formed by removing one or more hydrogen atoms (as necessary for the particular group) from a hydrocarbon. A "hydrocarbyl group," "hydrocarbylene group," and "hydrocarbon group" can be acyclic or cyclic groups, and/or may be linear or branched. A "hydrocarbyl group," "hydrocarbylene group," and "hydrocarbon group" can include rings, ring systems, aromatic rings, and aromatic ring systems, which contain only carbon and hydrogen. "Hydrocarbyl groups," "hydrocarbylene groups," and "hydrocarbon groups" include, by way of example, aryl, arylene, arene, alkyl, alkylene, alkane, cycloalkyl, cycloalkylene, cycloalkane, aralkyl, aralkylene, and aralkane groups, among other groups, as members.

The term "alkane" whenever used in this specification and claims refers to a saturated hydrocarbon compound. Other identifiers can be utilized to indicate the presence of particular groups in the alkane (e.g. halogenated alkane indicates that the presence of one or more halogen atoms replacing an equivalent number of hydrogen atoms in the alkane). The term "alkyl group" is used herein in accordance with the definition specified by IUPAC: a univalent group formed by removing a hydrogen atom from an alkane. Similarly, an "alkylene group" refers to a group formed by removing two hydrogen atoms from an alkane (either two hydrogen atoms from one carbon atom or one hydrogen atom from two different carbon atoms). An "alkane group" is a general term that refers to a group formed by removing one or more hydrogen atoms (as necessary for the particular group) from an alkane. An "alkyl group," "alkylene group," and "alkane group" can be acyclic or cyclic groups, and/or can be linear or branched unless otherwise specified. Primary, secondary, and tertiary alkyl group are derived by removal of a hydrogen atom from a primary, secondary, tertiary carbon atom, respectively, of an alkane. An n-alkyl group is derived by removal of a hydrogen atom from a terminal carbon atom of a linear alkane. The groups RCH₂ (R ≠ H), R₂CH (R ≠ H), and R₃C (R ≠ H) are primary, secondary, and tertiary alkyl groups, respectively.

A cycloalkane is a saturated cyclic hydrocarbon, with or without side chains (e.g. cyclobutane or methylcyclobutane, among others). Unsaturated cyclic hydrocarbons having one or more endocyclic double or one triple bond are called cycloalkenes and cycloalkynes, respectively. Cycloalkenes and cycloalkynes having only one, only two, only three, etc... endocyclic double or triple bonds, respectively, can be identified by use of the term "mono," "di," "tri," etc ... within the name of the cycloalkene or cycloalkyne. Cycloalkenes and cycloalkynes can further identify the position of the endocyclic double or triple bonds.

A "cycloalkyl group" is a univalent group derived by removing a hydrogen atom from a ring carbon atom of a cycloalkane. For example, a 1-methylcyclopropyl group and a 2-methylcyclopropyl group are illustrated as follows. 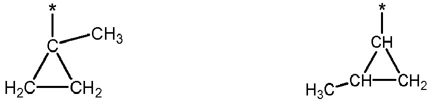 Similarly, a "cycloalkylene group" refers to a group derived by removing two hydrogen atoms from a cycloalkane, at least one of which is a ring carbon. Thus, a "cycloalkylene group" includes both a group derived from a cycloalkane in which two hydrogen atoms are formally removed from the same ring carbon, a group derived from a cycloalkane in which two hydrogen atoms are formally removed from two different ring carbons, and a group derived from a cycloalkane in which a first hydrogen atom is formally removed from a ring carbon and a second hydrogen atom is formally removed from a carbon atom that is not a ring carbon. A "cycloalkane group" refers to a generalized group formed by removing one or more hydrogen atoms (as needed for the particular group and at least one of which is a ring carbon) from a cycloalkane. It should be noted that according the definitions provided herein, general cycloalkane groups (including cycloalkyl groups and cycloalkylene groups) include those having zero, one, or more than one hydrocarbyl substituent groups attached to a cycloalkane ring carbon atom (e.g. a methylcyclopropyl group) and is member of the group of hydrocarbon groups. However, when referring to a cycloalkane group having a specified number of cycloalkane ring carbon atoms (e.g. cyclopentane group or cyclohexane group, among others), the base name of the cycloalkane group having a defined number of cycloalkane ring carbon atoms refers to the unsubstituted cycloalkane group (including having no hydrocarbyl groups located on cycloalkane group ring carbon atom). Consequently, a substituted cycloalkane group having a specified number of ring carbon atoms (e.g. substituted cyclopentane or substituted cyclohexane, among others) refers to the respective group having one or more substituent groups (including halogens, hydrocarbyl groups, or hydrocarboxy groups, among other substituent groups) attached to a cycloalkane group ring carbon atom. When the substituted cycloalkane group having a defined number of cycloalkane ring carbon atoms is a member of the group hydrocarbon groups (or a member of the general group of cycloalkane groups), each substituent of the substituted cycloalkane group having a defined number of cycloalkane ring carbon atoms is limited to hydrocarbyl substituent group. One can readily discern and select general groups, specific groups, and/or individual substituted cycloalkane group(s) having a specific number of ring carbons atoms which can be utilized as member of the hydrocarbon group (or a member of the general group of cycloalkane groups).

The term "olefin" whenever used in this specification and claims refers to compounds that have at least one carbon-carbon double bond that is not part of an aromatic ring or ring system. The term "olefin" includes aliphatic and aromatic, cyclic and cyclic, and/or linear and branched compounds having at least one carbon-carbon double bond that is not part of an aromatic ring or ring system unless specifically stated otherwise. The term "olefin," by itself, does not indicate the presence or absence of heteroatoms and/or the presence or absence of other carbon-carbon double bonds unless explicitly indicated. Olefins having only one, only two, only three, etc... carbon-carbon double bonds can be identified by use of the term "mono," "di," "tri," etc ... within the name of the olefin. The olefins can be further identified by the position of the carbon-carbon double bond(s).

The term "alkene" whenever used in this specification and claims refers a linear or branched hydrocarbon olefin that has one or more carbon-carbon double bonds. Alkenes having only one, only two, only three, etc... such multiple bond can be identified by use of the term "mono," "di," "tri," etc ... within the name. For example, alkamonoenes, alkadienes, and alkatrienes refer to a linear or branched hydrocarbon olefins having only one carbon-carbon double bond (general formula CₙH₂ₙ), only two carbon-carbon double bonds (general formula CₙH₂ₙ₋₂), and only three carbon-carbon double bonds (general formula CₙH₂ₙ₋₄), respectively. Alkenes can be further identified by the position of the carbon-carbon double bond(s). Other identifiers can be utilized to indicate the presence or absence of particular groups within an alkene. For example, a haloalkene refers to an alkene having one or more hydrogen atoms replace with a halogen atom.

An "alkenyl group" is a univalent group derived from an alkene by removal of a hydrogen atom from any carbon atom of the alkene. Thus, "alkenyl group" includes groups in which the hydrogen atom is formally removed from an sp² hybridized (olefinic) carbon atom and groups in which the hydrogen atom is formally removed from any other carbon atom. For example and unless otherwise specified, propen-1-yl (-CH=CHCH₃), propen-2-yl [(CH₃)C=CH₂], and propen-3-yl (-CH₂CH=CH₂) groups are all encompassed with the term "alkenyl group." Similarly, an "alkenylene group" refers to a group formed by formally removing two hydrogen atoms from an alkene, either two hydrogen atoms from one carbon atom or one hydrogen atom from two different carbon atoms. An "alkene group" refers to a generalized group formed by removing one or more hydrogen atoms (as necessary for the particular group) from an alkene. When the hydrogen atom is removed from a carbon atom participating in a carbon-carbon double bond, the regiochemistry of the carbon from which the hydrogen atom is removed, and regiochemistry of the carbon-carbon double bond can both be specified. Alkenyl groups can also have more than one multiple bond. Alkene groups can also be further identified by the position of the carbon-carbon double bond(s).

The term "alpha olefin" as used in this specification and claims refers to an olefin that has a carbon-carbon double bond between the first and second carbon atoms of the longest contiguous chain of carbon atoms. The term "alpha olefin" includes linear and branched alpha olefins unless expressly stated otherwise. In the case of branched alpha olefins, a branch can be at the 2- position (a vinylidene) and/or the 3-position or higher with respect to the olefin double bond. The term "vinylidene" whenever used in this specification and claims refers to an alpha olefin having a branch at the 2-position with respect to the olefin double bond. By itself, the term "alpha olefin" does not indicate the presence or absence of heteroatoms and/or the presence or absence of other carbon-carbon double bonds unless explicitly indicated. The terms "hydrocarbon alpha olefin" or "alpha olefin hydrocarbon" refer to alpha olefin compounds containing only hydrogen and carbon.

The term "linear alpha olefin" as used herein refers to a linear olefin having a carbon-carbon double bond between the first and second carbon atoms. The term "linear alpha olefin" by itself does not indicate the presence or absence of heteroatoms and/or the presence or absence of other carbon-carbon double bonds, unless explicitly indicated. The terms "linear hydrocarbon alpha olefin" or "linear alpha olefin hydrocarbon" refers to linear alpha olefin compounds containing only hydrogen and carbon.

The term "normal alpha olefin" whenever used in this specification and claims refers to a linear hydrocarbon mono-olefin having a carbon-carbon double bond between the first and second carbon atoms. It is noted that "normal alpha olefin" is not synonymous with "linear alpha olefin" as the term "linear alpha olefin" can include linear olefinic compounds having a double bond between the first and second carbon atoms and having heteroatoms and/or additional double bonds.

The term "consists essentially of normal alpha olefin(s)," or variations thereof, whenever used in this specification and claims refers to commercially available normal alpha olefin product(s). The commercially available normal alpha olefin product can contain non-normal alpha olefin impurities such as vinylidenes, internal olefins, branched alpha olefins, paraffins, and diolefins, among other impurities, which are not removed during the normal alpha olefin production process. One readily recognizes that the identity and quantity of the specific impurities present in the commercial normal alpha olefin product will depend upon the source of commercial normal alpha olefin product. Consequently, the term "consists essentially of normal alpha olefins" and its variants is not intended to limit the amount/quantity of the non-linear alpha olefin components any more stringently than the amounts/quantities present in a particular commercial normal alpha olefin product unless explicitly stated.

An aliphatic compound is an acyclic or cyclic, saturated or unsaturated carbon compound, excluding aromatic compounds. Thus, an aliphatic compound is an acyclic or cyclic, saturated or unsaturated carbon compound, excluding aromatic compounds; that is, an aliphatic compound is a non-aromatic organic compound. An "aliphatic group" is a generalized group formed by removing one or more hydrogen atoms (as necessary for the particular group) from carbon atom of an aliphatic compound. Aliphatic compounds and therefore aliphatic groups may contain organic functional group(s) and/or atom(s) other than carbon and hydrogen.

An aromatic compound is a compound containing a cyclically conjugated double bond system that follows the Hückel (4n+2) rule and contains (4n+2) pi-electrons, where n is an integer from 1 to 5. Aromatic compounds include "arenes" (hydrocarbon aromatic compounds) and "heteroarenes," also termed "hetarenes" (heteroaromatic compounds formally derived from arenes by replacement of one or more methine (-C=) carbon atoms of the cyclically conjugated double bond system with a trivalent or divalent heteroatoms, in such a way as to maintain the continuous pi-electron system characteristic of an aromatic system and a number of out-of-plane pi-electrons corresponding to the Hückel rule (4n + 2). While arene compounds and heteroarene compounds are mutually exclusive members of the group of aromatic compounds, a compound that has both an arene group and a heteroarene group are generally considered a heteroarene compound. Aromatic compounds, arenes, and heteroarenes can be monocyclic (e.g., benzene, toluene, furan, pyridine, methylpyridine) or polycyclic unless otherwise specified. Polycyclic aromatic compounds, arenes, and heteroarenes, include, unless otherwise specified, compounds wherein the aromatic rings can be fused (e.g., naphthalene, benzofuran, and indole), compounds where the aromatic groups can be separate and joined by a bond (e.g., biphenyl or 4-phenylpyridine), or compounds where the aromatic groups are joined by a group containing linking atoms (e.g., carbon - the methylene group in diphenylmethane; oxygen - diphenyl ether; nitrogen - triphenyl amine; among other linking groups). As disclosed herein, the term "substituted" can be used to describe an aromatic group, arene, or heteroarene wherein a non-hydrogen moiety formally replaces a hydrogen in the compound, and is intended to be non-limiting.

An "aromatic group" refers to a generalized group formed by removing one or more hydrogen atoms (as necessary for the particular group and at least one of which is an aromatic ring carbon atom) from an aromatic compound. For a univalent "aromatic group," the removed hydrogen atom must be from an aromatic ring carbon. For an "aromatic group" formed by removing more than one hydrogen atom from an aromatic compound, at least one hydrogen atom must be from an aromatic hydrocarbon ring carbon. Additionally, an "aromatic group" may have hydrogen atoms removed from the same ring of an aromatic ring or ring system (e.g., phen-1,4-ylene, pyridin-2,3-ylene, naphth-1,2-ylene, and benzofuran-2,3-ylene), hydrogen atoms removed from two different rings of a ring system (e.g., naphth-1,8-ylene and benzofuran-2,7-ylene), or hydrogen atoms removed from two isolated aromatic rings or ring systems (e.g., bis(phen-4-ylene)methane).

An arene is an aromatic hydrocarbon, with or without side chains (e.g. benzene, toluene, or xylene, among others. An "aryl group" is a group derived from the formal removal of a hydrogen atom from an aromatic hydrocarbon ring carbon atom from an arene compound. One example of an "aryl group" is *ortho-*tolyl (o-tolyl), the structure of which is shown here. 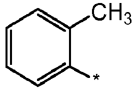 Similarly, an "arylene group" refers to a group formed by removing two hydrogen atoms (at least one of which is from an aromatic hydrocarbon ring carbon) from an arene. An "arene group" refers to a generalized group formed by removing one or more hydrogen atoms (as needed for the particular group and at least one of which is an aromatic hydrocarbon ring carbon) from an arene. However, if a group contains separate and distinct arene and heteroarene rings or ring systems (e.g. the phenyl and benzofuran moieties in 7-phenylbenzofuran) its classification depends upon the particular ring or ring system from which the hydrogen atom was removed, that is, an arene group if the removed hydrogen came from the aromatic hydrocarbon ring or ring system carbon atom (e.g. the 2 carbon atom in the phenyl group of 6-phenylbenzofuran and a heteroarene group if the removed hydrogen carbon came from a heteroaromatic ring or ring system carbon atom (e.g. the 2 or 7 carbon atom of the benzofuran group or 6-phenylbenzofuran). It should be noted that according the definitions provided herein, general arene groups (including an aryl group and an areylene group) include those having zero, one, or more than one hydrocarbyl substituent groups located on an aromatic hydrocarbon ring or ring system carbon atom (e.g a toluene group or a xylene group, among others) and is a member of the group of hydrocarbon groups. However, a phenyl group (or phenylene group) and/or a naphthyl group (or naphthylene group) refer to the specific unsubstituted arene groups (including no hydrocarbyl group located on an aromatic hydrocarbon ring or ring system carbon atom). Consequently, a substituted phenyl group or substituted naphthyl group refers to the respective arene group having one or more substituent groups (including halogens, hydrocarbyl groups, or hydrocarboxy groups, among others) located on an aromatic hydrocarbon ring or ring system carbon atom. When the substituted phenyl group and/or substituted naphthyl group is a member of the group of hydrocarbon groups (or a member of the general group of arene groups), each substituent is limited to a hydrocarbyl substituent group. One having ordinary skill in the art can readily discern and select general phenyl and/or naphthyl groups, specific phenyl and/or naphthyl groups, and/or individual substituted phenyl or substituted naphthyl groups which can be utilized as a member of the group of hydrocarbon groups (or a member of the general group of arene groups).

An "aralkyl group" is an aryl-substituted alkyl group having a free valance at a non-aromatic carbon atom (e.g. a benzyl group, or a 2-phenyleth-1yl group, among others). Similarly, an "aralkylene group" is an aryl-substituted alkylene group having two free valencies at a single non-aromatic carbon atom or a free valence at two non-aromatic carbon atoms while an "aralkane group" is a generalized is an aryl-substituted alkane group having one or more free valencies at a non-aromatic carbon atom(s). A "heteroaralkyl group" is a heteroaryl-substituted alkyl group having a free valence at a non-heteroaromatic ring or ring system carbon atom. Similarly a "heteroaralkylene group" is a heteroaryl-substituted alkylene group having two free valencies at a single non-heteroaromatic ring or ring system carbon atom or a free valence at two non-heteroaromatic ring or ring system carbon atoms while a "heteroaralkane group" is a generalized aryl-substituted alkane group having one or more free valencies at a non-heteroaromatic ring or ring system carbon atom(s). It should be noted that according the definitions provided herein, general aralkane groups include those having zero, one, or more than one hydrocarbyl substituent groups located on an aralkane aromatic hydrocarbon ring or ring system carbon atom and is a member of the group of hydrocarbon groups. However, specific aralkane groups specifying a particular aryl group (e.g. the phenyl group in a benzyl group or a 2-phenylethyl group, among others) refer to the specific unsubstituted aralkane groups (including no hydrocarbyl group located on the aralkane aromatic hydrocarbon ring or ring system carbon atom). Consequently, a substituted aralkane group specifying a particular aryl group refers to a respective aralkane group having one or more substituent groups (including halogens, hydrocarbyl groups, or hydrocarboxy groups, among others). When the substituted aralkane group specifying a particular aryl group is a member of the group of hydrocarbon groups (or a member of the general group of aralkane groups), each substituent is limited to a hydrocarbyl substituent group. One can readily discern and select substituted aralkane groups specifying a particular aryl group which can be utilized as a member of the group of hydrocarbon groups (or a member of the general group of aralkane groups).

A "heterocyclic compound" is a cyclic compound having at least two different elements as ring member atoms. For example, heterocyclic compounds may comprise rings containing carbon and nitrogen (for example, tetrahydropyrrole), carbon and oxygen (for example, tetrahydrofuran), or carbon and sulfur (for example, tetrahydrothiophene), among others. Heterocyclic compounds and heterocyclic groups can be either aliphatic or aromatic.

A "heterocyclyl group" is a univalent group formed by removing a hydrogen atom from a heterocyclic ring or ring system carbon atom of a heterocyclic compound. By specifying that the hydrogen atom is removed from a heterocyclic ring or ring system carbon atom, a "heterocyclyl group" is distinguished from a "cycloheteryl group," in which a hydrogen atom is removed from a heterocyclic ring or ring system heteroatom. For example, a pyrrolidin-2-yl group illustrated below is one example of a "heterocyclyl group," and a pyrrolidin-1-yl group illustrated below is one example of a "cycloheteryl" group." 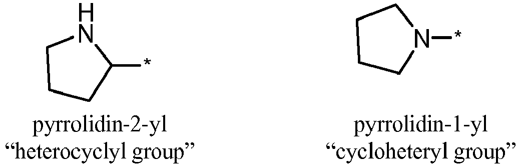 Similarly, a "heterocyclylene group" or more simply, a "heterocyclene group," refers to a group formed by removing two hydrogen atoms from a heterocyclic compound, at least one of which is from a heterocyclic ring or ring system carbon. Thus, in a "heterocyclylene group," at least one hydrogen is removed from a heterocyclic ring or ring system carbon atom, and the other hydrogen atom can be removed from any other carbon atom, including for example, the same heterocyclic ring or ring system carbon atom, a different heterocyclic ring or ring system ring carbon atom, or a non-ring carbon atom. A "heterocyclic group" refers to a generalized group formed by removing one or more hydrogen atoms (as necessary for the particular group and at least one of which is a heterocyclic ring carbon atom) from a heterocyclic compound. Generally, a heterocyclic compound may be aliphatic or aromatic unless otherwise specified.

A "cycloheteryl group" is a univalent group formed by removing a hydrogen atom from a heterocyclic ring or ring system heteroatom of a heterocyclic compound, as illustrated. By specifying that the hydrogen atom is removed from a heterocyclic ring or ring system heteroatom and not from a ring carbon atom, a "cycloheteryl group" is distinguished from a "heterocyclyl group" in which a hydrogen atom is removed from a heterocyclic ring or ring system carbon atom. Similarly, a "cycloheterylene group" refers to a group formed by removing two hydrogen atoms from an heterocyclic compound, at least one of which is removed from a heterocyclic ring or ring system heteroatom of the heterocyclic compound; the other hydrogen atom can be removed from any other atom, including for example, a heterocyclic ring or ring system ring carbon atom, another heterocyclic ring or ring system heteroatom, or a non-ring atom (carbon or heteroatom). A "cyclohetero group" refers to a generalized group formed by removing one or more hydrogen atoms (as necessary for the particular group and at least one of which is from a heterocyclic ring or ring system heteroatom) from a heterocyclic compound.

A "heteroaryl group" is a class of "heterocyclyl group" and is a univalent group formed by removing a hydrogen atom from a heteroaromatic ring or ring system carbon atom of a heteroarene compound. By specifying that the hydrogen atom is removed from a ring carbon atom, a "heteroaryl group" is distinguished from an "arylheteryl group," in which a hydrogen atom is removed from a heteroaromatic ring or ring system heteroatom. For example, an indol-2-yl group illustrated below is one example of a "heteroaryl group," and an indol-1-yl group illustrated below is one example of an "arylheteryl" group." 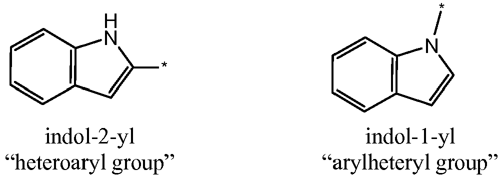 Similarly, a "heteroarylene group" refers to a group formed by removing two hydrogen atoms from a heteroarene compound, at least one of which is from a heteroarene ring or ring system carbon atom. Thus, in a "heteroarylene group," at least one hydrogen is removed from a heteroarene ring or ring system carbon atom, and the other hydrogen atom can be removed from any other carbon atom, including for example, a heteroarene ring or ring system carbon atom, or a non-heteroarene ring or ring system atom. A "heteroarene group" refers to a generalized group formed by removing one or more hydrogen atoms (as necessary for the particular group and at least one of which is a heteroarene ring or ring system carbon atom) from a heteroarene compound. If a hydrogen atom is removed from a heteroaromatic ring or ring system heteroatom and from a heteroaromatic ring or ring system carbon atom or an aromatic hydrocarbon ring or ring system carbon atom, the group is classified as an "arylheterylene group" or an "arylhetero group."

An "arylheteryl group" is a class of "cycloheteryl group" and is a univalent group formed by removing a hydrogen atom from a heteroaromatic ring or ring system heteroatom of a heteroaryl compound, as illustrated. By specifying that the hydrogen atom is removed from of a heteroaromatic ring or ring system heteroatom and not from a heteroaromatic ring or ring system carbon atom, an "arylheteryl group" is distinguished from a "heteroaryl group" in which a hydrogen atom is removed from a heteroaromatic ring or a ring system carbon atom. Similarly, a "arylheterylene group" refers to a group formed by removing two hydrogen atoms from an heteroaryl compound, at least one of which is removed from a heteroaromatic ring or ring system heteroatom of the heteroaryl compound; the other hydrogen atom can be removed from any other atom, including for example, a heteroaromatic ring or ring system ring carbon atom, another heteroaromatic ring or ring system heteroatom, or a non-ring atom (carbon or heteroatom) from a heteroaromatic compound. An "arylhetero group" refers to a generalized group formed by removing one or more hydrogen atoms (as necessary for the particular group and at least one of which is from a heteroaromatic ring or ring system) heteroatom from a heteroarene compound.

An "organoaluminum compound," is any compound that contains an aluminum-carbon bond. Thus, organoaluminum compounds include hydrocarbyl aluminum compounds such as trialkyl-, dialkyl-, or monoalkylaluminum compounds; hydrocarbyl alumoxane compounds, and aluminate compounds which contain an aluminum-organyl bond such as tetrakis(*p*-tolyl)aluminate salts, among others.

The term "reactor effluent," and it derivatives (e.g. oligomerization reactor effluent) generally refers to all the material which exits the reactor. The term "reactor effluent," and its derivatives, can also be prefaced with other descriptors that limit the portion of the reactor effluent being referenced. For example, while the term "reactor effluent" would refer to all material exiting the reactor (e.g. product and solvent or diluent, among others), the term "olefin reactor effluent" refers to the effluent of the reactor which contains an olefin (i.e. carbon-carbon) double bond.

The term "oligomerization," and its derivatives, refers to processes which produce a mixture of products containing at least 70 weight percent products containing from 2 to 30 monomer units. Similarly, an "oligomer" is a product that contains from 2 to 30 monomer units while an "oligomerization product" includes all product made by the "oligomerization" process including the "oligomers" and products which are not "oligomers" (e.g. product which contain more than 30 monomer units). It should be noted that the monomer units in the "oligomer" or "oligomerization product" do not have to be the same. For example, an "oligomer" or "oligomerization product" of an "oligomerization" process using ethylene and propylene as monomers can contain both ethylene and/or propylene units.

The term "trimerization," and it derivatives, refers to a process which produces a mixture of products containing at least 70 weight percent products containing three and only three monomer units. A "trimer" is a product which contains three and only three monomer units while a "trimerization product" includes all products made by the trimerization process including trimer and product which are not trimer (e.g. dimers or tetramers). Generally, an olefin trimerization reduces number of olefinic bonds, i.e., carbon-carbon double bonds, by two when considering the number of olefin bonds in the monmer units and the number of olefin bonds in the trimer. It should be noted that the monomer units in the "trimer" or "trimerization product" do not have be the same. For example, a "trimer" of a "trimerization" process using ethylene and butene as monomers can contain ethylene and/or butene monomer units. That is to say the "trimer" will include C₆, C₈, C₁₀, and C₁₂ products. In another example, a "trimer" of a "trimerization" process using ethylene as the monomer can contain ethylene monomer units. It should also be noted that a single molecule can contain two monomer units. For example, dienes such as 1,3-butadiene and 1,4-pentadiene have two monomer units within one molecule.

The term "tetramerization," and it derivatives, refers to a process which produces a mixture of products containing at least 70 weight percent products containing four and only four monomer units. A "tetramer" is a product which contains four and only four monomer units while a "tetramerization product" includes all products made by the tetramerization process including tetramer and product which are not tetramer (e.g. dimers or trimer). Generally, an olefin tetramerization reduces number of olefinic bonds, i.e., carbon-carbon double bonds, by three when considering the number of olefin bonds in the monmer units and the number of olefin bonds in the tetramer. It should be noted that the monomer units in the "tetramer" or "tetramerization product" do not have be the same. For example, a "tetramer" of a "tetramerization" process using ethylene and butene as monomers can contain ethylene and/or butene monomer units. In an example, a "tetramer" of a "tetramerization" process using ethylene as the monomer can contain ethylene monomer units. It should also be noted that a single molecule can contain two monomer units. For example, dienes such as 1,3-butadiene and 1,4-pentadiene have two monomer units within one molecule.

The term "trimerization and tetramerization," and it derivatives, refers to a process which produces a mixture of products containing at least 70 weight percent products containing three and/or four and only three and/or four monomer units. A "trimerization and tetramerization product" includes all products made by the "trimerization and tetramerization" process including trimer, tetramer, and product which are not tetramer (e.g. dimers). In an example, a "trimerization and tetramerization" process using ethylene as the monomer produces a mixture of products containing at least 70 weight percent hexene and/or octene.

The term or variation of the terms an "oligomerized product having X carbon atoms" and "Cx oligomer product," wherein X can be any positive non-zero integer, refers to materials produced by monomer oligomerization which have X carbon atoms. Thus, the term oligomerized product having X carbon atoms excludes materials having X carbon atoms which were not produced by the olefin oligomerization (e.g. solvent). These terms can also include other descriptive words (e.g. olefin, liquid, and mixture, among others) without detracting from the essence of the term referring to materials having X carbon atoms, produced by monomer oligomerization, and fitting the additional descriptive terms.

Catalyst system activity is defined as grams of a product produced per gram of metal of the metal complex or metal compound utilized in the catalyst system over the first 30 minutes of an oligomerization or polymerization reaction beginning from the time when the complete catalyst system is contacted with the olefin. Catalyst system activity can be stated in terms of various products of an olefin oligomerization or polymerization. For example in an ethylene trimerization and tetramerization process utilizing a chromium based catalyst system, catalyst system activities which can be utilized include (g C₆)/(g Cr), (g C₈)/(g Cr), (C₆ + C₈) /(g Cr), (g ethylene oligomer)/(g Cr), and (total product)/(g Cr), among other activities.

In an embodiment, the catalyst system comprises a metal compound complexed to a heteroatomic ligand; or alternatively, a metal compound and a heteroatomic ligand. The heteroatomic ligand comprises a moiety characterized by having a P-N-P (phosphorus-nitrogen-phosphorus) linkage. The moiety having the P-N-P linkage can hereafter be referred to a PNP moiety or as a diphosphino aminyl moiety. The heteroatomic ligand comprising the diphosphino aminyl moiety can be referred to as a PNP ligand or as a diphosphino aminyl ligand.

In an embodiment, the heteroatomic ligand comprises a diphosphino aminyl moiety having Structure 1: 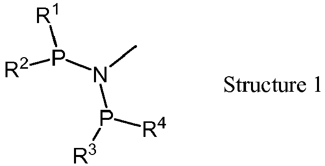 wherein R¹ R², R³, and R⁴ can be any group described herein and the undesignated aminyl nitrogen valence represents the remainder of the heteroatomic ligand. In an embodiment, R¹, R², R³, and R⁴ can each be different. In some embodiments, R¹, R², R³, and R⁴ can each be the same. In other embodiments, R¹ and R² can be the same and R³ and R⁴ can be the same but different from R¹ and R². In yet other embodiments, R¹ and R³ can be the same and R² and R⁴ can be the same but different from R¹ and R³.

In an embodiment, R¹, R², R³, and R⁴ independently can be an organyl group; alternatively, an organyl group consisting of inert functional groups; or alternatively, a hydrocarbyl group. In an embodiment, the organyl group which can be utilized as R¹, R², R³, and R⁴ can be a C₁ to C₃₀ organyl group; alternatively, a C₁ to C₂₀ organyl group; alternatively, a C₁ to C₁₅ organyl group; alternatively, a C₁ to C₁₀ organyl group; or alternatively, a C₁ to C₅ organyl group. In an embodiment, the organyl group consisting of inert functional groups which can be utilized as R¹, R², R³, and R⁴ can be a C₁ to C₃₀ organyl group consisting of inert functional groups; alternatively, a C₁ to C₂₀ organyl group consisting of inert functional groups; alternatively, a C₁ to C₁₅ organyl group consisting of inert functional groups; alternatively, a C₁ to C₁₀ organyl group consisting of inert functional groups; or alternatively, a C₁ to C₅ organyl group consisting of inert functional groups. In an embodiment, the hydrocarbyl group which can be utilized as R¹, R², R³, and R⁴ can be a C₁ to C₃₀ hydrocarbyl group; alternatively, a C₁ to C₂₀ hydrocarbyl group; alternatively, a C₁ to C₁₅ hydrocarbyl group; alternatively, a C₁ to C₁₀ hydrocarbyl group; or alternatively, a C₁ to C₅ hydrocarbyl group. In further embodiments, two or more of R¹, R², R³, and R⁴ can be joined to form a ring or a ring system.

In some embodiments, R¹, R², R³, and R⁴ independently can be an alkyl group, a substituted alkyl group, a cycloalkyl group, a substituted cycloalkyl group, an aliphatic heterocyclyl group, a substituted aliphatic heterocyclyl group, an aryl group, a substituted aryl group, an aralkyl group, a substituted aralkyl group, a heteroaryl group, or a substituted heteroaryl group; or alternatively, an alkyl group, a substituted alkyl group, a cycloalkyl group, a substituted cycloalkyl group, an aryl group, a substituted aryl group, an aralkyl group, or a substituted aralkyl group. In other embodiments, the R¹, R², R³, and R⁴ independently can be an alkyl group or a substituted alkyl group; alternatively, a cycloalkyl group or a substituted cycloalkyl group; alternatively, an aliphatic heterocyclyl group or a substituted aliphatic heterocyclyl group; alternatively, an aryl group or a substituted aryl group; alternatively, an aralkyl group or a substituted aralkyl group; alternatively, a heteroaryl group or a substituted heteroaryl group; or alternatively, an alkyl group, a cycloalkyl group, an aryl group, or an aralkyl group. In yet other embodiments, the R¹, R², R³, and R⁴ independently can be an alkyl group; alternatively, a substituted alkyl group, alternatively, a cycloalkyl group; alternatively, a substituted cycloalkyl group; alternatively, an aliphatic heterocyclyl group; alternatively, a substituted heterocyclyl group; alternatively, an aryl group; alternatively, a substituted aryl group; alternatively, an aralkyl group; alternatively, a susbtituted aralkyl group; alternatively, a heteroaryl group; or alternatively, a substituted heteroaryl group.

In any aspect or embodiment disclosed herein, each alkyl group which can be utilized as R¹, R², R³, and R⁴ independently can be a C₁ to C₃₀ alkyl group; alternatively, a C₁ to C₂₀ alkyl group; alternatively, a C₁ to C₁₀ alkyl group; or alternatively, C₁ to C₅ alkyl group. In any aspect or embodiment disclosed herein, each substituted alkyl group which can be utilized as R¹, R², R³, and R⁴ independently can be a C₁ to C₃₀ substituted alkyl group; alternatively, a C₁ to C₂₀ substituted alkyl group; alternatively, a C₁ to C₁₀ substituted alkyl group; or alternatively, C₁ to C₅ substituted alkyl group. In any aspect or embodiment disclosed herein, each cycloalkyl group which can be utilized as R¹, R², R³, and R⁴ independently can be a C₄ to C₃₀ cycloalkyl group; alternatively, a C₄ to C₂₀ cycloalkyl group; alternatively, a C₄ to C₁₅ cycloalkyl group; or alternatively, C₄ to C₁₀ cycloalkyl group. In any aspect or embodiment disclosed herein, each substituted cycloalkyl group which can be utilized as R¹, R², R³, and R⁴ independently can be a C₄ to C₃₀ substituted cycloalkyl group; alternatively, a C₄ to C₂₀ substituted cycloalkyl group; alternatively, a C₄ to C₁₅ substituted cycloalkyl group; or alternatively, a C₄ to C₁₀ substituted cycloalkyl group. In any aspect or embodiment disclosed herein, each aliphatic heterocyclyl group can be a C₃ to C₃₀ aliphatic heterocyclyl group; alternatively, a C₄ to C₂₀ aliphatic heterocyclyl group; alternatively, a C₄ to C₁₅ aliphatic heterocyclyl group; or alternatively, a C₄ to C₁₀ aliphatic heterocyclyl group. In any aspect or embodiment disclosed herein, each substituted aliphatic heterocyclyl group can be a C₃ to C₃₀ substituted aliphatic heterocyclyl group; alternatively, a C₄ to C₂₀ substituted aliphatic heterocyclyl group; alternatively, a C₄ to C₁₅ substituted aliphatic heterocyclyl group; or alternatively, a C₄ to C₁₀ substituted aliphatic heterocyclyl group. In any aspect or embodiment disclosed herein, each aryl group which can be utilized as R¹, R², R³, and R⁴ independently can be a C₆ to C₃₀ aryl group; alternatively, a C₆ to C₂₀ aryl group; alternatively, a C₆ to C₁₅ aryl group; or alternatively, a C₆ to C₁₀ aryl group. In any aspect or embodiment disclosed herein, each substituted aryl group which can be utilized as R¹, R², R³, and R⁴ independently can be a C₆ to C₃₀ substituted aryl group; alternatively, a C₆ to C₂₀ substituted aryl group; alternatively, a C₆ to C₁₅ substituted aryl group; or alternatively, a C₆ to C₁₀ substituted aryl group. In any aspect or embodiment disclosed herein, each aralkyl group which can be utilized as R¹, R², R³, and R⁴ independently can be a C₇ to C₃₀ aralkyl group; alternatively, a C₇ to C₂₀ aralkyl group; alternatively, a C₇ to C₁₅ aralkyl group; or alternatively, a C₇ to C₁₀ aralkyl group. In any aspect or embodiment disclosed herein, each substituted aryl group which can be utilized as R¹, R², R³, and R⁴ independently can be a C₇ to C₃₀ substituted aralkyl group; alternatively, a C₇ to C₂₀ substituted aralkyl group; alternatively, a C₇ to C₁₅ aralkyl group; or alternatively, a C₇ to C₁₀ substituted aralkyl group. In any aspect or embodiment disclosed herein, each heteroaryl group can be a C₃ to C₃₀ heteroaryl group; alternatively, a C₄ to C₂₀ heteroaryl group; alternatively, a C₄ to C₁₅ heteroaryl group; or alternatively, a C₄ to C₁₀ heteroaryl group. In any aspect or embodiment disclosed herein, each substituted heteroaryl group can be a C₃ to C₃₀ substituted heteroaryl group; alternatively, a C₄ to C₂₀ substituted heteroaryl group; alternatively, a C₄ to C₁₅ substituted heteroaryl group; or alternatively, a C₄ to C₁₀ substituted heteroaryl group. Each substituent of a substituted alkyl group (general or specific), a substituted cycloalkyl group (general or specific), susbstituted heteroaryl group (general or specific), a substituted aryl group (general or specific), a substituted aralkyl group (general or specific), and/or a substituted heteroaryl group (general or specific can be a halogen, a hydrocarbyl group, or a hydrocarboxy group; alternatively, a halogen or a hydrocarbyl group; alternatively, a halogen or a hydrocarboxy group; alternatively, a hydrocarbyl group or a hydrocarboxy group; alternatively, a halogen; alternatively, a hydrocarbyl group; or alternatively, a hydrocarboxy group. Substituent halogens, substituent hydrocarbyl groups, and substituent hydrocarboxy groups are independently disclosed herein. These substituent halogens, substitutent hydrocarboxy groups, and substituent hydrocarboxy groups can be utilized without limitation to further describe R¹, R², R³, and R⁴.

In an embodiment, R¹, R², R³, and R⁴ indepenently can be a methyl group, an ethyl group, a propyl group, a butyl group, a pentyl group, a hexyl group, a heptyl group, an octyl group, a nonyl group, a decyl group, a undecyl group, a dodecyl group, a tridecyl group, a tetradecyl group, a pentadecyl group, a hexadecyl group, a heptadecyl group, an octadecyl group, or a nonadecyl group; or alternatively, a methyl group, an ethyl group, a propyl group, a butyl group, a pentyl group, a hexyl group, a heptyl group, an octyl group, a nonyl group, or a decyl group. In some embodiments, R¹, R², R³, and R⁴ indepenently can be a methyl group, an ethyl group, an n-propyl (1-propyl) group, an isopropyl (2-propyl) group, an n-butyl (1-butyl) group, a *sec*-butyl (2-butyl) group, an isobutyl (2-methyl-1-propyl) group, a *tert*-butyl (2-methyl-2-propyl) group, an n-pentyl (1-pentyl) group, a 2-pentyl group, a 3-pentyl group, a 2-methyl-1-butyl group, a *tert*-pentyl (2-methyl-2-butyl) group, a 3-methyl-1-butyl group, a 3-methyl-2-butyl group, or a neo-pentyl (2,2-dimethyl-1-propyl) group; alternatively, a methyl group, an ethyl group, an iso-propyl (2-propyl) group, a tert-butyl (2-methyl-2-propyl) group, or a neopentyl (2,2-dimethyl-1-propyl) group; alternatively, a methyl group; alternatively, an ethyl group; alternatively, a n-propyl (1-propyl) group; alternatively, an iso-propyl (2-propyl) group; alternatively, a tert-butyl (2-methyl-2-propyl) group; or alternatively, a neopentyl (2,2-dimethyl-1-propyl) group. In some embodiments, the alkyl groups which can be utilized as R¹, R², R³, and R⁴ can be substituted. Each substituent of a substituted alkyl group independently can be a halogen or a hydrocarboxy group; alternatively, a halogen; or alternatively, a hydrocarboxy group. Substituent halogens and substituent hydrocarboxy groups are independently disclosed herein. These substituent halogens and substituent hydrocarboxy groups can be utilized without limitation to further describe a substituted alkyl group which can be utilized as R¹, R², R³, and R⁴.

In an embodiment, R¹, R², R³, and R⁴ independently can be a cyclobutyl group, a substituted cyclobutyl group, a cyclopentyl group, a substituted cyclopentyl group, a cyclohexyl group, a substituted cyclohexyl group, a cycloheptyl group, a substituted cycloheptyl group, a cyclooctyl group, or a substituted cyclooctyl group. In some embodiments, R¹, R², R³, and R⁴ independently can be a cyclopentyl group, a substituted cyclopentyl group, a cyclohexyl group, or a substituted cyclohexyl group. In other embodiments, R¹, R², R³, and R⁴ independently can be a cyclobutyl group or a substituted cyclobutyl group; alternatively, a cyclopentyl group or a substituted cyclopentyl group; alternatively, a cyclohexyl group or a substituted cyclohexyl group; alternatively, a cycloheptyl group or a substituted cycloheptyl group; or alternatively, a cyclooctyl group, or a substituted cyclooctyl group. In further embodiments, R¹, R², R³, and R⁴ independently can be a cyclopentyl group; alternatively, a substituted cyclopentyl group; a cyclohexyl group; or alternatively, a substituted cyclohexyl group. In an embodiment, R¹, R², R³, and R⁴ independently can be a 2-substituted cyclohexyl group, a 2,6-disubstituted cyclohexyl group, a 2-subsituted cyclopentyl group, or a 2,5-disubstituted cyclopentyl group; alternatively, a 2-substituted cyclohexyl group or a 2,6-disubstituted cyclohexyl group; alternatively, a 2-subsituted cyclopentyl group or a 2,5-disubstituted cyclopentyl group; alternatively, a 2-substituted cyclohexyl group or a 2-subsituted cyclopentyl group; alternatively, a 2,6-disubstituted cyclohexyl group or a 2,5-disubstituted cyclopentyl group; alternatively, a 2-substituted cyclohexyl group; alternatively, a 2,6-disubstituted cyclohexyl group; alternatively, a 2-subsituted cyclopentyl group; or alternatively, a 2,5-disubstituted cyclopentyl group. Each substituent of a cycloalkyl group having a specified number of ring carbon atoms independently can be a halogen, a hydrocarbyl group, or a hydrocarboxy group; alternatively, a halogen or a hydrocarbyl group; alternatively, a halogen or a hydrocarboxy group; alternatively, a hydrocarbyl group or a hydrocarboxy group; alternatively, a halogen, alternatively, a hydrocarbyl group; or alternatively, a hydrocarboxy group. Substituent halogens, substituent hydrocarbyl groups, and substituent hydrocarboxy groups are independently disclosed herein. These substituent halogens, substituent hydrocarbyl groups, and substituent hydrocarboxy groups can be utilized without limitation to further describe a substituted cycloalkyl group (general or specific) which can be utilized as R¹, R², R³, and R⁴.

In an embodiment, R¹, R², R³, and R⁴ independently can be a tetrahydrofuranyl group or a substituted tetrahydrofuranyl group; alternatively, a tetrahydrofuranyl group; or alternatively, a substituted tetrahydrofuranyl group. Each substituent of a substituted tetrahydrofuranyl group (general or specific) independently can be a halogen, a hydrocarbyl group, or a hydrocarboxy group; alternatively, a halogen or a hydrocarbyl group; alternatively, a halogen or a hydrocarboxy group; alternatively, a hydrocarbyl group or a hydrocarboxy group; alternatively, a halogen, alternatively, a hydrocarbyl group; or alternatively, a hydrocarboxy group. Substituent halogens, substituent hydrocarbyl groups, and substituent hydrocarboxy groups are independently disclosed herein. These substituent halogens, substituent hydrocarbyl groups, and substituent hydrocarboxy can be utilized without limitation to further describe a substituted tetrahydrofuranyl group (general or specific) which can be utilized as R¹, R², R³, and R⁴.

In an embodiment, R¹, R², R³, and R⁴ independently can be a phenyl group, a substituted phenyl group, a naphthyl group, a substituted naphthyl group, a 4-phenyl-phenyl (4-biphenyl) group, a substituted 4-phenyl-phenyl (4-biphenyl) group, an anthracenyl group, or a substituted anthracenyl group; alternatively, a phenyl group or a substituted phenyl group; alternatively, a naphthyl group or a substituted naphthyl group; alternatively, a phenyl group or a naphthyl group; alternatively, a phenyl group; alternatively, a substituted phenyl group; alternatively, a naphthyl group; alternatively a substituted naphthyl group; alternatively, a 4-phenyl-phenyl (4-biphenyl) group or a substituted 4-phenyl-phenyl (4-biphenyl) group; or alternatively, an anthracenyl group or a substituted anthracenyl group. In an embodiment, R¹, R², R³, and R⁴ independently can be a 2-substituted phenyl group, a 3-substituted phenyl group, a 4-substituted phenyl group, a 2,4-disubstituted phenyl group, a 2,6-disubstituted phenyl group, a 3,5-disubstituted phenyl group, or a 2,4,6-trisubstituted phenyl group. In other embodiments, R¹, R², R³, and R⁴ independently can be a 2-substituted phenyl group, a 4-substituted phenyl group, a 2,4-disubstituted phenyl group, or a 2,6-disubstituted phenyl group; alternatively, a 3-subsituted phenyl group or a 3,5-disubstituted phenyl group; alternatively, a 2-substituted phenyl group or a 4-substituted phenyl group; alternatively, a 2,4-disubstituted phenyl group or a 2,6-disubstituted phenyl group; alternatively, a 2-substituted phenyl group; alternatively, a 3-substituted phenyl group; alternatively, a 4-substituted phenyl group; alternatively, a 2,4-disubstituted phenyl group; alternatively, a 2,6-disubstituted phenyl group; alternatively, a 3,5-disubstituted phenyl group; or alternatively, a 2,4,6-trisubstituted phenyl group. In some embodiments, R¹, R², R³, and R⁴ independently can be a 2-naphthyl group or a substituted 2-naphthyl group; alternatively, a 2-naphthyl group; or alternatively, a substituted 2-naphthyl group. Each substituent of a substituted phenyl group (general or specific), a substituted naphthyl group (general of specific), a substituted 4-phenyl-phenyl (4-biphenyl) group (general or specific), or a substituted anthracenyl group (general or specific) independently can be a halogen, a hydrocarbyl group, or a hydrocarboxy group; alternatively, a halogen or a hydrocarbyl group; alternatively, a halogen or a hydrocarboxy group; alternatively, a hydrocarbyl group or a hydrocarboxy group; alternatively, a halogen, alternatively, a hydrocarbyl group; or alternatively, a hydrocarboxy group. Substituent halogens, substituent hydrocarbyl groups, and substituent hydrocarboxy groups are independently disclosed herein. These substituent halogens, substituent hydrocarbyl groups, and substituent hydrocarboxy can be utilized without limitation to further describe a substituted phenyl group (general or specific), a substituted naphthyl group (general or specific), a substituted 4-phenyl-phenyl (4-biphenyl) group (general or specific), or a substituted anthracenyl group (general or specific) which can be utilized as R¹, R², R³, and R⁴.

In an aspect, R¹, R², R³, and R⁴ independently can be a pyridinyl group, a substituted pyridinyl group, a furyl group, a substituted furyl group, a thiophenyl group, or a susbstituted thiophenyl group; alternatively, a pyridinyl group or a substituted pyridinyl group; alternatively, a furyl group or a substituted furyl group; alternatively, a thiophenyl group, or a susbstituted thiophenyl group; alternatively, a pyridinyl group or a furyl group; or alternatively, a pyridinyl group; alternatively, a substituted pyridinyl group; alternatively, a furyl group; alternatively, a substituted furyl group; alternatively, a thiophenyl group; or alternatively, a susbstituted thiophenyl group. In an embodiment, R¹, R², R³, and R⁴ independently can be a pyridin-2-yl group, a substituted pyridin-2-yl group, a pyridin-3-yl group, a substituted pyridin-3-yl group, a pyridin-4-yl group, or a substituted pyridin-4-yl group; alternatively, a pyridin-2-yl group, a pyridin-3-yl group, or a pyridin-4-yl group; alternatively, a pyridin-2-yl group or a substituted pyridin-2-yl group; alternatively, a pyridin-3-yl group or a substituted pyridin-3-yl group; alternatively a pyridin-4-yl group, or a substituted pyridin-4-yl group; alternatively, a pyridin-2-yl group; alternatively, a substituted pyridin-2-yl group; alternatively, a pyridin-3-yl group; alternatively, a substituted pyridin-3-yl group; alternatively, a pyridin-4-yl group; or alternatively, a substituted pyridin-4-yl group. In an embodiment, the substituted pyridinyl R¹, R², R³, and R⁴ group can be a 2-substituted pyridin-3-yl group, a 4-substituted pyridin-3-yl group, a 5-substituted pyridin-3-yl group, a 6-substituted pyridin-3-yl group, a 2,4-disubstituted pyridin-3-yl group, a 2,6-disubstituted pyridin-3-yl group, or a 2,4,6-trisubstituted pyridin-3-yl group; alternatively, a 2-substituted pyridin-3-yl group, a 4-substituted pyridin-3-yl group, a 6-substituted pyridin-3-yl group; alternatively, a 2,4-disubstituted pyridin-3-yl group or a 2,6-disubstituted pyridin-3-yl group; alternatively, a 2-substituted pyridin-3-yl group; alternatively, a 4-substituted pyridin-3-yl group; alternatively, a 5-substituted pyridin-3-yl group; alternatively, a 6-substituted pyridin-3-yl group; alternatively, a 2,4-disubstituted pyridin-3-yl group; alternatively, a 2,6-disubstituted pyridin-3-yl group; or alternatively, a 2,4,6-trisubstituted pyridin-3-yl group. In an embodiment, the substituted pyridinyl R¹, R², R³, and R⁴ group can be a 2-substituted pyridin-4-yl group, a 3-substituted pyridin-4-yl group, a 5-substituted pyridin-4-yl group, a 6-substituted pyridin-4-yl group, a 2,6-disubstituted pyridin-4-yl group, or a 3,5-disubstituted pyridin-4-yl group; alternatively, a 2-substituted pyridin-4-yl group, a 6-substituted pyridin-4-yl group; alternatively, a 3-substituted pyridin-4-yl group or a 5-substituted pyridin-4-yl group; alternatively, a 2-substituted pyridin-4-yl group; alternatively, a 3-substituted pyridin-4-yl group; alternatively, a 5-substituted pyridin-4-yl group; alternatively, a 6-substituted pyridin-4-yl group; alternatively, a 2,6-disubstituted pyridin-4-yl group; or alternatively, a 3,5-disubstituted pyridin-4-yl group. In an embodiment, the substituted furyl R¹, R², R³, and R⁴ group can be a fur-2-yl group, a substituted fur-2-yl group, a fur-3-yl group, or a substituted fur-3-yl group; alternatively, a fur-2-yl or a fur-3-yl group. In some embodiments, the substituted furyl R¹, R², R³, and R⁴ group can be a fur-2-yl group or a substituted fur-2-yl group; alternatively, a fur-3-yl group, or a substituted fur-3-yl group; alternatively, a fur-2-yl group; alternatively, a substituted fur-2-yl group; alternatively, a fur-3-yl group; or alternatively, a substituted fur -3-yl group. In an embodiment, the substituted furyl R¹, R², R³, and R⁴ group can be a 2-substituted fur-3-yl group, a 4-substituted fur-3-yl group, or a 2,4-disubstituted fur-3-yl group; alternatively, a 2-substituted fur-3-yl group; alternatively, a 4-substituted fur-3-yl group; or alternatively, a 2,4-disubstituted fur-3-yl group. Each substituent of a substituted pyridinyl group (general or specific) or a substituted furyl group (general of specific) independently can be a halogen, a hydrocarbyl group, or a hydrocarboxy group; alternatively, a halogen or a hydrocarbyl group; alternatively, a halogen or a hydrocarboxy group; alternatively, a hydrocarbyl group or a hydrocarboxy group; alternatively, a halogen, alternatively, a hydrocarbyl group; or alternatively, a hydrocarboxy group. Substituent halogens, substituent hydrocarbyl groups, and substituent hydrocarboxy groups are independently disclosed herein. These substituent halogens, substituent hydrocarbyl groups, and substituent hydrocarboxy can be utilized without limitation to further describe a substituted pyridinyl group (general or specific) or a substituted fury group (general or specific) which can be utilized as R¹, R², R³, and R⁴.

In an aspect, R¹, R², R³, and R⁴ independently can be a benzyl group or a substituted benzyl group. In some embodiments R¹, R², R³, and R⁴ independently can be a benzyl group; or alternatively, a substituted benzyl group. Each substituent of a substituted benzyl group independently can be a halogen, a hydrocarbyl group, or a hydrocarboxy group; alternatively, a halogen or a hydrocarbyl group; alternatively, a halogen or a hydrocarboxy group; alternatively, a hydrocarbyl group or a hydrocarboxy group; alternatively, a halogen, alternatively, a hydrocarbyl group; or alternatively, a hydrocarboxy group. Substituent halogens, substituent hydrocarbyl groups, and substituent hydrocarboxy groups are independently disclosed herein. These substituent halogens, substituent hydrocarbyl groups, and substituent hydrocarboxy can be utilized without limitation to further describe a substituted benzyl which can be utilized as R¹, R², R³, and R⁴.

In an embodiment, R¹, R², R³, and R⁴ can each be independently selected from the group consisting of methyl, ethyl, n-propyl (1-propyl), isopropyl (2-propyl), n-butyl (1-butyl), *sec*-butyl (2-butyl), isobutyl (2-methyl-1-propyl), *tert*-butyl (2-methyl-2-propyl), n-pentyl (1-pentyl), 2-pentyl, 3-pentyl, 2-methyl-1-butyl, *tert*-pentyl (2-methyl-2-butyl), 3-methyl-1-butyl, 3-methyl-2-butyl, neo-pentyl (2,2-dimethyl-1-propyl), n-hexyl (1-hexyl) cyclopentyl, substituted cyclopentyl cyclohexyl, a substituted cyclohexyl, benzyl, substituted benzyl, phenyl, a substituted phenyl, 4-phenyl-phenyl (4-biphenyl) substituted 4-phenyl-phenyl (substituted biphenyl), 2-naphthyl, substituted 2-naphthyl, anthracenyl, substituted anthracenyl pyridinyl, substituted pyridinyl, tetrahydrofuranyl, and a substituted tetrahydrofuranyl group. In some embodiments, R¹, R², R³, and R⁴ can each be independently selected from the group consisting of methyl, ethyl, isopropyl (2-propyl), *tert*-butyl (2-methyl-2-propyl), neo-pentyl (2,2-dimethyl-1-propyl), benzyl, substituted benzyl, phenyl, and a substituted phenyl group. Alternatively, R¹, R², R³, and R⁴ can each independently be a phenyl group or a substituted phenyl group. In some embodiments, R¹, R², R³, and R⁴ can each independently be a phenyl group; or alternatively, a substituted phenyl group.

In an embodiment, each halide substituent utilized as substituent of a subsituted alkyl group (general or specific), a substituted cycloalkyl group (general or specific), a substituted aliphatic heterocyclyl group (general or specific), a substituted aryl group (general or specific), a substituted aralkyl group (general or specific), or a substituted heteroaryl group (general or specific) independently can be a fluoride, chloride, bromide, or iodide; alternatively, fluoride or chloride. In some embodiments, each halide substituent utilized as substituent of a subsituted alkyl group (general or specific), a substituted cycloalkyl group (general or specific), a substituted aliphatic heterocyclyl group (general or specific), a substituted aryl group (general or specific), or a substituted heteroaryl group (general or specific) can be a fluoride; alternatively, a chloride; alternatively, a bromide; or alternatively, an iodide.

In an embodiment, each hydrocarbyl substituent utilized as substituent of a subsituted alkyl group (general or specific), a substituted cycloalkyl group (general or specific), a substituted aliphatic heterocyclyl group (general or specific), a substituted aryl group (general or specific), a substituted aralkyl group (general or specific), or a substituted heteroaryl group (general or specific) independently can be a C₁ to C₁₅ hydrocarbyl group; alternatively, a C₁ to C₁₀ hydrocarbyl group; or alternatively, a C₁ to C₅ hydrocarbyl group. In some embodiments, each hydrocarbyl substituent utilized as substituent of a subsituted alkyl group (general or specific), a substituted cycloalkyl group (general or specific), a substituted aliphatic heterocyclyl group (general or specific), a substituted aryl group (general or specific), a substituted aralkyl group (general or specific), or a substituted heteroaryl group (general or specific) independently can be an alkyl group, an aryl group, or an aralkyl group; alternatively, an alkyl group; alternatively, an aryl group; or alternatively, an aralkyl group. In an embodiment, alkyl groups which can be utilized as substituents can be C₁ to C₁₀ alkyl group; or alternatively, a C₁ to C₅ alkyl group. In some embodiments, each alkyl substituent utilized as substituent of a subsituted alkyl group (general or specific), a substituted cycloalkyl group (general or specific), a substituted aliphatic heterocyclyl group (general or specific), a substituted aryl group (general or specific), a substituted aralkyl group (general or specific), or a substituted heteroaryl group (general or specific) independently can be a methyl group, an ethyl group, an n-propyl (1-propyl) group, an isopropyl (2-propyl) group, an n-butyl (1-butyl) group, a sec-butyl (2-butyl) group, an isobutyl (2-methyl-1-propyl) group, a tert-butyl (2-methyl-2-propyl) group, an n-pentyl (1-pentyl) group, a 2-pentyl group, a 3-pentyl group, a 2-methyl-1-butyl group, a tert-pentyl (2-methyl-2-butyl) group, a 3-methyl-1-butyl group, a 3-methyl-2-butyl group, or a neo-pentyl (2,2-dimethyl-1-propyl) group; alternatively, a methyl group, an ethyl group, an isopropyl (2-propyl) group, a tert-butyl (2-methyl-2-propyl) group, or a neo-pentyl (2,2-dimethyl-1-propyl) group; alternatively, a methyl group; alternatively, an ethyl group; alternatively, an isopropyl (2-propyl) group; alternatively, a tert-butyl (2-methyl-2-propyl)group; or alternatively, a neo-pentyl (2,2-dimethyl-1-propyl) group. In an embodiment, the aryl group which can be utilized as a substituent can be a C₁ to C₁₅ aryl group; or alternatively, a C₁ to C₁₀ aryl group. In some embodiments, each aryl substituent utilized as substituent of a subsituted alkyl group (general or specific), a substituted cycloalkyl group (general or specific),a substituted aliphatic heterocyclyl group (general or specific), a substituted aryl group (general or specific), a substituted aralkyl group (general or specific), or a substituted heteroaryl group (general or specific) independently can be a phenyl group, a tolyl group, a xylyl group, or a 2,4,6-trimethylphenyl group; alternatively, a phenyl group; alternatively, a tolyl group, alternatively, a xylyl group; or alternatively, a 2,4,6-trimethylphenyl group. In an embodiment, the aralkyl group which can be utilized as a substituent can be a C₁ to C₁₅ aralkyl group; or alternatively, a C₁ to C₁₀ aralkyl group. In some embodiments, each aralkyl substituent utilized as substituent of a subsituted alkyl group (general or specific), a substituted cycloalkyl group (general or specific), a substituted aliphatic heterocyclyl group (general or specific), a substituted aryl group (general or specific), a substituted aralkyl group (general or specific), or a substituted heteroaryl group (general or specific) independently can be a benzyl group or an ethylphenyl group (2-phenyleth-1-yl or 1-phenyleth-1-yl); alternatively, a benzyl group; alternatively, a ethylphenyl group; alternatively a 2-phenyleth-1-yl group; or alternatively, a 1-phenyleth-1-yl group .

In an embodiment, each hydrocarboxy substituent substituent utilized as a substituent of a subsituted alkyl group (general or specific), a substituted cycloalkyl group (general or specific), a substituted aliphatic heterocyclyl group (general or specific), a substituted aryl group (general or specific), a substituted aralkyl group (general or specific), or a substituted heteroaryl group (general or specific) independently can be a C₁ to C₁₅ hydrocarboxy group; alternatively, a C₁ to C₁₀ hydrocarboxy group; or alternatively, a C₁ to C₅ hydrocarboxy group. In some embodiments, each hydrocarbyl substituent utilized as a substituent of a subsituted alkyl group (general or specific), a substituted cycloalkyl group (general or specific), a substituted aliphatic heterocyclyl group (general or specific), a substituted aryl group (general or specific), a substituted aralkyl group (general or specific), or a substituted heteroaryl group (general or specific) independently can be an alkoxy group, an aryloxy group, or an aralkoxy group; alternatively, an alkoxy group; alternatively, an aryloxy group; or alternatively, an aralkoxy group. In an embodiment, alkoxy groups which can be utilized as substituents can be C₁ to C₁₀ alkoxy group; or alternatively, a C₁ to C₅ alkoxy group. In some embodiments, each alkoxy substituent utilized as a substituent of a subsituted alkyl group (general or specific), a substituted cycloalkyl group (general or specific), a substituted aliphatic heterocyclyl group (general or specific), a substituted aryl group (general or specific), a substituted aralkyl group (general or specific), or a substituted heteroaryl group (general or specific) independently can be a methoxy group, an ethoxy group, an n-propoxy (1-propoxy) group, an isopropoxy (2-propoxy) group, an n-butoxy (1-butoxy) group, a sec-butoxy (2-butoxy) group, an isobutoxy (2-methyl-1-propoxy) group, a tert-butoxy (2-methyl-2-propoxy) group, an n-pentoxy (1-pentoxy) group, a 2-pentoxy group, a 3-pentoxy group, a 2-methyl-1-butoxy group, a tert-pentoxy (2-methyl-2-butoxy) group, a 3-methyl-1-butoxy group, a 3-methyl-2-butoxy group, or a neo-pentoxy (2,2-dimethyl-1-propoxy) group; alternatively, a methoxy group, an ethoxy group, an isopropoxy (2-propoxy) group, a tert-butoxy (2-methyl-2-propoxy) group, or a neo-pentoxy (2,2-dimethyl-1-propoxy) group; alternatively, a methoxy group; alternatively, an ethoxy group; alternatively, an isopropoxy (2-propoxy) group; alternatively, a tert-butoxy (2-methyl-2-propoxy) group; or alternatively, a neo-pentoxy (2,2-dimethyl-1-propoxy) group. In an embodiment, the aroxy group which can be utilized as a substituent can be C₁ to C₁₅ aroxy group; or alternatively, a C₁ to C₁₀ aroxy group. In some embodiments, each aroxy substituent utilized as a substituent of a subsituted alkyl group (general or specific), a substituted cycloalkyl group (general or specific), a substituted aliphatic heterocyclyl group (general or specific), a substituted aryl group (general or specific), a substituted aralkyl group (general or specific), or a substituted heteroaryl group (general or specific) independently can be a phenoxy group, a toloxy group, a xyloxy group, or a 2,4,6-trimethylphenoxy group; alternatively, a phenoxy group; alternatively, a toloxy group, alternatively, a xyloxy group; or alternatively, a 2,4,6-trimethylphenoxy group. In an embodiment, the aralkoxy group which can be utilized as a substituent can be a C₁ to C₁₅ aralkoxy group; or alternatively, a C₁ to C₁₀ aralkoxy group. In some embodiments, each aralkoxy substituent substituent utilized as a substituent of a subsituted alkyl group (general or specific), a substituted cycloalkyl group (general or specific), a substituted aliphatic heterocyclyl group (general or specific), substituted aryl group (general or specific), or a substituted heteroaryl group (general or specific) independently can be a benzoxy group.

In other non-limiting embodiments, R¹, R², R³, and R⁴ independently can be a cyclohexyl group, a 2-alkylcyclohexyl group, or a 2,6-dialkylcyclohexyl group; alternatively, a cyclopentyl group, a 2-alkylcyclopentyl group, or a 2,5-dialkylcyclopentyl group; alternatively, cyclohexyl group; alternatively, a 2-alkylcyclohexyl group; alternatively, a 2,6-dialkylcyclohexyl group; alternatively, a cyclopentyl group; alternatively, a 2-alkylcyclopentyl group; or alternatively, or 2,5-dialkylcyclopentyl group. Alkyl substituent groups are independently described herein. These alkyl substituent groups can be utilized, without limitation, to further described an alkylcyclohexyl, dialkylcyclohexyl, alkylcyclopentyl, and/or dialkylcyclopentyl groups which can be utilized as R¹, R², R³, and R⁴. Generally, the alkyl substitutents of a disubstituted cyclohexyl or cyclopentyl group can be the same; or alternatively, the alkyl substituents of a dialkyl cyclohexyl or cyclopentyl group can be different.

In a non-limiting embodiment, R¹, R², R³, and R⁴ independently can be a 2-methylcyclohexyl group, a 2-ethylcyclohexyl group, a 2-isopropylcyclohexyl group, a 2-tert-butylcyclohexyl group, a 2,6-dimethylcyclohexyl group, a 2,6-diethylcyclohexyl group, a 2,6-diisopropylcyclohexyl group, or a 2,6-di-tert-butylcyclohexyl group. In a non-limiting embodiment, R¹, R², R³, and R⁴ independently can be, a 2-methylcyclohexyl group, a 2-ethylcyclohexyl group, a 2-isopropylcyclohexyl group, or a 2-tert-butylcyclohexyl group; alternatively, a 2,6-dimethylcyclohexyl group, a 2,6-diethylcyclohexyl group, a 2,6-diisopropylcyclohexyl group, or a 2,6-di-tert-butylcyclohexyl group; alternatively, a 2-methylcyclohexyl group; alternatively, a 2-ethylcyclohexyl group; alternatively, a 2-isopropylcyclohexyl group; alternatively, a 2-tert-butylcyclohexyl group; alternatively, a 2,6-dimethylcyclohexyl group; alternatively, a 2,6-diethylcyclohexyl group; alternatively, a 2,6-diisopropylcyclohexyl group; or alternatively, or 2,6-di-tert-butylcyclohexyl group.

In a non-limiting embodiment, R¹, R², R³, and R⁴ independently can be a phenyl group, a 2-alkylphenyl group, a 3-alkylphenyl group, a 4-alkylphenyl group, a 2,4-dialkylphenyl group a 2,6-dialkylphenyl group, a 3,5-dialkylphenyl group, or a 2,4,6-trialkylphenyl group; alternatively, a 2-alkylphenyl group, a 4-alkylphenyl group, a 2,4-dialkylphenyl group, a 2,6-dialkylphenyl group, or a 2,4,6-trialkylphenyl group; alternatively, a 2-alkylphenyl group or a 4-alkylphenyl group; alternatively, a 2,4-dialkylphenyl group a 2,6-dialkylphenyl group; alternatively, a 3-alkylphenyl group or a 3,5-dialkylphenyl group; alternatively, a 2-alkylphenyl group or a 2,6-dialkylphenyl group; alternatively, a 2-alkylphenyl group; alternatively, a 3-alkylphenyl group; alternatively, a 4-alkylphenyl group; alternatively, a 2,4-dialkylphenyl group; alternatively, a 2,6-dialkylphenyl group; alternatively, a 3,5-dialkylphenyl group; or alternatively, a 2,4,6-trialkylphenyl group. Alkyl substituent groups are independently described herein. These alkyl substituent groups can be utilized, without limitation, to further described an alkylphenyl, dialkylphenyl, and/or trialkylphenyl groups which can be utilized as R¹, R², R³, and R⁴. Generally, the alkyl substitutents of a dialkylphenyl groups or trialkyl groups can be the same; or alternatively, the alkyl substituents of a dialkylphenyl group can be different.

In some non-limiting embodiment, R¹, R², R³, and R⁴ independently can be a phenyl group, a 2-alkoxyphenyl group, a 3-alkoxyphenyl group, a 4-alkoxyphenyl group, or 3,5-dialkoxyphenyl group; alternatively, a 2-alkoxyphenyl group or a 4-alkoxyphenyl group; alternatively, a 3-alkoxyphenyl group or 3,5-dialkoxyphenyl group; alternatively, a 2-alkoxyphenyl group, alternatively, 3-alkoxyphenyl group; alternatively, a 4-alkoxyphenyl group; alternatively, a 3,5-dialkoxyphenyl group. Alkoxy group substituents are independently described herein. These alkoxy substituents can be utilized, without limitation, to further described the alkoxyphenyl group(s) and/or dialkoxyphenyl group(s) which can be utilized as R¹, R², R³, and R⁴. Generally, the alkoxy substitutents of a dialkoxyphenyl group can be the same; or alternatively, the alkoxy substituents of a dialkoxyphenyl group can be different.

In other non-limiting embodiments, R¹, R², R³, and R⁴ independently can be a phenyl group, a 2-halophenyl group, a 3-halophenyl group, a 4-halophenyl group, a 2,6-dihalophenylgroup, or a 3,5-dialkylphenyl group; alternatively, a 2-halophenyl group, a 4- halophenyl group, or a 2,6-dihalophenyl group; alternatively, a 2-halophenyl group or a 4-halophenyl group; alternatively, a 3-halophenyl group or a 3,5-dihalophenyl group; alternatively, a 2-halophenyl group; alternatively, a 3-halophenyl group; alternatively, a 4-halophenyl group; alternatively, a 2,6-dihalophenyl group; or alternatively, a 3,5-dihalophenyl group. Halide substituents are independently described herein. These halide substituents can be utilized, without limitation, to further describe a halophenyl group and/or a dihalophenyl group which can be utilized as R¹, R², R³, and R⁴. Generally, the halides of a dihalophenyl group can be the same; or alternatively, the halides of a dihalophenyl group can be different. In some non-limiting embodiments, the halogen of the halo substituted phenyl groups can be fluorine.

In a non-limiting embodiment, R¹, R², R³, and R⁴ independently can be a phenyl group, a 2-methylphenyl group, a 2-ethylphenyl group, a 2-n-propylphenyl group, a 2-isopropylphenyl group, a 2-tert-butylphenyl group, a 3-methylphenyl group, a 2,6-dimethylphenyl group, a 2,6-diethylphenyl group, a 2,6-di-n-propylphenyl group, a 2,6-diisopropylphenyl group, a 2,6-di-tert-butylphenyl group, a 2-isopropyl-6-methylphenyl group, a 3,5-dimethyl group, or a 2,4,6-trimethylphenyl group; alternatively, a 2-methylphenyl group, a 2-ethylphenyl group, a 2-n-propylphenyl group, a 2-isopropylphenyl group, or a 2-tert-butylphenyl group; alternatively, a 2,6-dimethylphenyl group, a 2,6-diethylphenyl group, a 2,6-di-n-propylphenyl group, a 2,6-diisopropylphenyl group, a 2,6-di-tert-butylphenyl group, or a 2-isopropyl-6-methylphenyl group; alternatively, a 2-methylphenyl group; alternatively, a 2-ethylphenyl group; alternatively, a 2-n-propylphenyl group; alternatively, a 2-isopropylphenyl group; alternatively, a 2-tert-butylphenyl group; alternatively, a 3-methylphenyl group; alternatively, a 2,6-dimethylphenyl group; alternatively, a 2,6-diethylphenyl group; alternatively, a 2,6-di-n-propylphenyl group; alternatively, a 2,6-diisopropylphenyl group; alternatively, a 2,6-di-tert-butylphenyl group; alternatively, a 2-isopropyl-6-methylphenyl group; alternatively, a 3,5-dimethylphenyl group; or alternatively, a 2,4,6-trimethylphenyl group. In some non-limiting embodiments, R¹, R², R³, and R⁴ independently can be a phenyl group, a 2-methoxyphenyl group, a 2-ethoxyphenyl group, a 2-isopropoxyphenyl group, a 2-tert-butoxyphenyl group, a 3-methoxyphenyl group, a 3-ethoxyphenyl group, a 3-isopropoxyphenyl group, a 3-tert-butoxyphenyl group, a 4-methoxyphenyl group, a 4-ethoxyphenyl group, a 4-isopropoxyphenyl group, a 4-tert-butoxyphenyl group, a 3,5-dimethoxyphenyl group, a 3,5-diethoxyphenyl group, a 3,5-diisopropoxyphenyl group, or a 3,5-di-tert-butoxyphenyl group; alternatively, a 2-methoxyphenyl group, a 2-ethoxyphenyl group, a 2-isopropoxyphenyl group, or a 2-tert-butoxyphenyl group; alternatively, 3-methoxyphenyl group, a 3-ethoxyphenyl group, a 3-isopropoxyphenyl group, or a 3-tert-butoxyphenyl group; alternatively, a 4-methoxyphenyl group, a 4-ethoxyphenyl group, a 4-isopropoxyphenyl group, or a 4-tert-butoxyphenyl group; or alternatively, a 3,5-dimethoxyphenyl group, a 3,5-diethoxyphenyl group, a 3,5-diisopropoxyphenyl group, or a 3,5-di-tert-butoxyphenyl group. In other non-limiting embodiments, R¹, R², R³, and R⁴ independently can be a 2-methoxyphenyl group; alternatively, a 2-ethoxyphenyl group; alternatively, a 2-isopropoxyphenyl group; alternatively, a 2-tert-butoxyphenyl group; alternatively, 3-methoxyphenyl group; alternatively, a 3-ethoxyphenyl group; alternatively, a 3-isopropoxyphenyl group; alternatively, a 3-tert-butoxyphenyl group; alternatively, a 4-methoxyphenyl group; alternatively, a 4-ethoxyphenyl group; alternatively, a 4-isopropoxyphenyl group; alternatively, a 4-tert-butoxyphenyl group; alternatively, a 3,5-dimethoxyphenyl group; alternatively, a 3,5-diethoxyphenyl group; alternatively, a 3,5-diisopropoxyphenyl group; or alternatively, a 3,5-di-tert-butoxyphenyl group.

In some embodiments, one or more of R¹, R², R³, and R⁴ can have Structure 2. 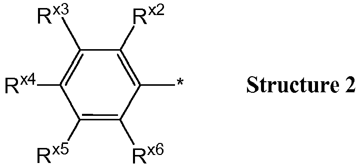 Within Structure 2 R^{x2}, R^{x3}, R^{x4}, R^{x5}, and R^{x6} independently can be hydrogen, a hydrocarbyl group, a hydrocarboxy group, or a halogen; alternatively, hydrogen, a hydrocarbyl group, or a halogen; alternatively, hydrogen, a hydrocarboxy group, or a halogen; alternatively, hydrogen, a hydrocarbyl group, or a hydrocarboxy group; alternatively, hydrogen or a hydrocarbyl group; alternatively, hydrogen or a hydrocarboxy group; or alternatively, hydrogoen or a halogen. Hydrocarbyl groups (general and specific), hydrocarboxy groups (general and specific), and halogens have be been independently described herein (e.g. as substitutents of a subsituted alkyl group, a substituted cycloalkyl group, a substituted aliphatic heterocyclyl group, a substituted aryl group, a substituted aralkyl group, and/or a substituted heteroaryl group and these hydrocarbyl groups (general and specific), hydrocarboxy groups (general and specific), and halogens can be utilized without limitation as R^{x2}, R^{x3}, R^{x4}, R^{x5}, and R^{x6} for a R¹, R², R³, and/or R⁴ group having Structure 2.

When the diphosphino aminyl moiety comprises more that one phenyl group, the substituents of one phenyl group can be independent of the substituents of one or more of the other phenyl groups. In this scenario, the substituent designations of each phenyl group can be represented by replacing the x within R group designations of Structure 2 with the number of the diphosphino aminyl moiety R group in Structure 1 which it represents. For example, if the R¹ group of the diphosphino aminyl moiety having Structure 1 is a phenyl group (or a substituted phenyl group), the designations of the R¹ phenyl group having Structure 2 would be represented by the designations R¹², R¹², R¹⁴, R¹⁵, and R¹⁶. Likewise, the designations of a R³ phenyl group (or substituted phenyl group) would be represented by the designations R³², R³³, R³⁴, R³⁵, and R³⁶.

In an embodiment, the diphosphino aminyl moiety can have Structure 3. 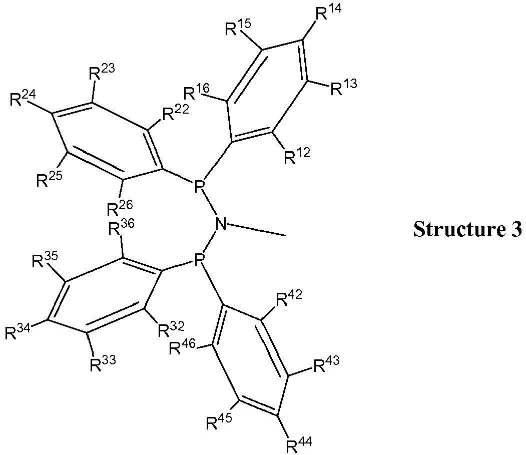 Generally, the substituents R¹², R¹³, R¹⁴, R¹⁵, and R¹⁶, R²², R²³, R²⁴, R²⁵, and R²⁶, R³², R³³, R³⁴, R³⁵, and R³⁶, and R⁴², R⁴³, R⁴⁴, R⁴⁵, and R⁴⁶ can independently have any combination of substituent(s) described herein and/or have any substituent pattern for the substituted phenyl groups as described herein.

In some non-limiting diphosphino aminyl moiety embodiments having Structure 3, R¹², R²², R³², and R⁴² are alkyl groups and R¹³, R¹⁴, R¹⁵, R¹⁶, R²³, R²⁴, R²⁵, R²⁶, R³³, R³⁴, R³⁵, R³⁶, R⁴³, R⁴⁴, R⁴⁵, and R⁴⁶ are hydrogen; alternatively, R¹², R²², and R⁴² are alkyl groups and R¹³, R¹⁴, R¹⁵, R¹⁶, R²³, R²⁴, R²⁵, R²⁶, R³², R³³ R³⁴, R³⁵, R³⁶, R⁴³, R⁴⁴, R⁴⁵, and R⁴⁶ are hydrogen; alternatively, R¹² and R⁴² are alkyl groups and R¹³ R¹⁴, R¹⁵, R¹⁶, R²², R²³, R²⁴, R²⁵, R²⁶, R³², R³³, R³⁴, R³⁵, R³⁶, R⁴³, R⁴⁴, R⁴⁵, and R⁴⁶ are hydrogen; alternatively, R¹² and R²², are alkyl groups and R¹³, R¹⁴, R¹⁵, R¹⁶, R²³, R²⁴, R²⁵, R²⁶, R³², R³³, R³⁴, R³⁵, R³⁶, R⁴², R⁴³, R⁴⁴, R⁴⁵, and R⁴⁶ are hydrogen; or alternatively, R¹² is an alkyl group and R¹³, R¹⁴, R¹⁵, R¹⁶, R²², R²³, R²⁴, R²⁵, R²⁶, R³², R³³, R³⁴, R³⁵, R³⁶, R⁴², R⁴³, R⁴⁴, R⁴⁵, and R⁴⁶ are hydrogen. In other diphosphino aminyl moiety embodiments having Structure 3, R¹⁴, R²⁴, R³⁴, and R⁴⁴ are alkyl groups and R¹², R¹³, R¹⁵, R¹⁶, R²², R²³, R²⁵, R²⁶, R³², R³³, R³⁵, R³⁶, R⁴², R³, R⁴⁵, and R⁴⁶ are hydrogen; alternatively, R¹⁴, R²⁴, and R⁴⁴ are alkyl groups and R¹², R¹³, R¹⁵, R¹⁶, R²², R²³, R²⁵, R²⁶, R³², R³³, R³⁴, R³⁵, R³⁶, R⁴², R⁴³ R⁴⁵, and R⁴⁶ are hydrogen; alternatively, R¹⁴ and R⁴⁴ are alkyl groups and R¹², R¹³, R¹⁵, R¹⁶, R²², R²³, R²⁴, R²⁵, R²⁶, R³², R³³, R³⁴, R³⁵, R³⁶, R⁴², R⁴³, R⁴⁵, and R⁴⁶ are hydrogen; alternatively, R¹⁴, and R²⁴ are alkyl groups and R¹², R¹³, R¹⁵, R¹⁶, R²², R²³, R²⁵, R²⁶, R³², R³³, R³⁴, R³⁵, R³⁶, R⁴², R⁴³, *R*⁴⁴*,* R⁴⁵, and R⁴⁶ are hydrogen; or alternatively, R¹⁴ is an alkyl group and R¹², R¹³, R15, R16, R²², R²³, R²⁴, R25, R²⁶, R³², R³³, R³⁴, R³⁵, R³⁶, R⁴², R⁴³, R⁴⁴, R⁴⁵, and R⁴⁶ are hydrogen. In yet other diphosphino aminyl moiety embodiments having Structure 3, R¹⁴, R²⁴, R³⁴, and R⁴⁴ are alkoxy groups and R¹², R¹³, R¹⁵, R¹⁶, R²², R²³, R²⁵, R²⁶, R³², R³³, R³⁵, R³⁶, R⁴², R³, R⁴⁵, and R⁴⁶ are hydrogen; alternatively, R¹⁴, R²⁴**,** and R⁴⁴ are alkoxy groups and R¹², R¹³, R¹⁵, R¹⁶, R²², R²³, R²⁵, R²⁶, R³², R³³, R³⁴, R³⁵, R³⁶, R⁴², R⁴³, R⁴⁵, and R⁴⁶ are hydrogen; alternatively, R¹⁴ and R⁴⁴ are alkoxy groups and R¹², R¹³, R¹⁵, R¹⁶, R²², R²³, R²⁴, R²⁵, R²⁶, R³², R³³, R³⁴, R³⁵, R³⁶, R⁴², R⁴³, R⁴⁵, and R⁴⁶ are hydrogen; alternatively, R¹⁴, and R²⁴ are etheryl groups and and R¹², R¹³, R¹⁵, R¹⁶, R²², R²³, R²⁵, R²⁶, R³², R³³, R³⁴, R³⁵, R³⁶, R⁴², R⁴³, R⁴⁴, R⁴⁵, and R⁴⁶are hydrogen; or alternatively, R¹⁴ is an alkoxy group and R¹², R¹³, R¹⁵, R¹⁶, R²², R²³, R²⁴, R²⁵, R²⁶, R³², R³³, R³⁴, R³⁵, R³⁶, R⁴², R⁴³, R⁴⁴, R⁴⁵, and R⁴⁶are hydrogen. In a further embodiment, R¹², R¹³, R¹⁴, R¹⁵, R¹⁶, R²², R²³, R²⁴, R²⁵, R²⁶, R³², R³³, R³⁴, R³⁵, R³⁶, R⁴² R⁴³, R⁴⁴, R⁴⁵, and R⁴⁶are hydrogen. In some non-limiting diphosphino aminyl moiety embodiments having Structure 3, R¹², R²², R³², and R⁴² are fluorines and R¹³, R¹⁴, R¹⁵, R¹⁶, R²³, R²⁴, R²⁵, R²⁶, R³³, R³⁴, R³⁵, R³⁶, R⁴³, R⁴⁴, R⁴⁵, and R⁴⁶ are hydrogen; alternatively, R¹², R²², and R⁴² are fluorines and R¹³, R¹⁴, R¹⁵, R¹⁶, R²³, R²⁴, R²⁵, R²⁶, R³², R³³, R³⁴, R³⁵, R³⁶, R⁴³, R⁴⁴, R⁴⁵, and R⁴⁶ are hydrogen; alternatively, R¹² and R⁴² are fluorines and R¹³, R¹⁴, R¹⁵, R¹⁶, R²², R²³, R²⁴, R²⁵, R ²⁶, R³², R³³, R³⁴, R³⁵, R³⁶, R⁴³, R⁴⁴, R⁴⁵, and R⁴⁶ are hydrogen; alternatively, R¹² and R²², are fluorines and R¹³, R¹⁴, R¹⁵, R¹⁶, R²³, R²⁴, R²⁵, R²⁶, R³², R³³, R³⁴, R³⁵, R³⁶, R⁴², R⁴³, R⁴⁴, R⁴⁵, and R⁴⁶ are hydrogen; or alternatively, R¹² is an fluorine and R¹³, R¹⁴, R¹⁵, R¹⁶, R²², R²³, R²⁴, R²⁵, R²⁶, R³², R³³, R³⁴, R³⁵, R³⁶, R⁴², R⁴³, R⁴⁴, R⁴⁵, and R⁴⁶ are hydrogen. In other diphosphino aminyl moiety embodiments having Structure 3, R¹⁴, R²⁴, R³⁴, and R⁴⁴ are fluorines and R¹², R¹³, R¹⁵, R¹⁶, R²², R²³, R²⁵, R²⁶, R³² R³³, R³⁵, R³⁶, R⁴², R³, R⁴⁵, and R⁴⁶ are hydrogen; alternatively, R¹⁴, R²⁴, and R⁴⁴ are fluorines and R¹², R¹³, R¹⁵, R¹⁶, R²², R²³, R²⁵, R²⁶, R³², R³³, R³⁴, R³⁵, R³⁶, R⁴², R⁴³, R⁴⁵, and R⁴⁶ are hydrogen; alternatively, R¹⁴ and R⁴⁴ are fluorines and R¹², R¹³, R¹⁵, R¹⁶, R²², R²³, R²⁴, R²⁵, R²⁶, R³², R³³, R³⁴, R³⁵, R³⁶, R⁴², R⁴³, R⁴⁵, and R⁴⁶ are hydrogen; alternatively, R¹⁴, and R²⁴ are fluorines and R¹², R¹³, R¹⁵, R¹⁶, R²², R²³, R²⁵, R²⁶, R³², R³³, R³⁴, R³⁵, R³⁶, R⁴², R⁴³, R⁴⁴, R⁴⁵, and R⁴⁶ are hydrogen; or alternatively, R¹⁴ is an fluorine and R¹², R¹³, R¹⁵, R¹⁶, R²², R²³, R²⁴, R²⁵, R²⁶, R³², R³³, R³⁴, R³⁵, R³⁶, R⁴², R⁴³, R⁴⁴, R⁴⁵, and R⁴⁶ are hydrogen. Alkyl group substituents (general and specific) and alkoxy group substituents (general and specific) have been described herein as substitutents of a subsituted alkyl group, a substituted cycloalkyl group, substituted aliphatic heterocyclyl group, substituted aryl group, and/or substituted heteroaryl group and these alkyl groups (general and specific) and alkoxy (general and specific) utilized for a R¹, R², R³, and/or R⁴ group and can be utilized, without limitation, to the diphosphino aminyl moiety substituent patterns having Structure 3 as described herein.

In a non-limiting embodiment, each diphosphino aminyl moiety of a diphosphino aminyl ligand can be (methyl₂P)₂N- (a bis(dimethylphosphino)aminyl group), (ethyl₂P)₂N- (a bis(diethylphosphino)aminyl group), (isopropyl₂P)₂N- (a bis(diisopropylphosphino)aminyl group), (*tert*-butyl-)P)₂N- (a bis(di-*tert-*butylphosphino)aminyl group), (neopentyl₂P)₂N- (a bis(dineopentylphosphino)aminyl group), (phenyl₂P)₂N-(a bis(diphenylphosphino)aminyl group) (2-naphthyl₂P)₂N- (a bis(di-2-naphthylphospino)aminyl group), or (4-biphenyl₂P)₂N- (a bis(di-4-biphenylphospino)aminyl group). In a non-limiting embodiment, the diphosphino aminyl moiety or moieties of the diphosphino aminyl ligand can be (phenyl₂P)₂N- (a bis(diphenylphospino)aminyl group).

In some non-limiting embodiments, each diphosphino aminyl moiety of a diphosphino aminyl ligand can be ((2-methylphenyl)₂P)₂N- (a bis(di(2-methylphenyl)phosphino)aminyl group), ((2-ethylphenyl)₂P)₂N- (a bis(di(2-ethylphenyl)phosphino)aminyl group), ((2-isopropylphenyl)₂P)₂N- (a bis(di(2-isopropylphenyl)phosphino)aminyl group), ((2-*tert*-butylphenyl)₂P)₂N - (a bis(di(2-*tert-*butylphenyl)phosphino)aminyl group), or ((2-neopentylphenyl)₂P)₂N- (a bis(di(2-neopentyl-phenyl)phosphino)aminyl group). In other non-limiting embodiments, each diphosphino aminyl moiety of diphosphino aminyl ligand can be ((4-methylphenyl)₂P)₂N- (a bis(di(4-methylphenyl)phosphino)aminyl group), ((4-ethylphenyl)₂P)₂N- (a bis(di(4-ethylphenyl)phosphino)aminyl group), ((4-isopropylphenyl)₂P)₂N- (a bis(di(4-isopropylphenyl)phosphino)aminyl group), ((4-*tert*-butylphenyl)₂P)₂N-(a bis(di(4-*tert*-butylphenyl)phosphino)aminyl group), or ((4-neopentylphenyl)₂P)₂N- (a bis(di(4-neopentyl-phenyl)phosphino)aminyl group).

In other non-limiting embodiments, each diphosphino aminyl moiety of a diphosphino aminyl ligand can be ((3-methoxyphenyl)₂P)₂N- (a bis(di(3-methoxyphenyl) phosphino)aminyl group), ((4-methoxyphenyl)₂P)₂N- (a bis(di(4-methoxyphenyl) phosphino)aminyl group), ((3-ethoxyphenyl)₂P)₂N- (a bis(di(3-ethoxyphenyl) phosphino)aminyl group), ((4-ethoxyphenyl)₂P)₂N- (a bis(di(4-ethoxyphenyl) phosphino)aminyl group), ((3-isopropoxyphenyl)₂P)₂N- (a bis(di(3-isopropoxyphenyl) phosphino)aminyl group), ((4-isopropoxyphenyl)₂P)₂N- (a bis(di(4-isopropoxyphenyl) phosphino)aminyl group), ((3-*tert-*butoxyphenyl)₂P)₂N- (a bis(di(3-*tert*-butoxyphenyl) phosphino)aminyl group), ((4-*tert*-butoxyphenyl)₂P)₂N-(a bis(di(4-*tert*-butoxyphenyl) phosphino)aminyl group). In a non-limiting embodiment, each diphosphino aminyl moiety of a diphosphino aminyl ligand can be ((3-methoxyphenyl)₂P)₂N- (a bis(di(3-methoxyphenyl) phosphino)aminyl group).

In yet other non-limiting embodiments, each diphosphino aminyl moiety of a diphosphino aminyl ligand can be ((2-thiophenyl)₂P)₂N- (a bis(di(2-thiophenyl)phosphino) aminyl group), ((3-thiophenyl)₂P)₂N- (a bis(di(3-thiophenyl)phosphino) aminyl group), ((3-ethyl-2-thiophenyl)₂P)₂N- (a bis (di(3-ethyl-2-thiophenyl)phosphino) aminyl group), ((2-ethyl-3-thiophenyl)₂P)₂N- (a bis(di(2-ethyl-3-thiophenyl)phosphino) aminyl group), ((2-pyridine)₂P)₂N- (a bis(di(2-pyridinyl) phosphino) aminyl group), ((3-pyridine)₂P)₂N- (a bis(di(3-pyridinyl) phosphino) aminyl group), ((4-pyridine)₂P)₂N- (a bis(di(4-pyridinyl) phosphino) aminyl group), or ((2-ethyl-4-pyridinyl)₂P)₂N- (a bis(di(2-ethyl-4-pyridinyl) phosphino aminyl group). In further non-limiting embodiments, each diphosphino aminyl moiety of a diphosphino aminyl ligand can be ((2-thiophenyl)₂P)₂N- (a bis(di(2-thiophenyl)phosphino) aminyl group); or alternatively, (2-pyridine)₂P)₂N- (a bis(di(2-pyridinyl) phosphino) aminyl group).

In an embodiment, the diphosphino aminyl ligand can have the formula R¹R²P-N(R⁵)-PR³R⁴ wherein the (R¹R²P) (PR³R⁴)N- represents the diphosphino aminyl moiety and R⁵ represent the remainder of the diphosphino aminyl ligand. The diphosphino aminyl moieties are described herein and can be utilized in any combination with any group R⁵ described herein to further describe a diphosphino aminyl ligand. The diphosphino aminyl ligands disclosed herein can be prepared using procedures known to one skilled in the art and procedures in published literature.

In an aspect, R⁵ can be an organyl group; alternatively, an organyl group consisting of inert functional groups; or alternatively, a hydrocarbyl group. In an embodiment, the organyl group which can be utilized as R⁵ can be a C₁ to C₃₀ organyl group; alternatively, a C₁ to C₂₀ organyl group; alternatively, a C₁ to C₁₅ organyl group; alternatively, a C₁ to C₁₀ organyl group; or alternatively, a C₁ to C₅ organyl group. In an embodiment, the organyl group consisting of inert functional groups which can be utilized as R⁵ can be a C₁ to C₃₀ organyl group consisting of inert functional groups; alternatively, a C₁ to C₂₀ organyl group consisting of inert functional groups; alternatively, a C₁ to C₁₅ organyl group consisting of inert functional groups; alternatively, a C₁ to C₁₀ organyl group consisting of inert functional groups; or alternatively, a C₁ to C₅ organyl group consisting of inert functional groups. In an embodiment, the hydrocarbyl group which can be utilized as R⁵ can be a C₁ to C₃₀ hydrocarbyl group; alternatively, a C₁ to C₂₀ hydrocarbyl group; alternatively, a C₁ to C₁₅ hydrocarbyl group; alternatively, a C₁ to C₁₀ hydrocarbyl group; or alternatively, a C₁ to C₅ hydrocarbyl group.

In some embodiments, R⁵ can be an alkyl group, a substituted alkyl group, a cycloalkyl group, a substituted cycloalkyl group, an aryl group, a substituted aryl group, an aralkyl group, or a substituted aralkyl group. In other embodiments, R⁵ can be an alkyl group or a substituted alkyl group; alternatively, a cycloalkyl group or a substituted cycloalkyl group; alternatively, an aryl group or a substituted aryl group; alternatively, an aralkyl group or a substituted aralkyl group; or alternatively, an alkyl group, a cycloalkyl group, an aryl group, or an aralkyl group. In yet other embodiments, R⁵ can be an alkyl group; alternatively, a substituted alkyl group, alternatively, a cycloalkyl group; alternatively, a substituted cycloalkyl group; alternatively, an aryl group; alternatively, a substituted aryl group; or alternatively, an aralkyl group.

In any aspect or embodiment disclosed herein, the alkyl group which can be utilized as R⁵ can be a C₁ to C₃₀ alkyl group; alternatively, a C₁ to C₂₀ alkyl group; alternatively, a C₁ to C₁₀ alkyl group; or alternatively, C₁ to C₅ alkyl group. In any aspect or embodiment disclosed herein, the substituted alkyl group which can be utilized as R⁵ can be a C₁ to C₃₀ substituted alkyl group; alternatively, a C₁ to C₂₀ substituted alkyl group; alternatively, a C₁ to C₁₀ substituted alkyl group; or alternatively, C₁ to C₅ substituted alkyl group. In any aspect or embodiment disclosed herein, the cycloalkyl group which can be utilized as R⁵ can be a C₄ to C₃₀ cycloalkyl group; alternatively, a C₄ to C₂₀ cycloalkyl group; alternatively, a C₄ to C₁₅ cycloalkyl group; or alternatively, C₄ to C₁₀ cycloalkyl group. In any aspect or embodiment disclosed herein, the substituted cycloalkyl group which can be utilized as R⁵ can be a C₄ to C₃₀ substituted cycloalkyl group; alternatively, a C₄ to C₂₀ substituted cycloalkyl group; alternatively, a C₄ to C₁₅ substituted cycloalkyl group; or alternatively, a C₄ to C₁₀ substituted cycloalkyl group. In any aspect or embodiment disclosed herein, the aryl group which can be utilized as R⁵ can be a C₆ to C₃₀ aryl group; alternatively, a C₆ to C₂₀ aryl group; alternatively, a C₆ to C₁₅ aryl group; or alternatively, C₆ to C₁₀ aryl group. In any aspect or embodiment disclosed herein, the substituted aryl group which can be utilized as R⁵ can be a C₆ to C₃₀ substituted aryl group; alternatively, a C₆ to C₂₀ substituted aryl group; alternatively, a C₆ to C₁₅ substituted aryl group; or alternatively, a C₆ to C₁₀ substituted aryl group. In any aspect or embodiment disclosed herein, each aralkyl group which can be utilized as R⁵ can be a C₇ to C₃₀ aralkyl group; alternatively, a C₇ to C₂₀ aralkyl group; alternatively, a C₇ to C₁₅ aralkyl group; or alternatively, C₇ to C₁₀ aralkyl group. In any aspect or embodiment disclosed herein, the substituted aryl group which can be utilized as R⁵ can be a C₇ to C₃₀ substituted aralkyl group; alternatively, a C₇ to C₂₀ substituted aralkyl group; alternatively, a C₇ to C₁₅ aralkyl group; or alternatively, C₇ to C₁₀ substituted aralkyl group. Each substituent of a substituted alkyl group (general or specific), substituted cycloalkyl group (general or specific), substituted aryl group (general or specific), and/or substituted aralkyl group (general or specific) can be a halogen, hydrocarbyl group, or a hydrocarboxy group; alternatively, a halogen or a hydrocarbyl group; alternatively, a halogen or a hydrocarboxyl group; alternatively, a hydrocarbyl group or a hydrocarboxy group; alternatively, a halogen; alternatively, a hydrocarbyl group; or alternatively, a hydrocarboxy group. Substituent halogens, hydrocarbyl groups, and substituent hydrocarboxy groups are independently disclosed herein (e.g. as potential substitutents of a subsituted alkyl group, a substituted cycloalkyl group, a substituted aliphatic heterocyclyl group, a substituted aryl group, a substituted aralkyl group, and/or a substituted heteroaryl group utilized for a R¹, R², R³, and/or R⁴ group). These substituent halogens, substitutent hydrocarbyl groups, and substituent hydrocarboxy groups can be utilized without limitation to further describe a substituted R⁵ group.

In an embodiment, R⁵ can be a methyl group, an ethyl group, a propyl group, a butyl group, a pentyl group, a hexyl group, a heptyl group, an octyl group, a nonyl group, a decyl group, a undecyl group, a dodecyl group, a tridecyl group, a tetradecyl group, a pentadecyl group, a hexadecyl group, a heptadecyl group, an octadecyl group, or a nonadecyl group; or alternatively, a methyl group, an ethyl group, a propyl group, a butyl group, a pentyl group, a hexyl group, a heptyl group, an octyl group, a nonyl group, a decyl group. In some embodiments, R⁵ can be a methyl group, an ethyl group, an n-propyl (1-propyl) group, an isopropyl (2-propyl) group, an n-butyl (1-butyl) group, a *sec*-butyl (2-butyl) group, an isobutyl (2-methyl-1-propyl) group, a *tert*-butyl (2-methyl-2-propyl) group, an n-pentyl (1-pentyl) group, a 2-pentyl group, a 3-pentyl group, a 2-methyl-1-butyl group, a *tert*-pentyl (2-methyl-2-butyl) group, a 3-methyl-1-butyl group, a 3-methyl-2-butyl group, or a neo-pentyl (2,2-dimethyl-1-propyl) group; alternatively, a methyl group, an ethyl group, an iso-propyl (2-propyl) group, a tert-butyl (2-methyl-2-propyl) group, or a neopentyl (2,2-dimethyl-1-propyl) group; alternatively, a methyl group; alternatively, an ethyl group; alternatively, a n-propyl (1-propyl) group; alternatively, an iso-propyl (2-propyl) group; alternatively, a tert-butyl (2-methyl-2-propyl) group; or alternatively, a neopentyl (2,2-dimethyl-1-propyl) group. In some embodiments, the alkyl groups which can be utilized as R⁵ can be substituted. Each substituent of a substituted alkyl group independently can be a halogen or a hydrocarboxy group; alternatively, a halogen; or alternatively, a hydrocarboxy group. Substituent halogens and substituent hydrocarboxy groups (general and specific) are independently described herein and these substitutent groups can be utilized without limitation to further describe a substituted alkyl group which can be utilized as R⁵.

In an aspect, R⁵ can be a cyclobutyl group, a substituted cyclobutyl group, a cyclopentyl group, a substituted cyclopentyl group, a cyclohexyl group, a substituted cyclohexyl group, a cycloheptyl group, a substituted cycloheptyl group, a cyclooctyl group, or a substituted cyclooctyl group. In some embodiments, R⁵ can be a cyclopentyl group, a substituted cyclopentyl group, a cyclohexyl group, a substituted cyclohexyl group. In other embodiments, R⁵ can be a cyclobutyl group or a substituted cyclobutyl group; alternatively, a cyclopentyl group or a substituted cyclopentyl group; alternatively, a cyclohexyl group or a substituted cyclohexyl group; alternatively, a cycloheptyl group or a substituted cycloheptyl group; or alternatively, a cyclooctyl group, or a substituted cyclooctyl group. In yet other embodiments, R⁵ can be a substituted cyclobutyl group, a substituted cyclopentyl group, a substituted cyclohexyl group, a substituted cycloheptyl group, or a substituted cyclooctyl group; or alternatively, a substituted cyclopentyl group or a substituted cyclohexyl group. In further embodiments, R⁵ can be a cyclopentyl group; alternatively, a substituted cyclopentyl group; a cyclohexyl group; or alternatively, a substituted cyclohexyl group. In further embodiments, R⁵ can be a 2-substituted cyclohexyl group, a 2,6-disubstituted cyclohexyl group, a 2-subsituted cyclopentyl group, or a 2,5-disubstituted cyclopentyl group; alternatively, a 2-substituted cyclohexyl group or a 2,6-disubstituted cyclohexyl group; alternatively, a 2-subsituted cyclopentyl group or a 2,5-disubstituted cyclopentyl group; alternatively, a 2-substituted cyclohexyl group or a 2-subsituted cyclopentyl group; alternatively, a 2,6-disubstituted cyclohexyl group or a 2,5-disubstituted cyclopentyl group; alternatively, a 2-substituted cyclohexyl group; alternatively, a 2,6-disubstituted cyclohexyl group; alternatively, a 2-subsituted cyclopentyl group; or alternatively, a 2,5-disubstituted cyclopentyl group. Each substituent of a cycloalkyl group having a specified number of ring carbon atoms independently can be a halogen, a hydrocarbyl group, or a hydrocarboxy group; alternatively, a halogen or a hydrocarbyl group; alternatively, a halogen or a hydrocarboxy group; alternatively, a hydrocarbyl group or a hydrocarboxy group; alternatively, a halogen, alternatively, a hydrocarbyl group; or alternatively, a hydrocarboxy group. Substituent halogens, substitutent hydrocarbyl group (general and specific), and substituent hydrocarboxy groups (general and specific) are independently described herein and these substitutent groups can be utilized without limitation to further describe a substituted cycloalkyl group (general or specific) which can be utilized as R⁵.

In an embodiment, R⁵ can be a phenyl group or a substituted phenyl group; alternatively, a phenyl group; or alternatively, a substituted phenyl group. In some embodiments, R⁵ can be a 2-substituted phenyl group, a 3-substituted phenyl group, a 4-substituted phenyl group, a 2,4-disubstituted phenyl group, a 2,6-disubstituted phenyl group, a 3,5-disubstituted phenyl group, or a 2,4,6-trisubstituted phenyl group. In other embodiments, R⁵ independently can be a 2-substituted phenyl group, a 4-substituted phenyl group, a 2,4-disubstituted phenyl group, or a 2,6-disubstituted phenyl group; alternatively, a 3-subsituted phenyl group or a 3,5-disubstituted phenyl group; alternatively, a 2-substituted phenyl group or a 4-substituted phenyl group; alternatively, a 2,4-disubstituted phenyl group, a 2,6-disubstituted phenyl group, or a 2,4,6-trisubstituted phenyl group; alternatively, a 2,4-disubstituted phenyl group or a 2,6-disubstituted phenyl group;alternatively, a 2-substituted phenyl group; alternatively, a 3-substituted phenyl group; alternatively, a 4-substituted phenyl group; alternatively, a 2,4-disubstituted phenyl group; alternatively, a 2,6-disubstituted phenyl group; alternatively, a 3,5-disubstituted phenyl group; or alternatively, a 2,4,6-trisubstituted phenyl group. Each substituent of a substituted phenyl group (general or specific) independently can be a halogen, a hydrocarbyl group, or a hydrocarboxy group; alternatively, a halogen or a hydrocarbyl group; alternatively, a halogen or a hydrocarboxy group; alternatively, a hydrocarbyl group or a hydrocarboxy group; alternatively, a halogen, alternatively, a hydrocarbyl group; or alternatively, a hydrocarboxy group. Substituent halogens, substituent hydrocarbyl groups(general and specific), and substituent hydrocarboxy groups (general and specific) are independently described herein and these substitutent groups can be utilized without limitation to further describe a substituted phenyl group (general or specific) which can be utilized as R⁵.

In an aspect, R⁵ can be a cycloalkyl group or a substituted cycloalkyl group. In an embodiment, R⁵ can be a cycloalkyl group; or alternatively, a substituted cycloalkyl group. In some embodiments, R⁵ can be a cyclobutyl group, a substituted cyclobutyl group, a cyclopentyl group, a substituted cyclopentyl group, a cyclohexyl group, a substituted cyclohexyl group, a cycloheptyl group, a substituted cycloheptyl group, a cyclooctyl group, or a substituted cyclooctyl group; or alternatively, a cyclopentyl group, a substituted cyclopentyl group, a cyclohexyl group, or a substituted cyclohexyl group. In other embodiments, R⁵ can be a cyclopentyl group, a cyclohexyl group, or a cycloheptyl group; or alternatively, a substituted cyclopentyl group, a substituted cyclohexyl group, or a substituted cycloheptyl group. In further embodiments, R⁵ can be a cyclopentyl group or a substituted cyclopentyl group; or alternatively, a cyclohexyl group or a substituted cyclohexyl group. In yet other embodiments, R⁵ can be a cyclopentyl group; alternatively, a substituted cyclopentyl group; alternatively, a cyclohexyl group; or alternatively, a substituted cyclohexyl group. Within substituted cycloalkyl group embodiments of R⁵, the cycloalkyl group substituents can be an organyl group; alternatively, an organyl group consisting of inert functional groups; alternatively, a hydrocarbyl group; or alternatievly, an inert functional group. In some embodiments, the cycloalkyl group substituents can be an alkyl group. In an embodiment, the substituted cycloalkyl group comprises an hydrocarbyl substituent, or alternatively an alkyl substituent, located on a carbon atom adjacent to (i.e. attached to) a carbon atom attached to the aminyl nitrogen atom of the diphosphino aminyl moiety. In some embodiments, the substituted cycloalkyl group comprises only one hydrocarbyl substituent (or alternatively, alkyl substituent) located on a carbon atom adjacent to a carbon atom attached to the aminyl nitrogen atom of the diphosphino aminyl moiety. In other embodiments, the substituted cycloalkyl group consists of only one hydrocarbyl subsitutent (or alternatively, alkyl substituent) located on a carbon atom adjacent to the carbon atom attached to an aminyl nitrogen atom of the diphosphino aminyl moiety.

In an embodiment, the alkyl substituents of the substituted cycloalkyl R⁵ group can have can have from 1 to 15 carbon atoms; alternatively, from 1 to 10 carbon atoms; or alternatively, from 1 to 5 carbon atoms. In some embodiments, the alkyl substituents of the substituted cycloalkyl R⁵ group can be a methyl, ethyl, n-propyl (1-propyl), isopropyl (2-propyl), n-butyl (1-butyl), *sec*-butyl (2-butyl), isobutyl (2-methyl-1-propyl), *tert*-butyl (2-methyl-2-propyl), n-pentyl (1-pentyl), 2-pentyl, 3-pentyl, 2-methyl-1-butyl, *tert*-pentyl (2-methyl-2-butyl), 3-methyl-1-butyl, 3-methyl-2-butyl, neo-pentyl (2,2-dimethyl-1-propyl), or n-hexyl (1-hexyl) group. In other embodiments, the alkyl substituents of the substituted cycloalkyl R⁵ group can be a methyl, ethyl, n-propyl (1-propyl), n-butyl (1-butyl), isobutyl (2-methyl-1-propyl), n-pentyl (1-pentyl), 2-methyl-1-butyl, 3-methyl-1-butyl, neo-pentyl (2,2-dimethyl-1-propyl), or n-hexyl (1-hexyl) group. In yet other embodiments, the alkyl substituent(s) of the substituted cycloalkyl R⁵ group can be a methyl group; alternatively, an ethyl group; alternatively, an isopropyl group; or alternatively, a *tert*-butyl group.

In an embodiment, R⁵ can be a cyclopentyl group, a 2-methylcyclopentyl group, a cyclohexyl group, a 2-methylcyclohexyl group, a cycloheptyl group, or a 2-methylcycloheptyl group. In some embodiments, R⁵ can be a cyclopentyl group, a cyclohexyl group, or a cycloheptyl group. In other embodiments, R⁵ can be a 2-methylcyclopentyl group, a 2-methylcyclohexyl group, or a 2-methylcycloheptyl group. In yet other embodiments, R⁵ can be a cyclopentyl group; alternatively, a 2-methylcyclopentyl group; cyclohexyl group; or alternatively, a 2-methylcyclohexyl group.

In an aspect, R⁵ can have Structure 4: 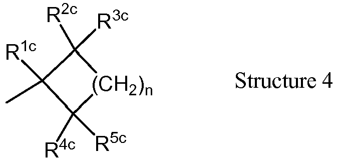 wherein undesignated valency is attached to the aminyl nitrogen atom of the diphosphino aminyl group. In an embodiment, R^{1c}, R^{2c}, R^{3c}, R^{4c}, and R^{5c} of Structure 4 can independently be hydrogen or a hydrocarbyl group; alternatively, hydrogen or an organyl group; alternatively, hydrogen or an organyl group consisting of inert functional groups; or alternatively, hydrogen or an inert functional group. In some embodiments, n can be an integer ranging from 1 to 5; or alternatively, n can be an integer ranging from 2 to 4. In some embodiments, n can be 2; alternatively, 3; or alternatively, 4. In an embodiment, R^{1c}, R^{2c}, R^{3c}, R^{4c}, and R^{5c} can independently be hydrogen, or an alkyl group. Within Structure 4, the substituents R^{1c}, R^{2c}, R^{3c}, R^{4c}, and R^{5c} can be any substituted cycloalkyl R⁵ group described herein and/or have any substituent pattern as described herein. In some embodiments, R^{2c} can be a hydrocarbyl group (or alternatively, an alkyl group) and R^{1c}, R^{3c}, R^{4c}, and R^{5c} are hydrogen.

In an embodiment, the diphosphino aminyl ligand can have Structure I. 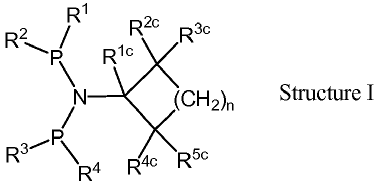 Generally, R¹, R², R³, and R⁴ can independently be any group as described herein. In an embodiment, R^{1c}, R^{2c}, R^{3c}, R^{4c}, and R^{5c} can independently be any cycloalkyl substituent described herein. In an embodiment, R^{1c}, R^{2c}, R^{3c}, R^{4c}, and R^{5c} can have any cycloalkyl substituent pattern described herein for the R⁵ group having Structure 4. In an embodiment, and n can have any value described herein for the R⁵ group having Structure 4. In some embodiments, the diphosphino aminyl ligands can have Structure I wherein R¹, R², R³, and R⁴ can independently be any group as described herein, R^{1c}, R^{2c}, R^{3c}, R^{4c}, and R^{5c} can have any substituent pattern comprising a hydrocarbyl subsitutent (or alternatively, an alkyl substituent) located on a carbon atom adjacent to the carbon atom attached to an aminyl nitrogen atom of the diphosphino aminyl moiety, and n can have any value described herein for the R⁵ group having Structure 4. In other embodiments, the diphosphino aminyl ligands can have Structure I wherein R¹, R², R³, and R⁴ can independently be any group as described herein, R^{1c}, R^{2c}, R^{3c}, R^{4c}, and R^{5c} can have any substituent pattern comprising only one hydrocarbyl subsitutent (or alternatively, alkyl substituent) located on a carbon atom adjacent to the carbon atom attached to an aminyl nitrogen atom of the diphosphino aminyl moiety, and n can have any value described herein for the R⁵ group having Structure 4. In yet other embodiments, the diphosphino aminyl ligands can have Structure I wherein R¹, R², R³, and R⁴ can independently be any group as described herein, R^{2c} is an hydrocarbyl subsitutent (or alternatively, alkyl substituent), R^{1c}, R^{3c}, R^{4c}, and R^{5c} are hydrogen, and n can have any value described herein for the R⁵ group having Structure 4.

In an embodiment, the diphosphino aminyl ligand can have Structure II: 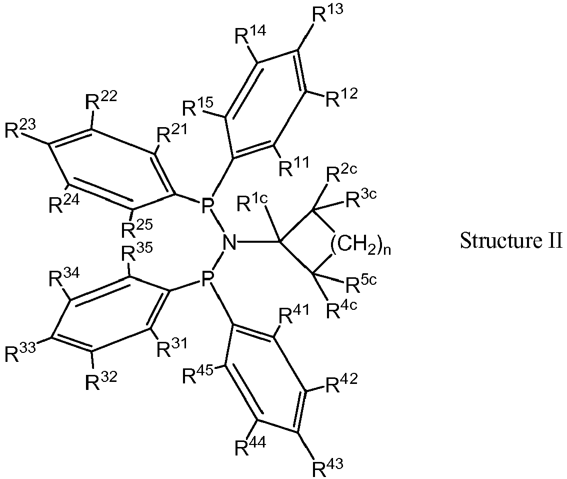 wherein the R¹¹, R¹², R¹³, R¹⁴, and R¹⁵, R²¹, R²², R²³, R²⁴, and R²⁵, R³¹, R³², R³³, R³⁴, and R³⁵, R⁴¹, R⁴², R⁴³, R⁴⁴, and R⁴⁵ can be any substituent described herein and/or have any substituent pattern described herein for the diphosphino aminyl moiety having Structure 3, R^{1c}, R^{2c}, R^{3c}, R^{4c}, and R^{5c} can be any cycloalkyl substituent described herein and/or have any cycloalkyl substituent pattern described herein for the R⁵ group having Structure 4, and n can have any value described herein for the R⁵ group having Structure 4.

In some embodiments, the diphosphino aminyl ligand can have Structure II wherein, R¹¹, R¹², R¹³, R¹⁴, and R¹⁵, R²¹, R²², R²³, R²⁴, and R²⁵, R³¹, R³², R³³, R³⁴, and R³⁵, R⁴¹, R⁴², R⁴³, R⁴⁴, and R⁴⁵ can independently be any substituent described herein and/or have any substituent pattern described herein for the diphosphino aminyl moiety having Structure 3, R^{1c}, R^{2c}, R^{3c}, R^{4c}, and R^{5c} can have any substituent pattern comprising a hydrocarbyl substituent (or alternatively, an alkyl substituent) located on a carbon atom adjacent to a carbon atom attached to an aminyl nitrogen atom of the diphosphino aminyl moiety, and n can have any value described herein for the R⁵ group having Structure 4. In other embodiments, the diphosphino aminyl ligand can have Structure II wherein R¹¹, R¹², R¹³, R¹⁴, and R¹⁵, R²¹, R²², R²³, R²⁴, and R²⁵, R³¹, R³², R³³, R³⁴, and R³⁵, R⁴¹, R⁴², R⁴³, R⁴⁴, and R⁴⁵ can independently be any substituent described herein and/or have any substituent pattern described herein for the diphosphino aminyl moiety having Structure 3, R^{1c}, R^{2c}, R^{3c}, R^{4c}, and R^{5c} can have any substituent pattern comprising only one hydrocarbyl substituent (or alternatively, alkyl substituent) located on a carbon atom adjacent to the carbon atom attached to an aminyl nitrogen atom of the diphosphino aminyl moiety, and n can have any value described herein for the R⁵ group having Structure 4. In yet other embodiments, the diphosphino aminyl ligand can have Structure II wherein R¹¹, R¹², R¹³, R¹⁴, and R¹⁵, R²¹, R²², R²³, R²⁴, and R²⁵, R³¹, R³², R³³, R³⁴, and R³⁵, R⁴¹, R⁴², R⁴³, R⁴⁴, and R⁴⁵ can independently be any substituent described herein and/or have any substituent pattern described herein for the diphosphino aminyl moiety having Structure 3, R^{2c} is hydrocarbyl substituent (or alternatively, an alkyl substituent), R^{1c}, R^{3c}, R^{4c}, and R^{5c} are hydrogen, and n can have any value described herein for the R⁵ group having Structure 4.

In an non-limiting embodiment, the diphosphino aminyl ligand can be (methyl₂P)₂N-(2-methylcyclohexyl), (ethyl₂P)₂N-(2-methylcyclohexyl), (isopropyl₂P)₂N-(2-methylcyclohexyl), (*tert-*butyl₂P)₂N-(2-methylcyclohexyl), (neopentyl₂P)₂N-(2-methylcyclohexyl), (phenyl₂P)₂N-(2-methyl-cyclohexyl), (2-naphthyl₂P)₂N-(2-methylcyclohexyl), or (4-biphenyl₂P)₂N-(2-methylcyclohexyl). In a non-limiting embodiment, the diphosphino aminyl ligand can be (phenyl₂P)₂N-(2-methylcyclohexyl).

In some non-limiting embodiments, the diphosphino aminyl ligand can be ((2-methylphenyl)₂P)₂N-(2-methylcyclohexyl), ((2-ethylphenyl)₂P)₂N-(2-methylcyclohexyl), ((2-isopropyl-phenyl)₂P)₂N-(2-methylcyclohexyl), ((2-*tert*-butylphenyl)₂P)₂N-(2-methylcyclohexyl), or ((2-neopentyl-phenyl)₂P)₂N-(2-methylcyclohexyl). In other non-limiting embodiments, the diphosphino aminyl ligand can be ((4-methylphenyl)₂P)₂N-(2-methylcyclohexyl), ((4-ethylphenyl)₂P)₂N-(2-methylcyclohexyl), ((4-isopropylphenyl)₂P)₂N-(2-methylcyclohexyl), ((4-*tert*-butylphenyl)₂P)₂N-(2-methylcyclohexyl), or ((4-neopentylphenyl)₂P)₂N-(2-methylcyclohexyl). In further embodiments, the diphosphino aminyl ligand can be ((2-methylphenyl)₂P)₂N-(2-methylcyclohexyl); or alternatively, ((4-methylphenyl)₂P)₂N-(2-methyl-cyclohexyl).

In a non-limiting embodiment, the diphosphino aminyl ligand can be ((3-methoxyphenyl)₂P)₂N-(2-methylcyclohexyl), ((4-methoxyphenyl)₂P)₂N-(2-methylcyclohexyl), ((3-ethoxyphenyl)₂P)₂N-(2-methylcyclohexyl), ((4-ethoxyphenyl)₂P)₂N-(2-methylcyclohexyl), ((3-isopropoxyphenyl)₂P)₂N-(2-methylcyclohexyl), ((4-isopropoxyphenyl)₂P)₂N-(2-methylcyclohexyl), ((3-*tert*-butoxyphenyl)₂P)₂N-(2-methylcyclohexyl), or ((4-*tert*-butoxyphenyl)₂P)₂N-(2-methylcyclohexyl). In some non-limiting embodiments, the diphosphino aminyl ligand can be ((3-methoxyphenyl)₂P)₂N-(2-methylcyclohexyl); or alternatively, ((4-methoxyphenyl)₂P)₂N-(2-methylcyclohexyl).

In a non-limiting embodiment, the diphosphino aminyl ligand can be ((2-thiophenyl)₂P)₂N-(2-methylcyclohexyl), ((3-thiophenyl)₂P)₂N-(2-methylcyclohexyl), ((3-ethyl-2-thiophenyl)₂P)₂N-(2-methylcyclohexyl), ((2-ethyl-3-thiophenyl)₂P)₂N-(2-methylcyclohexyl), ((2-pyridine)₂P)₂N-(2-methyl-cyclohexyl), ((3-pyridine)₂P)₂N-(2-methylcyclohexyl), ((4-pyridine)₂P)₂N-(2-methylcyclohexyl), or ((2-ethyl-4-pyridinyl)₂P)₂N-(2-methylcyclohexyl). In other non-limiting embodiments, the diphosphino aminyl ligand can be ((2-thiophenyl)₂P)₂N-(2-methylcyclohexyl); or alternatively, (2-pyridine)₂P)₂N-(2-methylcyclohexyl).

In a non-limiting embodiment, the diphosphino aminyl ligands can be represented by Structure (I) wherein R₁, R₂, R₃, and R₄ are phenyl groups and R⁵ is a methylcyclohexyl group (Structure III).

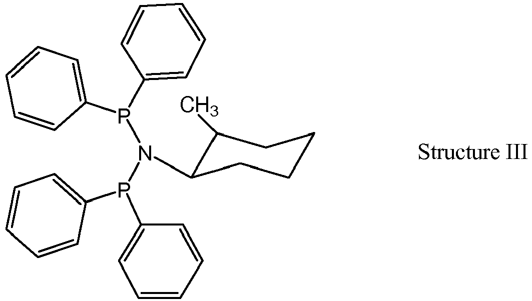

In an aspect, the the heteroatomic ligand can comprise at least one diphosphino aminyl moiety; or alternatively, comprise only one diphosphino aminyl moiety. In an aspect, the heteroatomic ligand can comprise multiple diphosphino aminyl moieties. In a non-limiting embodiment, the heteroatomic ligand can comprise at least 2 diphosphino aminyl moieties; alternatively, from 2 to 5 diphosphino aminyl moieties; or alternatively, from 2 to 3 diphosphino aminyl moieties. In an embodiment, the heteroatomic ligand comprises only 2 diphosphino aminyl moieties.

In some embodiments, the heteroatomic ligand comprising multiple diphosphino aminyl moieties further comprises a linking group, L, linking the aminyl nitrogen atoms of the diphosphino aminyl moieties. In some embodiments, the heteroatomic ligand comprises at least 2 diphosphino aminyl moieties (or any other number of diphosphino aminyl moieties described herein) and a linking group linking the aminyl nitrogen atoms of the diphosphino aminyl moieties. In other embodiments, the heteroatomic ligand comprises only 2 diphosphino aminyl moieties and a linking group linking the aminyl nitrogen atoms of the 2 diphosphino aminyl moieties. Hereafter the heteroatomic ligand comprising multiple diphosphino aminyl moieties and a linking group, L, linking the aminyl nitrogen atoms of the diphosphino aminyl moieties can denoted as (PNP)qL wherein PNP represent a diphosphino aminyl moiety, q represents the number of PNP moieties present in the heteroatomic ligand and L represents the linking group linking the aminyl nitrogen atoms of the diphosphino aminyl moieties. In an embodiment, q is greater than or equal to 2; alternatively, q ranges from 2 to 5; alternatively, q ranges from 2 to 3; or alternatively, q is exactly 2. The diphosphino aminyl moieties have been described herein and can be used without limitation and/or in any combination within the descriptions of the heteroatomic ligand containing multiple diphosphino aminyl moieties.

In an embodiment, the linking group, L, can be an organic group; alternatively, an organic group consisting of inert functional groups; or alternatively a hydrocarbon group. The organic linking group, organic linking group consisting of inert functional group, or hydrocarbon linking group can have from 1 to 50 carbon atoms; alternatively from 2 to 30 carbon atoms; or alternatively, from 2 to 20 carbon atoms.

In an embodiment, the linking group, linking the aminyl nitrogen atoms of the diphosphino aminyl moieties, can be acyclic. In some embodiments, the linking group, linking the aminyl nitrogen atoms of the diphosphino aminyl moieties, can comprise a cyclic group (i.e. a ring). In other embodiments, the linking group, linking the aminyl nitrogen atoms of the diphosphino aminyl moieties, can comprise at least one cyclic group; alternatively, at least two cyclic groups; alternatively, from 1 to 5 cyclic groups; alternatively, from 1 to 3 cyclic groups; or alternatively, from 1 to 2 cyclic groups. In further embodiments, the linking group, linking the aminyl nitrogen atoms of the diphosphino aminyl moieties, can comprise only one cyclic group; or alternatively, can comprise only two cyclic groups.

Generally, with exception of the presence of the groups containing the diphosphino aminyl moieties, the linking group, linking the aminyl nitrogen atoms of the diphosphino aminyl moieties, can comprise an unsubstituted cyclic group(s); or alternatively, can comprise a substituted cyclic group(s) (i.e. have moieties attached to a ring carbon which do not comprise a diphosphino aminyl moiety). In some embodiments wherein the linking group linking the aminyl nitrogen atoms of the diphosphino aminyl moieties comprises a cyclic group, the linking group can comprise a saturated cyclic group; alternatively, a substituted saturated cyclic group; alternatively, a saturated heteroatomic cyclic group; alternatively, a substituted saturated heteroatomic cyclic group; alternatively, an arene group; alternatively, a substituted arene group; alternatively, an aromatic cyclic group; alternatively, a substituted aromatic cyclic group; alternatively, a heteroaromatic cyclic group; or alternatively, a substituted heteroaromatic cyclic group. In an embodiment wherein the linking group linking the aminyl nitrogen atoms of the diphosphino aminyl moieties is a hydrocarbon linking group, the hydrocarbon linking group can comprise a saturated cyclic group; alternatively, a substituted saturated cyclic group; alternatively, an aromatic cyclic group; or alternatively, a substituted aromatic cyclic group.

In an embodiment wherein the linking group comprises a cyclic group, the aminyl nitrogen atom of the diphosphino aminyl moiety can be attached to a ring carbon of the linking group. In an embodiment wherein the diphosphino aminyl ligand comprises two or more diphosphino aminyl moieties, the linking group can comprise at least one cyclic group (or any other number of cyclic groups described herein) and the aminyl nitrogen atom of at least one of the diphosphino aminyl moieties can be attached to a ring carbon of the linking group. In another embodiment wherein the diphosphino aminyl ligand comprises two or more diphosphino aminyl moieties, the linking group can comprise at least one cyclic group (or any other number of cyclic groups described herein) and the aminyl nitrogen atoms of each diphosphino aminyl moiety can be attached to a ring carbon of the linking group. In some embodiments wherein the diphosphino aminyl ligand comprises two or more diphosphino aminyl moieties, the linking group can comprise at least one cyclic group (or any other number of cyclic groups described herein) and the aminyl nitrogen atoms of two or more of the diphosphino aminyl moieties can be attached to a ring carbon of the same cyclic group of the linking group.

In other embodiments wherein the diphosphino aminyl ligand comprises two or more diphosphino aminyl moieties, the linking group can comprise at least one cyclic group (or any other number of cyclic groups described herein) and the aminyl nitrogen atoms of each of the diphosphino aminyl moieties can be attached to a ring carbon of a different cyclic group of the linking group. In other embodiments wherein the diphosphino aminyl ligand comprises two or more diphosphino aminyl moieties, the linking group can comprise at least the same number of cyclic groups as there are diphosphino aminyl moieties and the aminyl nitrogen atoms of each diphosphino aminyl moiety can be attached to a ring carbon of a different cyclic group of the linking group. In yet other embodiments wherein the diphosphino aminyl ligand comprises two or more diphosphino aminyl moieties, the linking group can comprise the same number of cyclic groups as there are diphosphino aminyl moieties and the aminyl nitrogen atoms of each diphosphino aminyl moiety can be attached to a ring carbon of a different cyclic group of the linking group. Generally, each cyclic group of the linking group linking the aminyl nitrogen atoms of the diphosphino aminyl moieties can independently be an unsubstituted cyclic group or a substituted cyclic group(s). Furthermore, each cyclic groups of the linking group linking the aminyl nitrogen atoms of the diphosphino aminyl moieties can independently be any cyclic group or type of cyclic group (e.g. saturated cyclic, saturated heteroatomic, aromatic, or heteroaromatic) described herein.

In an embodiment, a linking group having at least one aminyl nitrogen atom of the diphosphino aminyl moiety attached to a ring carbon can comprise at least one hydrocarbyl substituent (or alternatively, alkyl substituent) located on a carbon atom adjacent to a carbon atom on which the aminyl nitrogen of the diphosphino aminyl moiety is attached. In other embodiments, a linking group having at least one aminyl nitrogen atom of the diphosphino aminyl moiety attached to ring carbon can comprise only one hydrocarbyl substituent (or alternatively, alkyl substituent) located on a carbon atom adjacent to each carbon atom on which the aminyl nitrogen atom of the diphosphino aminyl moiety is attached.

In an embodiment, a linking group having the aminyl nitrogen atom of each diphosphino aminyl moiety attached to a ring carbon can comprise at least one alkyl substituent located on a carbon atom adjacent to a carbon atom on which an aminyl nitrogen atom of the diphosphino aminyl moiety is attached. In some embodiments, the linking group having the aminyl nitrogen atom of each diphosphino aminyl moiety attached to a ring carbon can comprise at least one alkyl substituent located on a carbon atom adjacent to each carbon atom on which an aminyl nitrogen atom of the diphosphino aminyl moiety is attached. In other embodiments, the linking group having the aminyl nitrogen atom of each diphosphino aminyl moiety attached to a ring carbon can comprise only one alkyl substituent located on a carbon atom adjacent to each carbon atom on which an aminyl nitrogen atom of the diphosphino aminyl moiety is attached.

In an embodiment wherein the diphosphino aminyl ligand comprises only two diphosphino aminyl moieties, the linking group linking the aminyl nitrogen atoms of the two diphosphino aminyl moieties can be an organylene group; alternatively, an organylene group consisting of inert functional groups; or alternatively, a hydrocarbylene group. In an embodiment, the linking group linking the aminyl nitrogen atoms of the two diphosphino aminyl moieties can be a C₁ to C₅₀ organylene group; alternatively, a C₂ to C₃₀ organylene group; or alternatively, a C₂ to C₂₀ organylene group. In some embodiments, the linking group linking the aminyl nitrogen atoms of the two diphosphino aminyl moieties can be a C₁ to C₅₀ organylene group consisting of inert functional groups; alternatively, a C₂ to C₃₀ organylene group consisting of inert functional groups; or alternatively, a C₂ to C₂₀ organylene group consisting of inert functional groups. In other embodiments, the linking group linking the aminyl nitrogen atoms of the diphosphino aminyl moieties can be a C₂ to C₅₀ hydrocarbylene group; alternatively, a C₂ to C₃₀ hydrocarbylene group; or alternatively, a C₂ to C₂₀ hydrocarbylene group.

In an embodiment wherein the diphosphino aminyl ligand comprises only two diphosphino aminyl moieties, the linking group linking the aminyl nitrogen atoms of the two diphosphino aminyl moieties, can be acyclic. In some embodiments wherein the diphosphino aminyl ligand comprises only two diphosphino aminyl moieties, the linking group linking the aminyl nitrogen atoms of the two diphosphino aminyl moieties, can comprise a cyclic group; or alternatively, a substituted cyclic group. In other embodiments wherein the diphosphino aminyl ligand comprises only two diphosphino aminyl moieties, the linking group linking the aminyl nitrogen atoms of the two diphosphino aminyl moieties, can comprise a saturated cyclic group; alternatively, a substituted saturated cyclic group; alternatively, a saturated heteroatomic cyclic group; alternatively, a substituted saturated heteroatomic cyclic group; alternatively, an arene group; alternatively, a substituted arene group; alternatively, an aromatic group; alternatively, a substituted aromatic group; alternatively, a heteroaromatic group; or alternatively, a substituted heteroaromatic group. In some embodiments wherein the linking group linking the aminyl nitrogen atoms of the two diphosphino aminyl moieties is a hydrocarbon linking group, the hydrocarbon linking group, can comprises a saturated cyclic group; alternatively, a substituted saturated cyclic group; alternatively, an arene group; alternatively, a substituted arene group; alternatively, an aromatic group; or alternatively, a substituted aromatic group. In an embodiment wherein the diphosphino aminyl ligand comprises only two diphosphino aminyl moieties, at least one of the aminyl nitrogen atoms of the two diphosphino aminyl groups can be attached to a ring carbon atom of a linking group comprising any cyclic group or type of cyclic group (e.g. saturated, substituted, unsubstituted, heteroatomic, aromatic, or heteroaromatic) described herein.

In an embodiment wherein the diphosphino aminyl ligand comprises only two diphosphino aminyl moieties, the linking group can comprise at least one cyclic group and the aminyl nitrogen atom of at least one of the two diphosphino aminyl moieties can be attached to a ring carbon of the linking group. In some embodiments wherein the diphosphino aminyl ligand comprises only two diphosphino aminyl moieties, the linking group can comprise at least one cyclic group and the aminyl nitrogen atom of each of the two diphosphino aminyl moieties can be attached to a ring carbon atom of the linking group. In other embodiments wherein the diphosphino aminyl ligand comprises only two diphosphino aminyl moieties, the aminyl nitrogen atoms of the two diphosphino aminyl moieties are attached to ring carbons of the same cyclic group of the linking group. In further embodiments wherein the diphosphino aminyl ligand comprises only two diphosphino aminyl moieties, the aminyl nitrogen atoms of the two diphosphino aminyl moieties can be attached different ring carbons of the same cyclic group of the linking group. In some embodiments wherein the diphosphino aminyl ligand comprises only two diphosphino aminyl moieties, the linking group can comprise two (or can comprise only two) cyclic groups and the aminyl nitrogen atoms of the two diphosphino aminyl moieties can be attached to a ring carbon of different cyclic groups of the linking group. Generally, any cyclic group of the linking group linking the aminyl nitrogen atoms of the two diphosphino aminyl moieties can be an unsubstituted cyclic group or a substituted cyclic group. Furthermore, any cyclic group of the linking group linking the aminyl nitrogen atoms of the two diphosphino aminyl moieties can be any cyclic group or type of cyclic group (e.g. saturated, heteroatomic, aromatic, or heteroaromatic) described herein.

In an embodiment, the linking group linking the aminyl nitrogen atoms of the two diphosphino aminyl moieties, can comprise a biscyclohexylene group, or a bisphenylene group. Alternatively, the linking group linking the aminyl nitrogen atoms of the two diphosphino aminyl groups can be a substituted biscyclohexylene group; or alternatively, a substituted bisphenylene group. The substituents of the substituted linking group can be any substituent(s) described herein and can have any substituent pattern described herein. It is to be understood that since the diphosphino aminyl moiety and the linking group can be independently selected from the groups disclosed herein, the heteroatomic ligand can have any combination of the diphosphino moieties described herein and the linking group described herein.

In an embodiment, the hydrocarbylene linking group, linking the aminyl nitrogen atoms of the two diphosphino aminyl moieties, can be -(CR^{P}R^{P'})ₘ- where each R^{P} and R^{P'} can independently be hydrogen, methyl, ethyl, propyl, isopropyl, or butyl groups and m can be an integer from 1 to 12. In some embodiments, the linking group, linking the aminyl nitrogen atoms of the two diphosphino aminyl moieties, can be a methylene group (-CH₂-), an ethylene group(-CH₂CH₂-), a propylene group (-CH₂CH₂CH₂-), a -CH(CH₃)CH₂- group, -C(CH₃)₂- group, a butylene group (-CH₂CH₂CH₂-CH₂-), or a -CH₂CH(CH₃)-CH₂-group. In other non-limiting embodiments, the linking group, linking the aminyl nitrogen atoms of the two diphosphino aminyl moieties, can be a methylene group (-CH₂-), an ethylene group (-CH₂CH₂-), or a-CH(CH₃)CH₂-group; alternatively, a methylene group(-CH₂-); alternatively, an ethylene group (-CH₂CH₂-); alternatively, a propylene group (-CH₂CH₂CH₂-); alternatively, a -CH(CH₃)CH₂- group; alternatively, a-C(CH₃)₂- group; or alternatively, or a -CH₂CH(CH₃)-CH₂- group.

In an embodiment, the hydrocarbylene linking group linking the aminyl nitrogen atoms of the two diphosphino aminyl moieties can be 1,2-phenylene (benzene-1,2-diyl), a substituted 1,2-phenylene, 1,3-phenylene, a substituted 1,3-phenylene, 1,4-phenylene, a substituted 1,4-phenylene, 3,3'-biphenylene, a substituted 3,3'-biphenylene, 4,4'-biphenylene, a substituted 4,4'-biphenylene, bis(3-phenylene)methane, a substituted bis(3-phenylene)methane, bis(4-phenylene)methane, a substituted bis(4-phenylene)methane, 1,2-bis(3-phenylene)ethane, a substituted 1,2-bis(3-phenylene)ethane, 1,2-bis(4-phenylene)ethane, a substituted 1,2-bis(4-phenylene)ethane, 1,2-bis(3-phenylene)propane, a substituted 1,2-bis(3-phenylene)propane, 1,2-bis(4-phenylene)propane, a substituted 1,2-bis(4-phenylene)propane, 2,2-bis(3-phenylene)propane, a substituted 2,2-bis(3-phenylene)propane, 2,2-bis(4-phenylene)propane, or a substituted 2,2-bis(4-phenylene)propane. In some embodiments, the hydrocarbylene linking group linking the aminyl nitrogen atoms of the two diphosphino aminyl moieties can be a substituted 1,2-phenylene, a substituted 1,3-phenylene, a substituted 1,4-phenylene, a substituted 3,3'-biphenylene, a substituted 4,4'-biphenylene, a substituted bis(3-phenylene)methane, a substituted bis(4-phenylene)methane, a substituted 1,2-bis(3-phenylene)ethane, a substituted 1,2-bis(4-phenylene)ethane, a substituted 1,2-bis(3-phenylene)propane, a substituted 1,2-bis(4-phenylene)propane, a substituted 2,2-bis(3-phenylene)propane, or a substituted 2,2-bis(4-phenylene)propane.

In an embodiment, the hydrocarbylene linking group linking the aminyl nitrogen atoms of the two diphosphino aminyl moieties can be 1,2-cyclohexylene, a substituted 1,2-cyclohexylene, 1,3-cyclohexylene, a substituted 1,3-cyclohexylene, 1,4-cyclohexylene, a substituted 1,4-cyclohexylene, 3,3'-bicyclohexylene, a substituted 3,3'-bicyclohexylene, 4,4'-bicyclohexylene, a substituted 4,4'-bicyclohexylene, bis(3-cyclohexylene)methane, a substituted bis(3-cyclohexylene)methane, bis(4-cyclohexylene)methane, a substituted bis(4-cyclohexylene)methane, 1,2-bis(3-cyclohexylene)ethane, a substituted 1,2-bis(3-cyclohexylene)ethane, 1,2-bis(4-cyclohexylene)ethane, a substituted 1,2-bis(4-cyclohexylene)ethane, 1,2-bis(3-cyclohexylene)propane, a substituted 1,2-bis(3-cyclohexylene)propane, 1,2-bis(4-cyclohexylene)propane, a substituted 1,2-bis(4-cyclohexylene)propane, 2,2-bis(3-cyclohexylene)-propane, a substituted 2,2-bis(3-cyclohexylene)propane, 2,2-bis(4-cyclohexylene)propane, or a substituted 2,2-bis(4-cyclohexylene)propane. In some embodiments, the hydrocarbylene linking group linking the aminyl nitrogen atoms of the two diphosphino aminyl moieties can be a substituted 1,2-cyclohexylene, a substituted 1,3-cyclohexylene, a substituted 1,4-cyclohexylene, a substituted 3,3'-bicyclohexylene, a substituted 4,4'-bicyclohexylene, a substituted bis(3-cyclohexylene)methane, a substituted bis(4-cyclohexylene)methane, a substituted 1,2-bis(3-cyclohexylene)ethane, a substituted 1,2-bis(4-cyclohexylene)ethane, a substituted 1,2-bis(3-cyclohexylene)propane, a substituted 1,2-bis(4-cyclohexylene)propane, a substituted 2,2-bis(3-cyclohexylene)propane, or a substituted 2,2-bis(4-cyclohexylene)propane.

In an embodiment, the linking group can be a 3,3'-dihydrocarbyl-4,4'-biphenylene; alternatively, a bis(3-hydrocarbyl-4-phenylene)methane; alternatively, 1,2-bis(3-hydrocarbyl-4-phenylene)ethane; alternatively, 1,2-bis(3-hydrocarbyl-4-phenylene)propane; or alternatively, 2,2-bis(3-hydrocarbyl-4-phenylene)propane. In othe embodiment, the linking group can be a 3,3'-dihydrocarbyl-4,4'-bicyclohexylene; alternatively, a bis(3-hydrocarbyl-4-cyclohexylene)methane; alternatively, 1,2-bis(3-hydrocarbyl-4-cyclohexylene)ethane; alternatively, 1,2-bis(3-hydrocarbyl-4-cyclohexylene)propane; or alternatively, 2,2-bis(3-hydrocarbyl-4-cyclohexylene)propane. In some embodiments, the linking group can be a 3,3'-dialkyl-4,4'-biphenylene; alternatively, a bis(3-alkyl-4-phenylene)methane; alternatively, 1,2-bis(3-alkyl-4-phenylene)ethane; alternatively, 1,2-bis(3-alkyl-4-phenylene)propane; or alternatively, 2,2-bis(3-alkyl-4-phenylene)propane. In other embodiments the linking group can be a 3,3'-dialkyl-4,4'-bicyclohexylene; alternatively, a bis(3-alkyl-4-cyclohexylene)methane; alternatively, 1,2-bis(3-alkyl-4-cyclohexylene)ethane; alternatively, 1,2-bis(3-alkyl-4-cyclohexylene)propane; or alternatively, 2,2-bis(3-alkyl-4-cyclohexylene)propane. In some non-limiting embodiments the each hydrocarbyl group (or alkyl group) can be any hydrocarbyl substituent (or alkyl substituent) described herein.

In an embodiment, the substituents of the substituted linking group, L, can be a hydrocarbyl group; alternatively, an organyl group; alternatively, an organyl group consisting of an inert functional group; or alternatively, an inert functional group. In an embodiment, the hydrocarbyl (general or specific group members - e.g. alkyl, aryl, or aralkyl), organyl (general or specific group members), or organyl consisting of inert functional group (general or specific group members) substituents of the substituted linking group, L, can have from 1 to 15 carbon atoms; alternatively, from 1 to 10 carbon atoms; or alternatively, from 1 to 5 carbon atoms. In some embodiments, each inert functional group can be a halogen or a hydrocarboxy group; alternatively, a halogen; or alternatively, a hydrocarboxy group. Substituent halogens, substituent hydrocarbyl groups (general and specific), and substituent hydrocarboxy groups (general and specific) are independently described herein and these substitutent groups can be utilized without limitation to further describe the substituents which can be utilized on a substituted linking group, L.

In an embodiment, the diphosphino aminyl ligand can comprise any number of diphosphino aminyl moieties disclosed herein. In ssome embodiments, the diphosphino aminyl ligand can comprise at least one primary diphosphino aminyl moiety; alternatively, at least one secondary diphosphino aminyl moiety; or alternatively, at least one tertiary diphosphino aminyl moiety. In some embodiments, the diphosphino aminyl moieties can consist of secondary diphosphino aminyl moieties. The terms primary, secondary, and tertiary diphosphino aminyl moieties refers to the type of carbon atom that the aminyl nitrogen atom of the diphosphino aminyl moiety is attached. A primary diphosphino moiety is one in which the aminyl nitrogen atom of the diphosphino aminyl moiety is attached to a carbon atom which is attached to only one other carbon atom. A secondary diphosphino moiety is one in which the aminyl nitrogen atom of the diphosphino aminyl moiety is attached to a carbon atom which is attached to two other carbon atoms. A tertiary diphosphino moiety is one in which the aminyl nitrogen atom of the diphosphino aminyl moiety is attached to a carbon atom which is attached to three other carbon atoms. The terms "wherein the diphosphino aminyl moieties consist of secondary diphosphino aminyl moieties" only refers to the type of carbon atom to which the diphosphino aminyl moiety of the diphosphino aminyl ligand is attached and does not imply the presence or absence of any other diphosphino aminyl ligand element. The diphosphino aminyl ligand can and can likely contain other elements as described herein.

In an embodiment, the diphosphino aminyl ligand, regardless of the number of diphosphino aminyl moieties, can comprise at least one tertiary carbon atom adjacent (i.e. attached) to a carbon atom to which the aminyl nitrogen atom of a diphosphino aminyl moiety is attached. In some embodiments, the diphosphino aminyl ligand, regardless of the number of diphosphino aminyl moieties, can comprise at least one tertiary carbon atom adjacent (i.e. attached) to each carbon atom to which the aminyl nitrogen atom of the diphosphino aminyl moiety is attached. In other embodiments, the diphosphino aminyl ligand, regardless of the number of diphosphino aminyl moieties, can have only one tertiary carbon atom adjacent (i.e. attached) to each carbon atom to which the aminyl nitrogen atom of the diphosphino aminyl moiety is attached.

For illustrative purposes, general structures AA-AU comprising only two diphosphino moieties linked by a saturated hydrocarbylene and having various tertiary carbon atoms within the hydrocarbylene linking group are presented. Statements regarding which of structures AA-AU meet or do not meet three criteria for the location of the tertiary carbon atoms in relation to the diphosphino aminyl moiety(ies) are presented to assist the skilled artisan in visualizing the relationship between the tertiary carbon atoms and the aminyl nitrogen atom of the diphosphino aminyl moiety. Structures AA-AU do not limit the herein disclosed invention to these structures, to diphosphino aminyl ligands comprising only 2 diphosphino aminyl moieties, and/or saturated hydrocarbylene linking groups. The diphosphino aminyl ligand can have any number of diphosphino aminyl moieties as described herein and/or any type of linking group linking the aminyl nitrogen atoms of the diphosphino aminyl moieties as described herein (e.g. aromatic of heteroaromatic linking groups). Upon review a skilled artisan will be able to apply the criteria for the location of tertiary carbon atoms to build and determine other materials having 1, 2, 3 or more diphosphino aminyl moieties that can meet the criteria for the location of the tertiary carbon atoms in relation to the diphosphino aminyl moiety(ies).

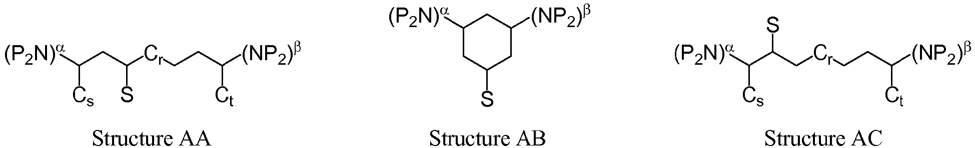

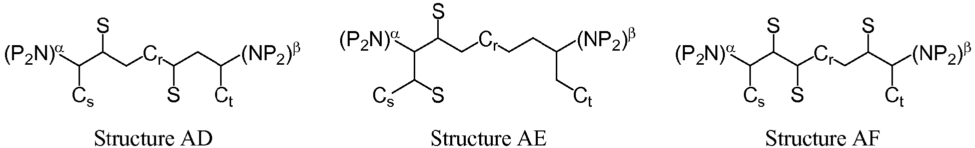

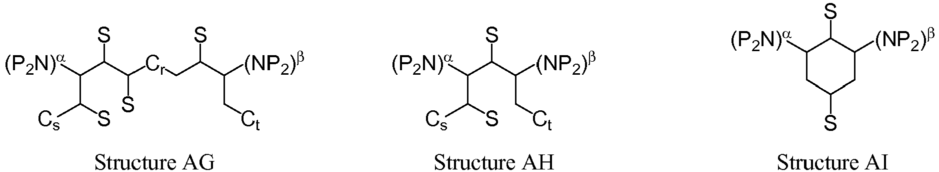

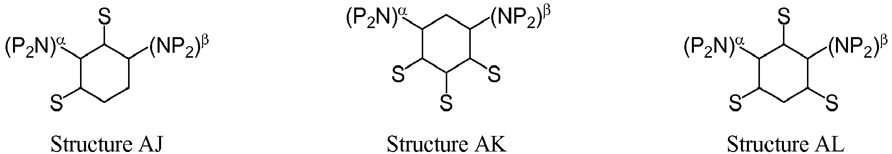

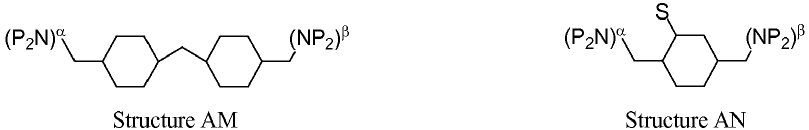

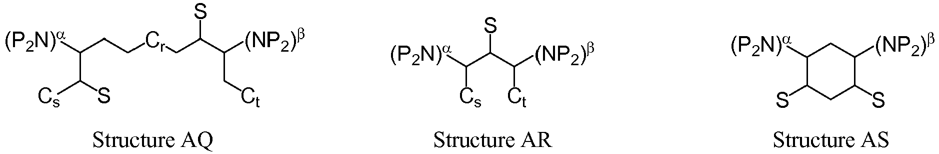

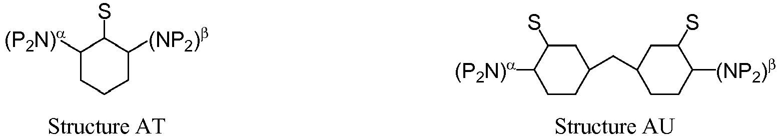

Within Structures AA-AU, (P₂N)^{α}- and -(NP₂)^{β} represent generalized diphosphino aminyl moieties, Cᵣ can represent a generalized hydrocarbon group, a generalized organyl group, or a generalized organyl group consisting of inert functional groups, Cₛ, and Cₜ can represent a hydrogen atom, a generalized hydrocarbyl group, a generalized organyl group, or a generalized organyl group consisting of inert functional groups, and S represents a generalized substituent.

Structures AA and AB do not satisfy the criteria of the phrase "comprises at least one tertiary carbon atom adjacent to a carbon atom on which an aminyl nitrogen atom of a diphosphino aminyl moiety is attached." While each of these structures have a tertiary carbon atom(s) within the structure, the tertiary carbon atom is not adjacent to the carbon atom on which the aminyl nitrogen atom of the diphosphino aminyl moiety alpha or beta are attached.

Structures AC-AU satisfy the criteria of the phrase "comprises at least one tertiary carbon atom adjacent to a carbon atom on which an aminyl nitrogen atom of a diphosphino aminyl moiety is attached."
1. Structures AC, AD, AI, AR, and AT each have one tertiary carbon atom located on a carbon atom adjacent to a carbon atom on which the aminyl nitrogen atom of the diphosphino aminyl moiety alpha is attached.
   (a) Structures AC and AD have one tertiary carbon atom located on a carbon adjacent to the carbon atom on which the aminyl nitrogen atom of a diphosphino aminyl moiety is attached.
   (b) Structures AI, AR, and AT each have one tertiary carbon atom located on a carbon atom adjacent to both the carbon atom on which the aminyl nitrogen atom of diphosphino aminyl moiety alpha is attached and the aminyl nitrogen atom of diphosphino aminyl moiety beta is attached.
   (c) Structures AD and AI have an additional tertiary carbon atom not located on carbon atom adjacent to a carbon atom on which an aminyl nitrogen atom of a diphosphino aminyl moiety is attached. However, Structures AD and AI meet the requirement of comprising at least one tertiary carbon atom adjacent to a carbon atom on which an aminyl nitrogen atom of a diphosphino aminyl moiety is attached.
2. Structures AE, AF, AH, AJ, AK, AM, AN, AQ, AS, and AU have two tertiary carbon atoms located on a carbon atoms adjacent to a carbon atom on which the aminyl nitrogen atom of the diphosphino aminyl moiety is attached.
   (a) Structure AE has two tertiary carbon atoms located on a carbon atom adjacent to the carbon on which the aminyl nitrogen atom of diphosphino aminyl moiety alpha is located.
   (b) Structures AF, AK, AM, AN, AQ, AS, and AU have a tertiary carbon atom located on a carbon atom adjacent to the carbon atom on which the aminyl nitrogen atom of diphosphino aminyl moiety alpha is located and a second tertiary carbon atom located on a carbon atom adjacent to the carbon atom on which the aminyl nitrogen atom of diphosphino aminyl moiety beta is located.
   (c) Structures AH and AJ have a tertiary carbon atom located on a carbon atom adjacent to the carbon atom on which the aminyl nitrogen atom of diphosphino aminyl moiety alpha is located and a second tertiary carbon atom located adjacent to the carbon atom on which the aminyl nitrogen atom of diphosphino aminyl moiety alpha and the aminyl nitrogen atom of diphosphino aminyl moiety beta is located.
   (d) Structures AF, AK, AM, and AN have additional tertiary carbon atoms not located on carbon atoms which are not adjacent to a carbon atom on which an aminyl nitrogen atom of a diphosphino aminyl moiety is attached. However, Structures AF, AK, AM, and AN meet the criteria of comprising "at least one tertiary carbon atom adjacent to a carbon atom on which an aminyl nitrogen atom of a diphosphino aminyl moiety is attached."
3. Structures AG and AL have three tertiary carbon atoms located on carbon atoms adjacent to the carbon atom on which the aminyl nitrogen atom of a diphosphino aminyl moiety is attached.
   (a) Structure AG has two tertiary carbon atoms located on a carbon atom adjacent to the carbon atom on which the aminyl nitrogen atom of diphosphino aminyl moiety alpha is located and a third tertiary carbon atom located on a carbon atom adjacent to a carbon atom on which the aminyl nitrogen atom of diphosphino aminyl moiety beta is located.
   (b) Structure AL has one tertiary carbon atom located on a carbon atom adjacent to the carbon atom on which the aminyl nitrogen atom of diphosphino aminyl moiety alpha is located, a second tertiary carbon atom located on a carbon atom adjacent to the carbon atom on which the aminyl nitrogen atom of diphosphino aminyl moiety beta is located, and a third tertiary carbon atom located on a carbon atom adjacent to the carbon atoms on which the aminyl nitrogen atom of diphosphino aminyl moiety alpha and the aminyl nitrogen atom of diphosphino aminyl moiety beta is located.
   (c) Structure AG has an additional tertiary carbon atom not located on carbon atom adjacent to a carbon atom on which an aminyl nitrogen atom of a diphosphino aminyl moiety is attached. However, Structure AG meets the criteria of comprising "at least one tertiary carbon atom adjacent to a carbon atom on which an aminyl nitrogen atom of a diphosphino aminyl moiety is attached."

Structures AA, AB, AC, AD, and AE do not satisfy the criteria of the phrase "comprises at least one tertiary carbon atom adjacent to each carbon atom to which the aminyl nitrogen atom of the diphosphino aminyl moiety is attached."
1. Structures AA and AB do not have any tertiary carbon atoms located on carbon atom adjacent to a carbon atoms on which an aminyl nitrogen atom of which a diphosphino aminyl moiety is attached.
2. Structures AC, AD, and AE have at least one tertiary atom located adjacent to the carbon atom on which the aminyl nitrogen atom of diphosphino aminyl moiety alpha is attached but no tertiary carbon atom adjacent to the carbon atom on which the diphosphino aminyl moiety beta is attached.

Structures AF-AU satisfy the criteria of the phrase "comprises at least one tertiary carbon atom adjacent to each carbon atom on which an aminyl nitrogen atom of a diphosphino aminyl moiety is attached."
1. Structures AI, AR, and AT each have one tertiary carbon atom located on a carbon atom adjacent to both the carbon atom on which the diphosphino aminyl moiety alpha is located and the aminyl nitrogen atom of diphosphino aminyl moiety beta is attached. Structure AI has an additional tertiary carbon atom not located on carbon atom adjacent to a carbon atom on which an aminyl nitrogen atom of a diphosphino aminyl moiety is attached. However, Structure AI meets the criteria of comprising "at least one tertiary carbon atom adjacent to each carbon atom on which an aminyl nitrogen atom of a diphosphino aminyl moiety is attached."
2. Structures AF, AH, AJ, AK, AM, AN, AQ, AS, and AU have two tertiary carbon atoms located on a carbon atoms adjacent to a carbon atom on which the aminyl nitrogen atom of the diphosphino aminyl moiety is attached.
   (a) Structures AF, AK, AM, AN, AQ, AS, and AU have a tertiary carbon atom located on a carbon atom adjacent to the carbon atom on which a diphosphino aminyl moiety alpha is located and a second tertiary carbon atom located on a carbon atom adjacent to the carbon atom on which a diphosphino aminyl moiety beta is located.
   (b) Structures AH and AJ have a tertiary carbon atom located on a carbon atom adjacent to the carbon atom on which a diphosphino aminyl moiety alpha is located and a second tertiary carbon atom located adjacent to both the carbon atom on which the aminyl nitrogen atom of diphosphino aminyl moiety alpha is located and the aminyl nitrogen atom of diphosphino aminyl moiety beta is located.
   (c) Structures AF, AK, AM, and AN have additional tertiary carbon atoms not located on carbon atoms adjacent to a carbon atom on which an aminyl nitrogen atom of a diphosphino aminyl moiety is attached. However, Structures AF, AK, AM, and AN meet the criteria of comprising "at least one tertiary carbon atom adjacent to a carbon atom on which an aminyl nitrogen atom of a diphosphino aminyl moiety is attached."
3. Structures AG and AL have three tertiary carbon atoms located on carbon atoms adjacent to the carbon atom on which the aminyl nitrogen atom of a diphosphino aminyl moiety is attached.
   (a) Structure AG has two tertiary carbon atoms located on a carbon atom adjacent to the carbon atom on which the aminyl nitrogen atom of diphosphino aminyl moiety alpha is located and a third tertiary carbon atom located on a carbon atom adjacent to a carbon atom on which the aminyl nitrogen atom of diphosphino aminyl moiety beta is located.
   (b) Structure AL has one tertiary carbon atom located on a carbon atom adjacent to the carbon atom on which the aminyl nitrogen atom of diphosphino aminyl moiety alpha is located, a second tertiary carbon atom located adjacent to a carbon atom on which the aminyl nitrogen atom of diphosphino aminyl moiety beta is located, and a third tertiary carbon atom located adjacent to the carbon atom on which the aminyl nitrogen atom of diphosphino aminyl moiety alpha and the aminyl nitrogen atom of diphosphino aminyl moiety beta is located.
   (c) Structure AG has an additional tertiary carbon atom not located on carbon atom adjacent to a carbon atom on which an aminyl nitrogen atom of a diphosphino aminyl moiety is attached. However, Structure AG meets the criteria of comprising "at least one tertiary carbon atom adjacent to a carbon atom on which an aminyl nitrogen atom of a diphosphino aminyl moiety is attached."

Structures AA-AE, AG-AH, AJ, and AL do not satisfy the criteria of the phrase "comprises only one tertiary carbon atom adjacent to each carbon atom to which the aminyl nitrogen atom of the diphosphino aminyl moiety is attached."
1. Structures AA and AB do not have any tertiary carbon atoms located on a carbon atom adjacent to a carbon atoms on which an aminyl nitrogen atom of which a diphosphino aminyl moiety is attached.
2. Structures AC, AD, and AE have at least one tertiary atom located on a carbon atom adjacent to the carbon atom on which the aminyl nitrogen atom of diphosphino aminyl moiety alpha is attached but no tertiary carbon atom adjacent to the carbon atom on which the diphosphino aminyl moiety beta is attached.
3. Structures AG, AH, AJ, and AL have more that one tertiary carbon atom located on a carbon atom adjacent to the carbon atom on which the aminyl nitrogen atom of diphosphino aminyl moiety alpha is located.

Structures AF, AI, AK, AM, AN, AQ, AR, AS, AT, and AU satisfy the criteria of the phrase "comprises only one tertiary carbon atom adjacent to each carbon atom to which the aminyl nitrogen atom of the diphosphino aminyl moiety is attached."
1. Structures AI, AR, and AT each have one tertiary carbon atom located on a carbon atom adjacent to both the carbon atom on which the diphosphino aminyl moiety alpha is located and the aminyl nitrogen atom of diphosphino aminyl moiety beta is attached and has no other tertiary carbon atoms located on a carbon atom adjacent to a carbon atom on which the aminyl nitrogen atom of a diphosphino aminyl moiety is attached.
2. Structures AF, AK, AM, AN, AQ, AS, and AU have one tertiary carbon atom located on a carbon atom adjacent to the carbon atom on which the aminyl nitrogen atom of a diphosphino aminyl moiety alpha is located and a second tertiary carbon atom located on a carbon atom adjacent to the carbon atom on which the aminyl nitrogen atom diphosphino aminyl moiety beta is located.
3. Structures AF, AI, AK, and AM have at least one additional tertiary carbon atom that is not located on carbon atom adjacent to a carbon atom on which an aminyl nitrogen atom of a diphosphino aminyl moiety is attached. However, AF, AI, AK, AM, and AO meets the criteria of comprising "only one tertiary carbon atom adjacent to each carbon atom to which the aminyl nitrogen atom of the diphosphino aminyl moiety is attached."

In an aspect, the heteroatomic ligand having multiple diphosphino aminyl moieties can have two diphosphino aminyl moieties connected by a linking group. In an embodiment, the heteroatomic ligand can be represented by the chemical formula (R¹R²P)(R³R⁴P)N-L-N(R^{1'}R^{2'}P)(R^{3'}R^{4'}P), Structure IV: 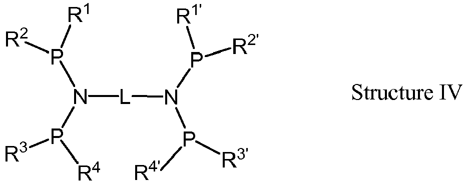 wherein the two diphosphino aminyl moieties can independently be any diphosphino aminyl moiety described herein and wherein L can be any linking group linking the two diphosphino aminyl moieties as described herein. In some embodiments, the first diphosphino aminyl moiety (R¹R²P)(R³R⁴P)N-) and the second diphosphino aminyl group (-N(R^{1'}R^{2'}P)(R^{3'}R^{4'}P)) are different; or alternatively, the first diphosphino aminyl group (R¹R²P)(R³R⁴P)N-) and the second diphosphino aminyl group (-N(R^{1'}R^{2'}P)(R^{3'}R^{4'}P)) are the same. In another embodiment, a heteroatomic ligand can be represented by Structure V: 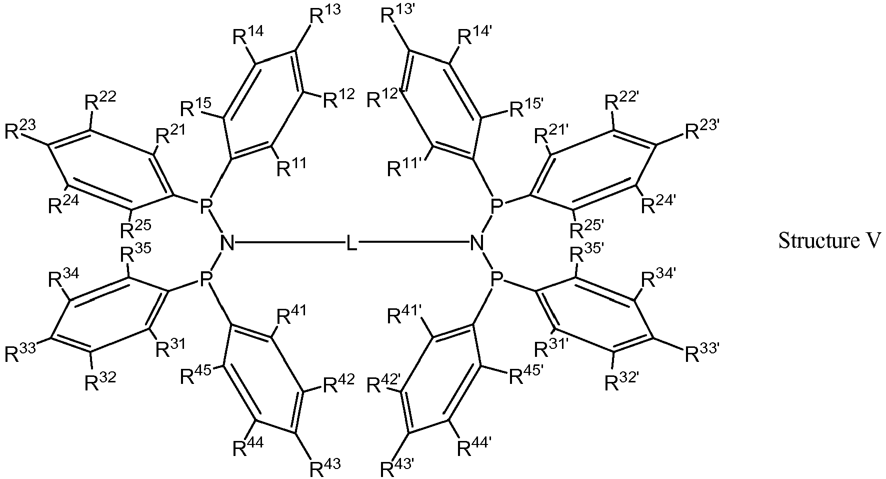 wherein the two bis(diphenylphosphino) aminyl moieties (substituted phenyl rings or unsubstituted phenyl rings) can independently be any bis(diphenylphosphino) aminyl moiety (substituted phenyl rings or unsubstituted phenyl ring) described herein and L can be any linking group linking the nitrogen atoms of the bis(diphenylphosphino) aminyl moieties described herein. In some embodiments, the two bis(diphenylphosphino) aminyl moieties (substituted phenyl rings or unsubstituted phenyl rings)are different; or alternatively, the two bis(diphenylphosphino) aminyl moieties (substituted phenyl rings or unsubstituted phenyl rings)are the same.

In an embodiment, the linking group can have any Structure in Table 1. In some embodiments, the linking group can have Structure 1L; alternatively, Structure 2L; alternatively, Structure 3L; alternatively Structure 4L; alternatively, Structure 5L; alternatively, Structure 6L; alternatively, Structure 7L; alternatively, Structure 8L; alternatively, Structure 9L; or alternatively, Structure 10L.

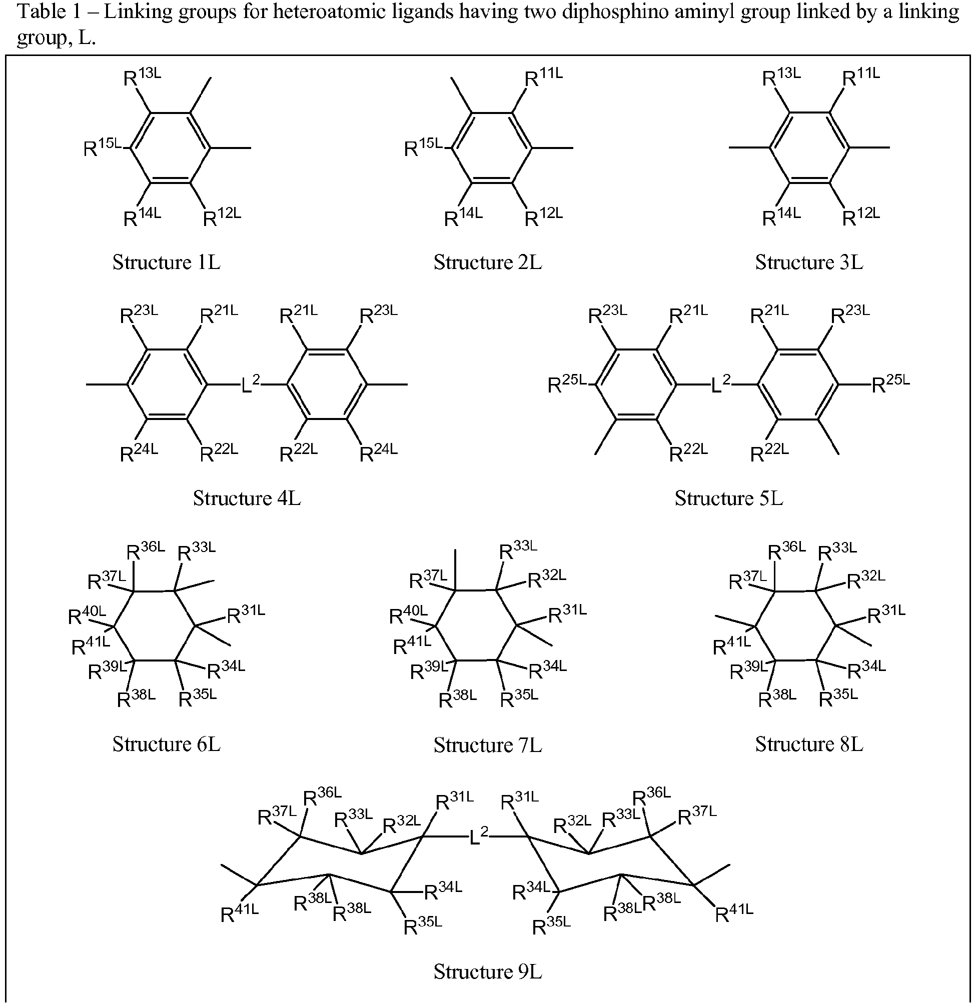

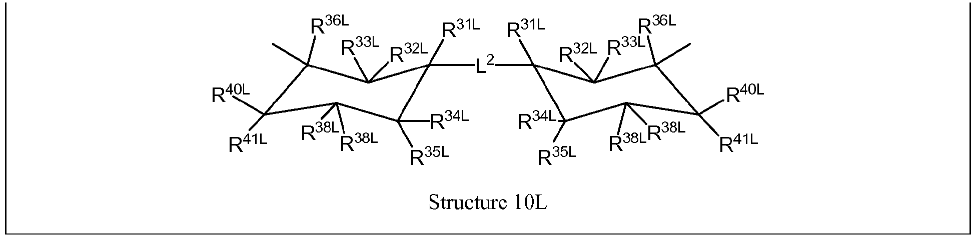

Within the Structures of Table 1, R^{11L} to/through R^{15L}, R^{21L} to/through R^{25L}, R^{31L} to/through R^{41L} can each independently be hydrogen, an organyl group consisting of inert functional groups, or an inert functional group; alternatively, hydrogen, a hydrocarbyl group, or an inert functional groups. Within the Structure of Table 1, L² represents a linking group, and the undesignated valencies represent the positions at which the nitrogen atoms of the diphosphino aminyl moieties are attached. General and specific organyl group consisting of inert functional groups, hydrocarbyl groups, and inert functional are generally desecribed herein and these general and specific groups can be utilized without limitation as R^{11L} to/through R^{15L}, R^{21L} to/through R^{25L}, R^{31L} to/through R^{41L} in each Structure of Table 1. Additionally, the substituent(s) of the linking groups having Structure 1L, Structure 2L, Structure 3L, Structure 4L, Structure 5L, Structure 6L, Structure 7L, Structure 8L, Structure 9L or Structure 10L can can have any substituent pattern described herein, and/or have any pattern necessary to meet any criteria of the diphosphino aminyl ligand as described herein.

In an embodiment, linking group L² can be -(CR^{L}R^{L})ₘ- where each R^{L} and R^{L} can independently be hydrogen, methyl, ethyl, propyl, isopropyl, or butyl groups and m can be an integer from 1 to 5. In other embodiments, the linking group L² can be a bond, a methylene group (-CH₂-), an ethylene group (-CH₂CH₂-), a propylene group (-CH₂CH₂CH₂-), a -CH(CH₃)CH₂- group, -C(CH₃)₂- group, or a butylene group (-CH₂CH₂CH₂CH₂-). In some non-limiting embodiments, the linking group L² can be a methylene group (-CH₂-), an ethylene group (-CH₂CH₂-), or a -CH(CH₃)CH₂- group; or alternatively, an ethylene group (-CH₂CH₂-), or a -CH(CH₃)CH₂- group. In yet other embodiments, the linking group can be a methylene group; alternatively, an ethylene group; alternatively, a propylene group; alternatively, a-CH(CH₃)CH₂- group; or alternatively -C(CH₃)₂- group.

In some embodiments, the heteroatomic ligand can have Structure VI: 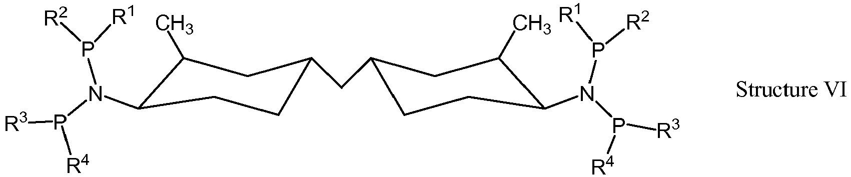 wherein the diphosphino aminyl moieties, (R¹R²P)(R³R⁴P)N-, can be any described herein and the undesignated valencies represent hydrogen. In other embodiments, the heteroatomic ligand can have Structure VII: 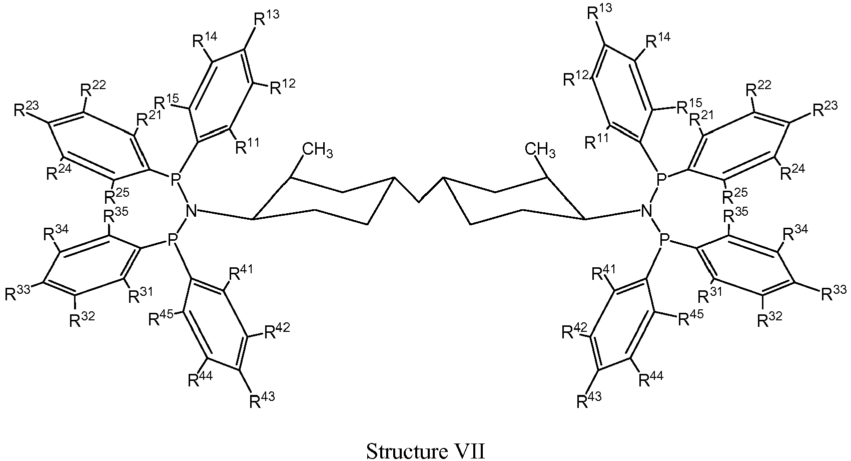 wherein the bis(diphenylphosphino) aminyl moieties (substituted phenyl groups or unsubstituted phenyl groups) can be any described herein and the undesignated valencies represent hydrogen. In other embodiments, the heteroatomic ligand can have Structure VIII: 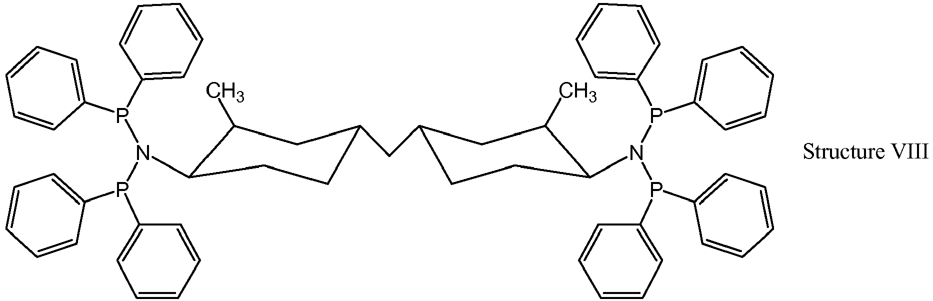 wherein the undesignated valencies represent hydrogen. Skilled artisans will readily know how to produce other heteroatomic ligand comprising multiple diphosphino aminyl moieties and a linking group linking the nitrogen atoms of the diphosphino aminyl moieties by utilizing the diphosphino aminyl moieties described herein, the linking groups L and/or L² described herein, the substituents described herein, the substituent patterns described herein, and other aspects of the diphosphino aminyl moieties and/or diphosphino aminyl ligands as described herein. Though not specifically drawn, these structures are incorporated in this disclosure.

In an embodiment, the metal compound, M-Xₚ, of the olefin oligomerization catalyst system comprising a metal compound complexed to a heteroatomic ligand can comprise any transition metal. In some embodiments, the metal compound comprises a group IVB, VB, or VIB metal (the group designation is CAS group designations); alternatively, a Group IVB metal; alternatively, a Group VB metal; or alternatively, a Group VIB metal. In other embodiments, the metal compound comprises titanium, vanadium, or chromium. In yet other embodiments, the metal compound comprises chromium. In still other embodiments, the metal compound comprising chromium can comprise chromium trichloride.

The anion X, of the metal compound can be any anion. In some embodiments, the anion X can be a halide, carboxylate, β-diketonate, alkoxide, phenoxide, nitrate, sulfate, phosphate, or chlorate. In some embodiments, the anion, X, is a halide, carboxylate, or β-diketonate. In other embodiments, the anion can be a halide; alternatively, a carboxylate; or alternatively, a β-diketonate.

In an embodiment, the halide anion can be fluoride, chloride, bromide, iodide, or combinations thereof; alternatively, chloride, bromide, iodide, or combinations thereof. In other embodiments, the halide anion can be chloride; alternatively, bromide; or alternatively, iodide.

In carboxylate, acetonate, alkoxide or phenoxide embodiments, the carboxylate, β-diketonate, alkoxide, or phenoxide can be any C₁ to C₂₀ carboxylate, β-diketonate, alkoxide, or phenoxide; or alternatively, any C₁ to C₁₀ carboxylate, β-diketonate, alkoxide, or phenoxide. In some embodiments, the anion, X, can be a C₁ to C₁₀ β-diketonate; alternatively, a C₁ to C₁₀ carboxylate; alternatively, a C₁ to C₁₀ alkoxide; or alternatively, a C₁ to C₁₀ phenoxide. In other embodiments, the anion X, can be acetylacetonate; alternatively, acetate; alternatively, 2-ethylhexanoate; or alternatively, triflate.

Generally, the number, p, of anions, X, is such that the total number of negative charges on the total number of X anions equals the oxidation state of M. In an embodiment, p is 2, or 3, and the total number of negative charges on the Xs bonded to the metal atom is equal to the oxidation state of M. In other embodiments, the total number of anions, p, is 2; or alternatively, 3.

In some embodiments wherein the metal compound can comprise chromium. In some embodiments, the metal compound can be a chromium(II) compound, chromium(III) compound, or combinations thereof. Suitable chromium(II) compounds include, but are not limited to, chromium(II) fluoride, chromium(II) chloride, chromium(II) bromide, chromium(II) iodide, chromium(II) (2-ethylhexanoate), chromium(II) acetate, chromium(II) butyrate, chromium(II) neopentanoate, chromium(II) laurate, chromium(II) stearate, chromium(II) oxalate, chromium(II) benzoate, or combinations thereof. Suitable chromium(III) compounds include, but are not limited to, chromium(III) trichloride tris-tetrahydrofuran complex, chromium(III) 2,2,6,6-tetramethylheptanedionate, chromium(III) naphthenate, chromium(III) chloride, chromium(III) bromide, chromium(III) chloride, chromium(III) fluoride, chromium(III) acetylacetonate, or combinations thereof.

In an embodiment, the metal compound can be a chromium(III) carboxylate. Without limitation, examples of chromium(III) carboxylates include chromium(III) isooctanoate, chromium(III) (2-ethylhexanoate), chromium(III) oxy-2-ethylhexanoate, chromium(III) dichloroethylhexanoate, chromium(III) acetate, chromium(III) butyrate, chromium(III) neopentanoate, chromium(III) laurate, chromium(III) stearate, chromium(III) oxalate, chromium(III) benzoate, chromium(III) octanoate, chromium(III) propionate, or combinations thereof. In an embodiment, the chromium-containing compound can be chromium(III) (2-ethylhexanoate).

In another embodiment, the metal compound can be a chromium β-diketonate. Examples of such chromium β-diketonates include without limitation chromium (III) acetylacetonate, chromium (III) hexafluoroacetylacetonate and tris(2,2,6,6-tetramethyl-3,5-heptanedionato)chromium (III). In an embodiment, the chromium β-diketonate is chromium (III) acetylacetonate (also referred to as Cr(acac)₃).

In an aspect, the oligomerization catalyst system can comprise a metal complex comprising a metal compound complexed to a heteroatomic ligand. In an embodiment, the heteroatomic ligand can be any heteroatomic ligand described herein. In some embodiments, the heteroatomic ligand can be any ligand comprising any dialkylphosphino moiety described herein; alternatively, any heteroatomic ligand comprising multiple dialkylphosphino moieties described herein; alternatively, any heteroatomic ligand comprising at least two dialkylphosphino moieties; alternatively, alternatively, any heteroatomic ligand comprising only one dialkylphosphino moiety; or alternatively, any heteroatomic ligand comprising only two dialkylphosphino moieties. In general, the heteroatomic ligand and the metal compound are independent elements of the catalyst. Thus, the metal complex comprising a metal compound complexed to a heteroatomic ligand can be described using any combination of the heteroatomic ligand described herein and the metal compound described herein.

In an embodiment, the metal complex can be described as a product of contacting a metal compound with a heteroatomic ligand. As the metal compound and the heteroatomic ligand are independent elements, the metal complex can be as described as contacting any metal compound described herein with any heteroatomic ligand described herein. In an embodiment, the metal complex can be described as the product of contacting a metal compound with a heteroatomic ligand comprising a diphosphino aminyl moiety; alternatively, with a heteroatomic ligand comprising at least 2 diphosphino aminyl moieties; alternatively, with a heteroatomic ligand comprising 2 to 5 diphosphino aminyl moieties; alternatively, with a heteroatomic ligand comprising 2 to 3 diphosphino aminyl moieties; alternatively, with a heteroatomic ligand comprising only one diphosphino aminyl moiety; or alternatively, with a heteroatomic ligand comprising only 2 diphosphino aminyl moieties.

In an embodiment, the heteroatomic ligand can be a diphosphino aminyl ligand. In some embodiments, the diphosphino aminyl moiety(ies) of the diphosphino aminyl igand can be any diphosphibo aminyl moiety described herein. In some embodiments, the diphosphino aminyl moiety(ies) can have Structure 1, Structure 3, or any combination thereof. In other embodiments, the diphosphino aminyl moiety(ies) can have Strutue 1, or alternatively Struture 3.

In an embodiment, the metal complex comprising a metal compound complexed to a heteroatomic ligand can be a metal compound complexed to heteroatomic ligand having Structure I, Structure II, Structure III, or any combination thereof. In some embodiments, the metal complex comprising a metal compound complexed to a heteroatomic ligand can be a metal compound complexed to heteroatomic ligand having Structure I; alternatively, Structure II; or alternatively, Structure III. In other embodiments, the metal complex comprising a metal compound complexed to a heteroatomic ligand can be a metal compound complexed to heteroatomic ligand having Structure IV, Structure V, Structure VI, Structure VII, Structure VIII, or any combination thereof. In yet other embodiments, the metal complex comprising a metal compound complexed to a heteroatomic ligand can be a metal compound complexed to heteroatomic ligand having Structure VI; alternatively, Structure VII; or alternatively, Structure VIII.

In an embodiment, the metal complex can be represented by the formula [R¹R²P-N(R⁵)-PR³R⁴]M-Xp, wherein R¹, R², R³, and R⁴ can be any substituent or have any substituent pattern described herein, R⁵ can be any group as described herein, and M-Xₚ can be any metal compound as described herein. In some embodiments, the metal complex can be represented by Structure X: 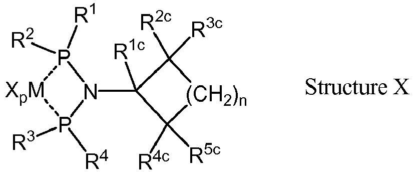 Within the metal complex having Structure X embodiments, R¹, R², R³, and R⁴ can be any substituent or have any substituent pattern described herein for the dialkyl phosphino aminyl moiety, R^{1c}, R^{2c}, R^{3c}, R^{4c}, and R^{5c} can be any substituent or have any substituent pattern described herein for the cycloalkyl aminyl nitrogen group, n of the cycloalkyl aminyl nitrogen group can be any value described herein, and M-Xₚ can be any metal compound described herein. In some embodiments, the metal complex can have Structure XI: 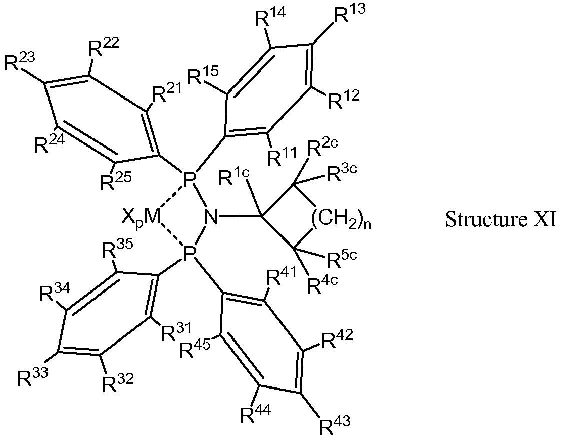 Within the metal complex embodiments having Structure XI, R¹¹, R¹², R¹³, R¹⁴, R¹⁵, R²¹, R²², R²³, R²⁴, R²⁵, R³¹, R³², R³³, R³⁴, R³⁵, R⁴¹, R⁴², R⁴³, R⁴⁴, and R⁴⁵ can be any substituent or have any substituent pattern described herein for the diphosphino aminyl moiety, R^{1c}, R^{2c}, R^{3c}, R^{4c}, and R^{5c} can be any substituent or have any substituent pattern described herein for the cycloalkyl aminyl nitrogen group, n of the cycloalkyl aminyl nitrogen group can be any value described herein, and MXₚ can be any metal compound described herein. In other embodiments, the metal complex can have Structure XII:

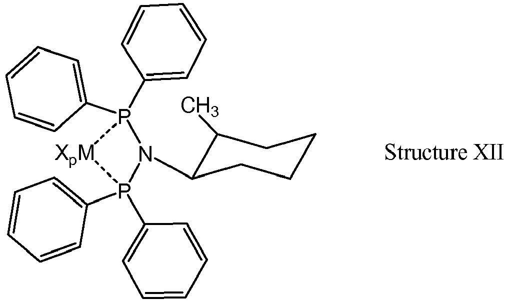

Alternatively, the metal complex can be the product of contacting a metal compound, M-Xₚ, with a heteroatomic ligand comprising multiple diphosphino aminyl moieties, (PNP)_{q}L(M-Xₚ)_{q}, wherein PNP represents the diphosphino aminyl moieties, L represents the linking group linking the aminyl nitrogen atoms of the diphosphino aminyl moieties, M-Xₚ represents the metal compound, and q represents the number of diphosphino aminyl moieties present in the diphosphino aminyl ligand and the number of metal compounds in the metal complex. Generally, the diphosphino moieties, the linking group linking the diphosphino moieties, the metal compound, and q are independent elements of the metal complex. Therefore, the metal complex can be described as a metal compound complexed to a diphosphino aminyl ligand wherein the diphosphino aminyl ligand can have any combination of diphosphino aminyl moieties as described herein, linking group linking the diphosphino aminyl moieties as described herein, and number of diphosphino aminyl moieties, q, as described herein, and the metal compound can be any metal compound described herein.

Alternatively, the metal complex can be the product of contacting a metal compound, M-Xₚ, with a heteroatomic ligand comprising two diphosphino aminyl moieties and a linking group linking the aminyl nitrogen atoms of the two diphosphino aminyl moieties; (PNP)₂L(M-Xₚ)₂. The resulting metal complex can be dinuclear and have the chemical formula [R¹R²P-N(R⁵)-PR³R⁴]₂L(M-Xₚ)₂. In an embodiment, the metal complex is represented by Structure XIII: 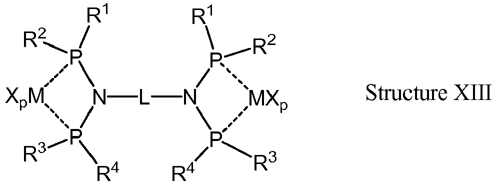 wherein R¹, R², R³, and R⁴ can be any substituent or have any substituent pattern described herein, L can be any linking group described herein and M-Xₚ can be any metal compound described herein. In some embodiments, the metal complex is represented by Structure XIV: 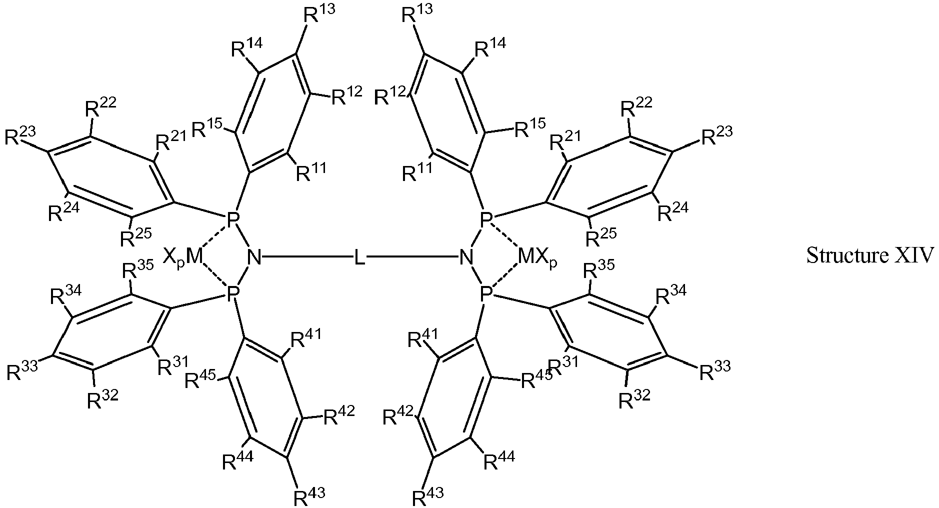 wherein R¹¹, R¹², R¹³, R¹⁴, R¹⁵, R²¹, R²², R²³, R²⁴, R²⁵, R³¹, R³², R³³, R³⁴, R³⁵, R⁴¹, R⁴², R⁴³, R⁴⁴, and R⁴⁵ can be any substituent or have any substituent pattern described herein, L can be any linking group described herein and M-Xₚ can be any metal compound described herein. In other embodiments, the metal complex can be represented by Structure XV: 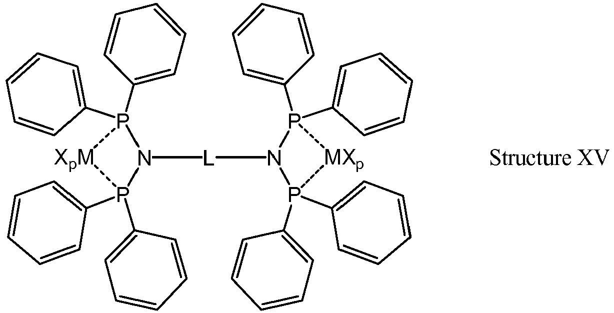 wherein L can be any linking group described herein and M-Xₚ can be any metal compound described herein, and the undesignated phenyl ring valencies are hydrogen.

In an embodiment, the metal complex can have Structure XVI: 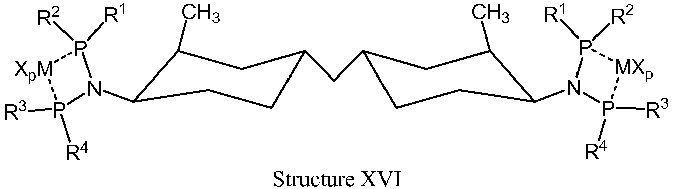 wherein R¹, R², R³, and R⁴ can be any substituent or have any substituent pattern described herein, M-Xₚ can be any metal compound described herein, and the undesignated valencies represent hydrogen. In an embodiment, the metal complex can have Structure XVII: 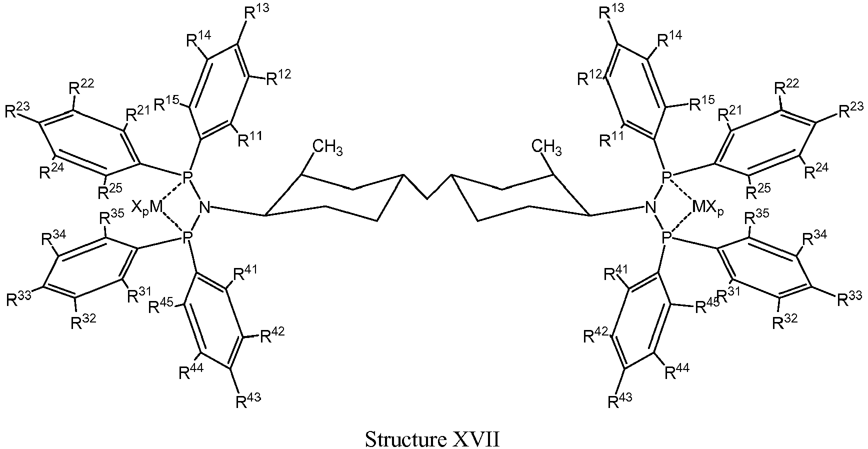 wherein R¹¹, R¹², R¹³, R¹⁴, R¹⁵, R²¹, R²², R²³, R²⁴, R²⁵, R³¹, R³², R³³, R³⁴, R³⁵, R⁴¹, R⁴², R⁴³, R⁴⁴, and R⁴⁵ can be any substituent or have any substituent pattern described herein, M-Xₚ can be any metal compound described herein and the undesignated valencies represent hydrogen. In an embodiment, the metal complex can have Structure XVIII: 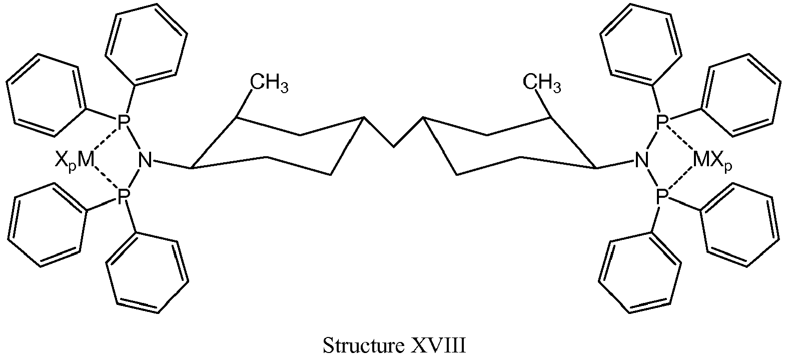 wherein the undesignated valencies represent hydrogen.

In an embodiment, the metal compound of the metal complex having Structure X, Structure XI, Structure XII, Structure XIII, Structure XIV, Structure XV, Structure XVI, Structure XVII, or Structure XVIII, can comprise titanium, vanadium, or chromium. In an embodiment, the metal compound of the metal complex having Structure X, Structure XI, Structure XII, Structure XIII, Structure XIV, Structure XV, Structure XVI, Structure XVII, or Structure XVIII, can comprise chromium. Generally, the chromium compound can have a formula of CrXₚ, wherein X can be the same or different and can be any organic or inorganic radical, and p can be an integer from 0 to 6. Suitable organic radicals are described herein. In some embodiments, the metal compound of the metal complex having Structure X, Structure XI, Structure XII, Structure XIII, Structure XIV, Structure XV, Structure XVI, Structure XVII, or Structure XVIII can be a chromium halide, a chromium carboxylate, a chromium β-diketonate, or mixture thereof. In other embodiments, the metal compound of the metal complex having Structure X, Structure XI, Structure XII, Structure XIII, Structure XIV, Structure XV, Structure XVI, Structure XVII, or Structure XVIII can be a chromium halide; alternatively, a chromium carboxylate; or alternatively, a chromium β-diketonate. Applicable chromium halides, chromium carboxylates, and chromium β-diketonate are described herein and can generally be utilized as the metal compound of the metal complexes having Structure X, Structure XI, Structure XII, Structure XIII, Structure XIV, Structure XV, Structure XVI, Structure XVII, or Structure XVIII. In some embodiments the chromium compound can be chromium(III) tris(2-ethylhexanoate); or alternatively, chromium (III) acetylacetonate (Cr(acac)₃).

One skilled in the art will recognize that metal complex structures formally show a monomeric form of a metal compound complexed to a heteroatomic ligand (or a metal compound complexed to a ligand comprising a diphosphino aminyl moiety). However, it should be noted that these structures do not necessarily imply that dimeric and/or oligomeric forms of structures having bridging Xₚ groups which connect metal atoms complexed to the heteroatomic ligand (or the ligand comprising a diphosphino aminyl moiety) are not formed. The monomeric structures provided herein can encompass the dimeric and/or oligomeric forms of structures having bridging Xₚ groups which can connect metal atoms complexed to the heteroatomic ligand (or the ligand comprising a diphosphino aminyl moiety).

In an embodiment, the olefin oligomerization catalyst system comprises a metal complex and a metal alkyl comprising an aluminoxane. A metal alkyl can be any organometallic compound capable of activating the metal complex comprising a metal compound complexed to a heteroatomic ligands described herein to polymerize or oligomerize olefins. Metal alkyls can include monomeric or oligomeric metal alkyls, metal aryls, metal alkyl-aryls comprising B, Al, Be, Mg, Ca, Sr, Ba, Li, Na, K, Rb, Cs, Zn, Cd, and Sn. A metal alkyl can be an organoaluminum compound, organoboron compound, organomagnesium compound, organozinc compound, organolithium compound, or mixtures thereof. A metal alkyl can be an organoaluminum compound. Organoaluminum compounds can include, but non limited to, trialkyl-aluminums, alkylaluminum halides, alumoxanes, or mixtures thereof. The metal alkyl comprises an alumoxane (also referred to as an aluminoxane). In other embodiments, the cocatalyst consists essentially of one or more alumoxanes. In yet other embodiments, the metal alkyl consists of one or more alumoxanes. The metal alkyl can have any number of carbon atoms. However, due to commercial availability and ease of use, the metal alkyl can comprise less than about 70 carbon atoms per metal alkyl molecule; or alternatively, less than about 20 carbon atoms per molecule.

Metal alkyls include, but are not limited to, n-butyllithium, s-butyllithium, t-butyllithium, diethylmagnesium, dibutylmagnesium, diethylzinc, triethylaluminum, trimethylaluminum, tripropylaluminum, tributylaluminum, triisobutylaluminum, tri-n-hexylaluminum, tri-n-octylaluminum, diethylaluminum ethoxide, diethylaluminum phenoxide, ethylaluminum dichloride, diethylaluminum chloride, diethylaluminum bromide, diethylaluminum sesquichloride, diisobutylaluminum chloride, ethylaluminum sesquichloride, diethylaluminum bromide, diethylaluminum iodide, ethylaluminumethoxychloride, and mixtures thereof. A metal alkyl can be triethylaluminum.

A metal alkyl can comprise at least one alkylaluminum compound. A metal alkyl can be a trialkylaluminum compound. Organoaluminum compounds can include, but are not limited to, trimethylaluminum, triethylaluminum, tripropylaluminum, tri-n-butylaluminum, tri-iso-butylaluminum, tri-n-hexylaluminum, tri-n-octylaluminum, diethylaluminumchloride, diethylaluminumbromide, diethylaluminumethoxide, diethylaluminum phenoxide, ethylaluminumethoxychloride, diethylaluminum cyanide, ethylaluminum dichloride, diethylaluminum chloride, diethylaluminum bromide, ethylaluminum sesquichloride, diisobutylaluminum chloride, methylaluminoxane (MAO), modified methylaluminoxane (MMAO), ethylaluminoxane, isobutyl alumoxane, t-butyl alumoxane, and mixtures thereof. In other embodiments, the metal alkyl can comprise, or consist consist essentially of, methylalumoxane (MAO), modified methylalumoxane (MMAO), isobutyl alumoxane, t-butyl alumoxane, or mixtures thereof. In other embodiments, the metal alkyl can comprise, or consist essentially of, methylalumoxane, modified methylalumoxane, or mixtures thereof. In yet other embodiments, the metal alkyl can comprise, or consist essentially of methylalumoxane; alternatively, modified methylalumoxane; isobutylalumoxane (IBAO); or alternatively, a partially hydrolyzed trialkylaluminum.

Described herein is an olefin oligomerization catalyst system which can comprise metal compound complexed to a diphosphino aminyl ligand and a metal alkyl; or alternatively, can comprise a metal compound and a diphosphino aminyl ligand. As the metal compound complexed to a diphosphino aminyl ligand and metal alkyl are independent elements of the olefin oligomerization catalyst system, the olefin oligomerization catalyst system can comprise any combination of the metal compound complexed to a diphosphino aminyl ligand described herein and the metal alkyl described herein. In some non-limiting embodiments, the metal complex comprising a metal compound complexed to a diphosphino aminyl ligand. In some embodiments, the olefin oligomerization catalyst system can comprise a metal compound complexed to a diphosphino aminyl ligand comprising multiple diphosphino aminyl moieties. In some embodiments, the olefin oligomerization catalyst comprises a metal compound complexed to a diphosphino aminyl ligand comprising at least 2 diphosphino aminyl moieties; alternatively, with a diphosphino aminyl ligand comprising 2 to 5 diphosphino aminyl moieties; alternatively, with a diphosphino aminyl ligand comprising 2 to 3 diphosphino aminyl moieties; alternatively, with a diphosphino aminyl ligand comprising only 1 diphosphino aminyl moieties; or alternatively, with a diphosphino aminyl ligand comprising only 2 diphosphino aminyl moieties. Further aspects of the diphosphino aminyl ligand are described herein and can be utilized to further describe the diphosphino aminyl ligand utilized in the olefin oligomerization catalyst system.

Generally, the olefin oligomerization catalyst system can be formed by contacting a metal compound complexed to a diphosphino aminyl ligand and a metal alkyl; or alternatively, by contacting a metal compound, a diphosphino aminyl ligand, and a metal alkyl. In an embodiment, the contacting can occur in the presence of a solvent. In an embodiment, an olefin oligomerization catalyst system can be formed by contacting a metal compound complexed to a diphosphino aminyl ligand and an aluminoxane; or alternatively, by contacting a metal compound, a diphosphino aminyl ligand, and an aluminoxane. In some embodiments, an olefin oligomerization catalyst system can be formed by contacting a chromium compound complexed to a diphosphino aminyl ligand and a metal alkyl; alternatively, by contacting a chromium compound, a diphosphino aminyl ligand, and a metal alkyl; alternatively, by contacting a chromium compound complexed to a diphosphino aminyl ligand and a metal alkyl in the presence of a solvent; or alternatively, by contacting a chromium compound, a diphosphino aminyl ligand, and a metal alkyl in the presence of a solvent. In other embodiments, an olefin oligomerization catalyst system can be formed by contacting a chromium compound complexed to a diphosphino aminyl ligand having at least two diphoshino aminyl groups (or any other number of diphosphino aminyl groups disclosed herein) and an alkyl aluminum compound; by contacting a chromium compound, a diphosphino aminyl ligand having at least two diphoshino aminyl groups (or any other number of diphosphino aminyl groups disclosed herein), and an alkyl aluminum compound; alternatively, by contacting a chromium compound complexed to a ligand having at least two diphosphino aminyl groups (or any other number of diphosphino aminyl groups disclosed herein) and an alkyl aluminum compound in the presence of a solvent; or alternatively, by contacting a chromium compound, a ligand having at least two diphosphino aminyl groups (or any other number of diphosphino aminyl groups disclosed herein), and an alkyl aluminum compound in the presence of a solvent. In yet other embodiments, an olefin oligomerization catalyst system can be formed by contacting Cr(acac)₃ complexed to a ligand having at least two diphosphino aminyl groups (or any other number of diphosphino aminyl groups disclosed herein) and an aluminoxane; alternatively, by contacting Cr(acac)₃, a ligand having at least two diphosphino aminyl groups (or any other number of diphosphino aminyl groups disclosed herein), and an aluminoxane; alternatively, by contacting an Cr(acac)₃ complexed to a ligand having at least two diphosphino aminyl groups (or any other number of diphosphino aminyl groups disclosed herein) and an aluminoxane in the presence of a solvent; or alternatively, by contacting an Cr(acac)₃, a ligand having at least two diphosphino aminyl groups (or any other number of diphosphino aminyl groups disclosed herein), and an aluminoxane in the presence of a solvent. In still other embodiments, an olefin oligomerization catalyst system can be formed by contacting CrCl₃ complexed to a ligand having at least two diphosphino aminyl groups (or any other number of diphosphino aminyl groups disclosed herein) and an aluminoxane; alternatively, by contacting CrCl₃, a ligand having at least two diphosphino aminyl groups (or any other number of diphosphino aminyl groups disclosed herein), and an aluminoxane; alternatively, by contacting an olefin oligomerization catalyst comprising CrCl₃ complexed to a ligand having at least two diphosphino aminyl groups (or any other number of diphosphino aminyl groups disclosed herein) and an aluminoxane in the presence of a solvent; or alternatively, by contacting an olefin oligomerization catalyst comprising CrCl₃, a ligand having at least two diphosphino aminyl groups (or any other number of diphosphino aminyl groups disclosed herein), and an aluminoxane in the presence of a solvent Without wishing to be limited by theory, some oligomerization catalyst systems disclosed can comprise chromium complexes formed when the heteroatomic ligands coordinate the chromium compounds of this disclosure. Said chromium complexes can comprise the chromium compound complexed with the heteroatomic ligand and solvent molecules (e.g. CrCl₃(THF)₃). Such chromium complexes can be further contacted with a metal alkyl to produce olefin oligomerization catalyst systems. The olefin oligomerization catalyst systems can function in the oligomerization of olefins such as the trimerization or tetramerization of ethylene to 1-hexene and/or 1-octene, respectively.

Generally, the molar ratio of the metal of the metal alkyl to the metal of the metal complex (alternatively, the metal alkyl metal to metal complex metal molar ratio) can be any molar ratio that produces an olefin oligomer product (oligomerized olefin product). In an embodiment, the molar ratio of the metal of the metal alkyl to the metal of the metal complex can be greater than 100:1; alternatively, greater than 200:1; alternatively, greater than 300:1; alternatively, greater than 400:1; alternatively greater than 500:1; alternatively, greater than 600:1; or alternatively greater than 700:1. In some embodiments, the molar ratio of the metal of the metal alkyl to the metal of the metal complex can range from 1:1 to 10,000:1; alternatively, from 10:1 to 5,000:1; or alternatively, from 100:1 to 3,000:1; alternatively, from 200:1 to 2,000:1; alternatively, from 400:1 to 1600:1; or alternatively, or alternatively, from 600:1 to 1000:1. In some embodiments wherein the metal of the metal complex comprises chromium and the metal alkyl is an alumoxane the molar ratio of aluminum to chromium can be greater than 100:1; alternatively, greater than 200:1; alternatively, greater than 300:1; alternatively, greater than 400:1; alternatively greater than 500:1; alternatively, greater than 600:1; alternatively, greater than 700:1; alternatively, range from 1:1 to 10,000:1; alternatively, from 10:1 to 5,000:1; alternatively, from 100:1 to 3,000:1; or alternatively, from 200:1 to 2,000:1.

When the catalys system comprises a metal compound, a diphosphino aminyl ligand and a metal alkyl the molar ratio of the metal of the metal alkyl to the metal of the metal compound (alternatively, the metal alkyl metal to metal compound metal molar ratio) can be any molar ratio that produces an olefin oligomer product (oligomerized olefin product). In an embodiment, the molar ratio of the metal of the metal alkyl to the metal of the metal compound can be greater than 100:1; alternatively, greater than 200:1; alternatively, greater than 300:1; alternatively, greater than 400:1; alternatively greater than 500:1; alternatively, greater than 600:1; or alternatively greater than 700:1. In some embodiments, the molar ratio of the metal of the metal alkyl to the metal of the metal compound can range from 1:1 to 10,000:1; alternatively, from 10:1 to 5,000:1; or alternatively, from 100:1 to 3,000:1; alternatively, from 200:1 to 2,000:1; alternatively, from 400:1 to 1600:1; or alternatively, or alternatively, from 600:1 to 1000:1. In some embodiments wherein the metal of the metal compound comprises chromium and the metal alkyl is an alumoxane the molar ratio of aluminum to chromium can be greater than 100:1; alternatively, greater than 200:1; alternatively, greater than 300:1; alternatively, greater than 400:1; alternatively greater than 500:1; alternatively, greater than 600:1; alternatively, greater than 700:1; alternatively, range from 1:1 to 10,000:1; alternatively, from 10:1 to 5,000:1; alternatively, from 100:1 to 3,000:1; or alternatively, from 200:1 to 2,000:1.

The catalyst system described herein can further comprise a molar diphosphino aminyl moiety to metal of the metal compound ratio greater than the 1:1 ratio required to form the oligomerization catalyst. This additional diphosphino aminyl moiety can be added during the preparation of the metal complex and/or added during the preparation of the catalyst system (e.g. with the metal alkyl). In an embodiment, the molar diphosphino aminyl moiety to metal of the metal compound ratio in the oligomerization catalyst system can be greater than 1.8:1; alternatively, greater than 1.9:1; alternatively, greater than 2.0:1; alternatively, greater than 2.5:1; or alternatively, greater than 3.0:1. In other embodiments, the molar diphosphino aminyl moiety to metal of the metal compound ratio in the catalyst system can range from 1.8:1 to 10:1; alternatively, ranges from 1.9:1 to 7:1; or alternatively, ranges from 1.95:1 to 5:1.

In an embodiment, the catalyst system can be described as comprising a metal compound, a ligand comprising a diphosphino aminyl moiety, and a metal alkyl. In some embodiments, the catalyst system can be further described by the diphosphino aminyl moiety to metal compound molar ratio and/or the metal alkyl metal to metal compound metal molar ratio utilized in the catalyst system. Generally, the metal compound, the ligand comprising a diphosphino aminyl moiety, the metal alkyl, the diphosphino aminyl moiety to metal compound metal molar ratio, and the metal alkyl metal to metal compound metal ratio utilized to describe the catalyst system are independent elements of the of catalyst system. Thus, the catalyst system can comprise any combination of metal compound described herein, diphosphino aminyl ligand described herein, metal alkyl described herein, diphosphino aminyl moiety to metal compound metal molar ratio described herein, and/or metal alkyl metal to metal compound molar ratio as described herein.

In a non-limiting embodiment, the catalyst system comprising a metal compound, a ligand comprising a diphosphino aminyl moiety, and a metal alkyl can have a diphosphino aminyl moiety to metal of the metal compound molar ratio greater than 1.8:1. In some non-limiting embodiments, the catalyst system can have a diphosphino aminyl moiety to metal of the metal compound molar ratio greater than 2.0:1; alternatively, greater than 2.5:1; or alternatively, greater than 3:1. In other non-limiting embodiments, the catalyst system can have a diphosphino aminyl moiety to metal of the metal compound molar ratio ranging from 1.8 to 10:1; alternatively, ranging from 2.0:1 to 10:1; alternatively, ranging from 3:1 to 8:1; or alternatively, 4:1 to 6:1. In an embodiment, the metal compound of the catalyst system can comprise chromium and the diphosphino aminyl moiety to metal molar ratio can be stated as a diphosphino aminyl moiety to chromium molar ratio.

In a non-limiting embodiment, the catalyst system comprising a metal compound, a ligand comprising a diphosphino aminyl moiety, and a metal alkyl can have a diphosphino aminyl moiety to metal of the metal compound molar ratio greater than 1.8:1 and a metal alkyl metal to metal of the metal compound molar ratio greater than 200:1. In some non-limiting embodiments, the catalyst system can have a diphosphino aminyl moiety to metal of the metal compound molar ratio greater than 1.8:1 and a metal alkyl metal to metal of the metal compound molar ratio greater than 200:1; alternatively, a diphosphino aminyl moiety to metal of the metal compound molar ratio greater than 2.0:1 and a metal alkyl metal to metal of the metal compound molar ratio greater than 300:1; alternatively, a diphosphino aminyl moiety to metal of the metal compound molar ratio greater than 2.5:1 and a metal alkyl metal to metal of the metal compound molar ratio greater than 400:1; alternatively, a diphosphino aminyl moiety to metal of the metal compound molar ratio ranging from 1.8 to 10:1 and an metal alkyl metal to metal of the metal compound molar ratio ranging from 100:1 to 3,000:1; alternatively, a diphosphino aminyl moiety to metal of the metal compound molar ratio ranging from 2.0:1 to 10:1 and a metal alkyl metal to metal of the metal compound molar ratio ranging from 400:1 to 1600:1; alternatively, a diphosphino aminyl moiety to metal of the metal compound molar ratio ranging from 3:1 to 8:1 and a metal alkyl metal to metal of the metal compound molar ratio ranging from 500:1 to 1,200:1; or alternatively, a diphosphino aminyl moiety to metal of the metal compound molar ratio ranging from 4:1 to 6:1 and a metal alkyl metal to metal of the metal compound molar ratio ranging from 600:1 to 1,000:1. In an embodiment, the metal compound of the catalyst system can comprise chromium and the metal alkyl can comprise aluminum. In such case, the diphosphino aminyl moiety to metal of the metal compound molar ratio can be stated as a diphosphino aminyl moiety to chromium molar ratio and the metal alkyl metal to metal of the metal compound molar ratio ranging can be stated as an aluminum to chromium molar ratio.

In a non-limiting embodiment, the catalyst system can comprise a metal compound comprising chromium, a diphosphino aminyl ligand, and an aluminoxane. In some non-limiting embodiments, the catalyst system can comprise a metal compound comprising chromium, a diphosphino aminyl ligand having at least one diphosphino aminyl moiety, and an aluminoxane; alternatively, a metal compound comprising chromium, a diphosphino aminyl ligand comprising at least two diphosphino aminyl moieties and linking group linking the aminyl nitrogen atoms of the aminyl moieties, and an aluminoxane; alternatively, a metal compound comprising chromium, a diphosphino aminyl ligand comprising only 1 diphosphino aminyl moieties and an aluminoxane; or alternatively, a metal compound comprising chromium, a diphosphino aminyl ligand comprising only 2 diphosphino aminyl moieties and a linking group linking the aminyl nitrogen atoms of the 2 diphosphino aminyl moieties, and an aluminoxane.

In embodiments utilizing a vessel for contacting the components, the components can be optionally mixed by a mixer disposed in the vessel and the formed mixture can then be removed for subsequent processing. In embodiments utilizing a tee or other means for combing lines such as a header, an optional in-line mixer can be placed in the commingled catalyst feed line to ensure that adequate contacting of the combined components takes place, and the mixture is thus formed as it passes through the commingled feed line. Where a method of making a catalyst recites contact or combination of catalyst components, such can be carried out by contacting or combining all or a portion of such components in various embodiments.

As used herein, a composition comprising a catalyst component includes the catalyst component alone or in combination with one or more additional compounds, solvents, or both. None, some, or all of the contacting steps can be carried out in the presence of a solvent (sometimes referred to as an optional solvent), which can be introduced to a contact zone via inclusion with one or more compositions comprising a catalyst component or can be introduced separately to a contact zone, for example in a solvent line or as an initial charge to a contact zone.

In an embodiment, water, acidic protons or both can be abated from any or all of the components of the described catalyst system using methods and conditions known to one of ordinary skill in the art. Such methods and conditions are disclosed in detail in U.S. Patent Application Serial No.: 11/207,232 filed August 19, 2005 and entitled "Methods of Preparation of an Olefin Oligomerization Catalyst" which was is i herein.

In an embodiment, the olefin oligomerization catalyst system can further comprise a solvent. The solvent can be a hydrocarbon solvent, a halogenated hydrocarbon solvent, or combinations thereof. Generally, the catalyst system solvent or diluent can comprise a C₄ to C₂₀ hydrocarbon; alternatively, a C₄ to C₁₀ hydrocarbon; alternatively, a C₁ to C₁₅ halogenated hydrocarbon; or alternatively, a C₁ to C₁₀ halogenated hydrocarbon. The hydrocarbon solvent can be a saturated hydrocarbon, an aromatic hydrocarbon, or an olefinic hydrocarbon. In an embodiment, the saturated hydrocarbon solvent or diluent can comprise butane, isobutane, pentane, n-hexane, hexanes, cyclohexane, n-heptane or n-octane, or mixtures thereof. In some embodiments, the aromatic solvent can be a C₆ to C₂₀ aromatic compound. Suitable aromatic hydrocarbons can include benzene, toluene, mixed xylenes, ortho-xylene, meta-xylene, para-xylene, ethylbenzene, or mixtures thereof. Suitable halogenated catalyst solvents or diluents can include, carbon tetrachloride, chloroform, methylene chloride, dichloroethane, trichloroethane, chlorobenzene, or dichlorobenzene, or mixtures thereof. In an embodiment, the solvent can be ethylbenzene.

In an embodiment when the catalyst system is prepared in a solvent (or a catalyst system mixture further comprises a solvent), the concentration of the metal of the metal compound or the metal of the metal complex can range from 1 x 10⁻⁶ M to 2 x 10⁻¹ M; alternatively, 5 x 10⁻⁵ M to 5 x 10⁻² M; or alternatively, 1 x 10⁻⁵ M to 1 x 10⁻² M. Solvents suitable for use with the catalyst system are disclosed and can be utilized without limitation in aspects and embodiment described herein.

The catalyst systems described in the present application can be employed in the oligomerization of olefins. Such a process can be carried out by contacting the catalyst system with one or more olefin monomers under reaction conditions suitable for polymerization or oligomerization of olefins. In some embodiments, the oligomerization process can comprise: a) contacting an olefin, a metal complex, and a metal alkyl; and b) forming an olefin oligomer product. In other embodiments, the oligomerization process can be an ethylene oligomerization process (or alpha olefin production process) comprising: a) contacting ethylene, a metal complex, and a metal alkyl; and b) forming olefin oligomer product. In other embodiments, the oligomerization process can be an alpha olefin production process comprising: a) contacting ethylene, a metal complex, and a metal alkyl; and b) forming an olefin oligomer product comprising an alpha olefin. Generally, the metal complex can have any structure provided herein or can have any description described herein. In futher embodiments, the oligomerization process can comprise: a) contacting an olefin and a catalyst system and b) forming an olefin oligomer product. In other embodiments, the oligomerization process can be an ethylene oligomerization process (or alpha olefin production process) comprising: a) contacting ethylene and a catalyst system; and b) forming an olefin oligomer product comprising olefins. In yet other embodiments, the oligomerization process can be an alpha olefin production process comprising: a) contacting ethylene and a catalyst system; and b) forming an olefin oligomer product comprising an alpha olefin. Generally, the catalyst system can be any catalyst system described herein. In some embodiment, the olefin oligomer product is formed under conditions suitable for forming an olefin oligomer product.

In an aspect, the present disclosure relates to an olefin oligomerization process. Within this disclosure, olefin oligomerization relates to processes which produce products of which at least 80 weight percent contain from 1 to 20 monomer units.

In an embodiment, the olefin oligomerization process can comprise: a) contacting an olefin and a catalyst system; and b) forming an olefin oligomer product. In some embodiments, the olefin oligomerization process can comprise, a) contacting an olefin, hydrogen, and a catalyst system; and b) forming an olefin oligomer product. In an embodiment, the olefin polymerization process can comprise: a) contacting an olefin and a catalyst system; and b) forming an olefin polymer product. In some embodiments, the olefin polymerization process can comprise a) contacting an olefin, hydrogen, and a catalyst system and b) forming an olefin polymer product. The catalyst system, olefin, and features of the olefin oligomer or olefin polymer product are independently described herein and can be utilized, without limitation to further describe the olefin oligomerization or olefin polymerization process. In an embodiment, the catalyst system can be prepared in a first solvent. In an embodiment, the olefin, catalyst system, and optionally hydrogen, can be contacted in a second solvent. Generally, a solvent in which the catalyst system can be prepared and the solvent in which the olefin and catalyst system can be contacted can be the same; or alternatively, can be different. In some embodiments, the olefin oligomer product is formed under conditions suitable for forming an olefin oligomer product.

In an embodiment, the olefin oligomerization process can comprise: a) contacting i) a metal complex comprising a metal compound complexed to a diphosphino aminyl ligand and ii) a metal alkyl to form a mixture; b) contacting the mixture with an olefin; and c) forming an olefin oligomer product. In some embodiments, the step of contacting the mixture with the olefin can be a step of contacting the mixture with an olefin and hydrogen. In some embodiments, the mixture can further comprise a solvent (e.g. a first solvent). In some embodiments, the mixture and olefin can be contacted in a solvent (e.g. a second solvent when the catalyst system is prepared in a solvent). In an embodiment, the olefin oligomerization process can comprise: a) contacting i) a metal complex comprising a metal compound complexed to a diphosphino aminyl ligand, ii) a metal alkyl, and iii) a first solvent to form a mixture; b) contacting the mixture with an olefin and a second solvent; and c) forming an olefin oligomer product. In some embodiments, the mixture formed in step a) can comprise, or consist essentially of, i) a metal complex comprising a metal compound complexed to a diphosphino aminyl ligand, ii) a metal alkyl, and iii) a first solvent. In some embodiments, the step of contacting the mixture with the olefin and the second solvent can be a step of contacting the mixture with an olefin, a second solvent, and hydrogen. The metal complex comprising a metal compound complexed to a diphosphino aminyl ligand, metal alkyl, olefin, solvents, and features of the olefin oligomer or olefin polymer product are independently described herein (among other catalyst system and olefin oligomerization features) and can be utilized, without limitation to further describe the olefin oligomerization or olefin polymerization process. In some embodiments, the first and second solvent can be the same; or alternatively, the first and second solvent can be different. In some embodiments, the metal alkyl can comprise, or consist essentially of, an aluminoxane. Ratios for the metal of the metal complex comprising metal complex comprising a metal compound complexed to a diphosphino aminyl ligand to the metal of the metal alkyl are independently provided herein (among other catalyst system and olefin oligomerization features) and can be utilized without limitation to further describe the olefin oligomerization or olefin polymerization process. In some embodiments, the olefin oligomer product is formed under conditions suitable for forming an olefin oligomer product.

In an embodiment, the olefin oligomerization process can comprise: a) contacting i) a metal compound, ii) a diphosphino aminyl ligand, and iii) a metal alkyl to form a mixture; b) contacting the catalyst system mixture with an olefin; and c) forming an olefin oligomer product. In some embodiments, the step of contacting the mixture with the olefin can be a step of contacting the mixture with an olefin and hydrogen. In some embodiments, the mixture can further comprise a solvent (e.g. a first solvent). In some embodiments, the mixture and olefin can be contacted in a solvent (e.g. a second solvent when the mixture is prepared in a solvent). In an embodiment, the olefin oligomerization process can comprise: a) contacting i) a metal compound, ii) a diphosphino aminyl ligand, iIi) a metal alkyl, and iv) a first solvent; b) contacting the mixture with an olefin and a second solvent; and c) forming an olefin oligomer product. In some embodiments, the mixture formed in step a) can comprise, or consist essentially of, i) a metal compound, ii) a diphosphino aminyl ligand, iii) a metal alkyl, and iv) a first solvent. The metal compound, the diphosphino aminyl ligand, metal alkyl, olefin, solvents, and features of the olefin oligomer or olefin polymer product are independently described herein (among other catalyst system and olefin oligomerization features) and can be utilized, without limitation to further describe the olefin oligomerization or olefin polymerization process. In some embodiments, the first and second solvent can be the same; or alternatively, the first and second solvent can be different. In some embodiments, the metal alkyl can comprise, or consist essentially of, an aluminoxane. Ratios for the metal of the metal compound to the metal of the metal alkyl and ratios for the metal compound to a diphosphino aminyl ligand are independently provided herein (among other catalyst system and olefin oligomerization features) and can be utilized without limitation to further describe the olefin oligomerization or olefin polymerization process. In some embodiments, the olefin oligomer product is formed under conditions suitable for forming an olefin oligomer product.

Generally, the metal complex utilized in the olefin oligomerization can be any metal complex described herein. Likewise when the catalyst system ustilizes a metal compound and a diphosphino aminyl ligand, the metal compound can be any metal compound disclosed herein and the diphosphino aminyl ligand can be any diphosphino aminyl ligand disclosed herein.

In a non-limiting embodiment, the metal complex utilized in the olefin oligomerization process can have the Structure X: 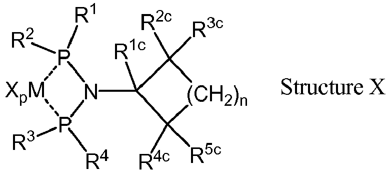 wherein R¹, R², R³, and R⁴ can be any substituent described herein or have any substituent pattern described herein for the dialkyl phosphino aminyl moiety, R^{1c}, R^{2c}, R^{3c}, R^{4c}, and R^{5c} can be any substituent described herein, have any substituent pattern described herein for the cycloalkyl aminyl nitrogen group, and/or have any substituent pattern necessary to meet a particular aspect of the diphosphino aminyl ligand described herein, n of the cycloalkyl aminyl nitrogen group can be any value described herein, and M-Xₚ can be any metal compound described herein. In an embodiment, R^{2c} can be any alkyl group described herein, R^{1c}, R^{3c}, R^{4c}, and R⁵ are hydrogen, and M-Xₚ comprises chromium. In some embodiments, R^{2c} can be a C₁ to C₄ alkyl group and R^{1c}, R^{3c}, R^{4c}, and R⁵ is hydrogen, and M-Xₚ comprises chromium. In some embodiments, R^{2c} is a methyl group, R^{1c}, R^{3c}, R^{4c}, and R⁵ are hydrogen, and M-Xₚ comprises chromium. In other non-limiting embodiments, the metal complex utilized in the olefin oligomerization process can have the Structure XI: 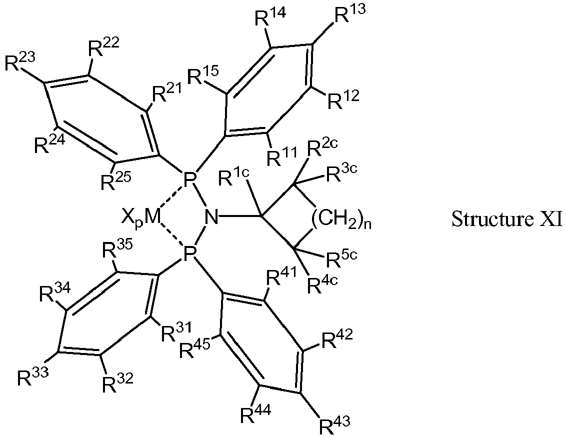 wherein R¹¹, R¹², R¹³, R¹⁴, R¹⁵, R²¹, R²², R²³, R²⁴, R²⁵, R³¹, R³², R³³, R³⁴, R³⁵, R⁴¹, R⁴², R⁴³, R⁴⁴, and R⁴⁵ can be any substituent described herein or have any substituent pattern described herein for the diphosphino aminyl moiety, R^{1c}, R^{2c}, R^{3c}, R^{4c}, and R^{5c} can be any substituent described herein, have any substituent pattern described herein for the cycloalkyl aminyl nitrogen group, and/or have any substituent pattern necessary to meet a particular aspect of the diphosphino aminyl ligand described herein, n of the cycloalkyl aminyl nitrogen group can be any value described herein, and M-Xₚ can be any metal compound described herein. In an embodiment, R^{2c} can be any alkyl group described herein, R^{1c}, R^{3c}, R^{4c}, and R⁵ are hydrogen, and M-Xₚ comprises chromium. In some embodiments, R^{2c} can be a C₁ to C₄ alkyl group and R^{1c}, R^{3c}, R^{4c}, and R⁵ is hydrogen, and M-Xₚ comprises chromium. In some embodiments, R^{2c} is a methyl group, R^{1c}, R^{3c}, R^{4c}, and R⁵ are hydrogen, and M-Xₚ comprises chromium. In yet other non-limiting embodiments, the metal complex utilized in the olefin oligomerization process can have the Structure XII: 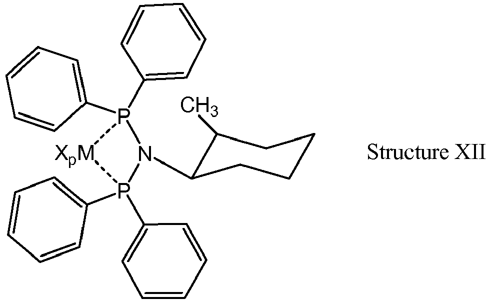 wherein M-Xₚ can be any metal compound described herein. In some embodiments, M-Xₚ comprises chromium.

In a non-limiting embodiment, the metal complex utilized in the olefin oligomerization process can comprise a metal compound complexed to a heteroatomic ligand comprising multiple diphosphino aminyl moieties and a linking group linking the aminyl nitrogen atoms of the diphosphino aminyl moieties. Generally, the metal complex described as a metal compound complexed to a heteroatomic ligand comprising multiple diphosphino aminyl moieties and a linking group linking the aminyl nitrogen atoms of the diphosphino aminyl moieties can be any metal compound complexed to a heteroatomic ligand comprising multiple diphosphino aminyl moieties and a linking group linking the aminyl nitrogen atoms of the diphosphino aminyl moieties described herein. In some embodiments, the metal complex utilized in the olefin oligomerization process can comprise a metal compound complexed to a heteroatomic ligand comprising only 2 diphosphino aminyl moieties and a linking group linking the aminyl nitrogen atoms of the two diphosphino aminyl moieties.

In a non-limiting embodiment, the metal complex utilized in the olefin oligomerization process can have the Structure XVI: 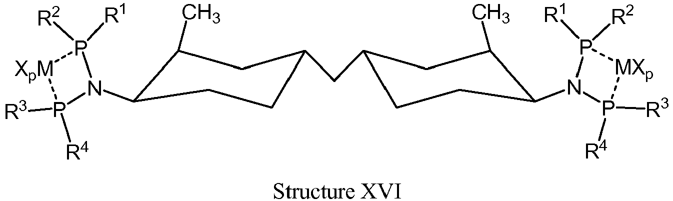 wherein R¹, R², R³, and R⁴ can be any substituent or have any substituent pattern described herein for the diphosphino aminyl moiety, and M-Xₚ can be any metal compound described herein. In some embodiments, M-Xₚ comprises chromium. In other embodiments, the metal complex utilized in the olefin oligomerization process can have the Structure XVII: 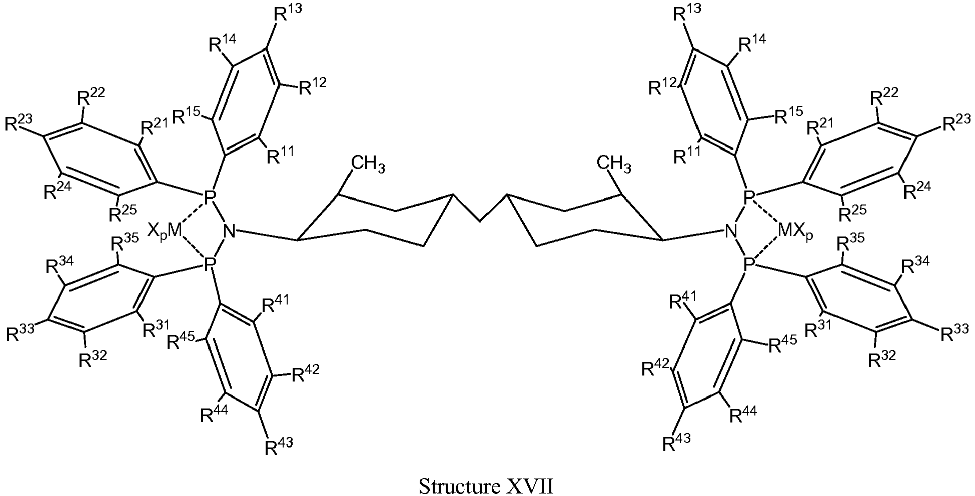 wherein R¹¹, R¹², R¹³, R¹⁴, R¹⁵, R²¹, R²², R²³, R²⁴, R²⁵, R³¹, R³², R³³, R³⁴, R³⁵, R⁴¹, R⁴², R⁴³, R⁴⁴, and R⁴⁵ can be any substituent or have any substituent pattern described herein for the diphosphino aminyl moiety, M-Xₚ can be any metal compound described herein. In some embodiments, M-Xₚ comprises chromium. In yet other embodiments, the metal complex utilized in the olefin oligomerization process can have the Structure XVIII. 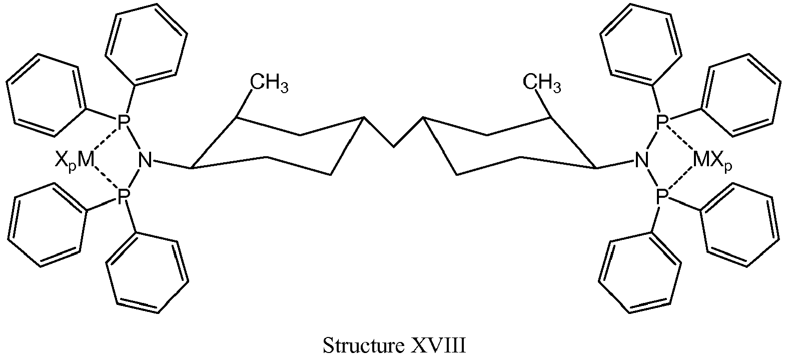 wherein -Xₚ can be any metal compound described herein. In some embodiments, M-Xₚ comprises chromium.

In an embodiment, a solvent utilized in any mixture including the olefin and the catalyst system (or utilized to form the olefin product) can be hydrocarbon solvent, a halogenated hydrocarbon solvent, or any combination thereof; alternatively, a hydrocarbon solvent; or alternatively, a halogenated hydrocarbon solvent. In some embodiments, a solvent utilized in any mixture including the olefin and the catalyst system (or utilized to form the olefin product) can be an aliphatic hydrocarbon solvent, a halogenated aliphatic hydrocarbon solvent, an aromatic hydrocarbon solvent, a halogenated aromatic solvent, or any combination thereof; alternatively, an aliphatic hydrocarbon solvent, a halogenated aliphatic hydrocarbon solvent, or any combination thereof; alternatively, an aromatic hydrocarbon solvent, a halogenated aromatic solvent, or any combination thereof; alternatively, an aliphatic hydrocarbon solvent; alternatively, a halogenated aliphatic hydrocarbon solvent; alternatively, an aromatic hydrocarbon solvent; or alternatively, a halogenated aromatic solvent. General and specific hydrocarbon solvents, halogenated hydrocarbon solvents, aliphatic hydrocarbon solvents, halogenated aliphatic hydrocarbon solvents, aromatic hydrocarbon solvents, and halogenated aromatic solvents are described herein and can be utilized without limitation to further describe the olefin oligomerization process described herein.

In some embodiments, the solvent utilized in preparing the catalyst system and the solvent utilized in any mixture including the olefin and the catalyst system (or utilized to form the olefin product) can be the same; or alternatively can be different. In an embodiment, the solvent utilized in preparing the catalyst system and the solvent utilized in any mixture including the olefin and the catalyst system (or utilized to form the olefin product) can have a boiling point which allows for its easy separation (e.g. by distillation) from the olefin oligomer product or olefin polymer product.

Generally, the olefin which can be oligomerized (which can also be referred to as the olefin monomer) can comprise, or consist essentially of, a C₂ to C₃₀ olefin; alternatively, a C₂ to C₁₆ olefin; or alternatively, a C₂ to C₁₀ olefin. In an embodiment, the olefin (or olefin monomer) can be an alpha olefin; alternatively, a linear alpha olefin; or alternatively a normal alpha olefin. In an embodiment, the olefin (or olefin monomer) can comprise, or consist essentially of, ethylene, propylene, or a combination thereof; alternatively ethylene; or alternatively, propylene. When the olefin (or olefin monomer) can consist essentially of ethylene, the olefin oligomerization process can be an ethylene oligomerization process.

In an aspect, the olefin oligomerization process can be an olefin trimerization process; alternatively, an olefin tetramerization process; or alternatively, an olefin trimerization and tetramerization process. When the olefin is ethylene, the olefin oligomerization process can be an ethylene trimerization process; alternatively, an ethylene tetramerization process; or alternatively, an ethylene trimerization and tetramerization process. When the process is an ethylene trimerization process, the olefin product can comprise hexene; or alternatively, can comprise 1-hexene. When the process is an ethylene tetramerization process, the olefin product can comprise octene; or alternatively, can comprise 1-octene. When the process is an ethylene trimerization and tetramerization process, the olefin product can comprise hexene and octene; or can comprise 1-hexene and 1-octene.

Unless specified otherwise, the terms contacted, combined, and "in the presence of" refer to any addition sequence, order, or concentration for contacting or combining two or more components of the oligomerization process. Combining or contacting of oligomerization components, according to the various methods described herein can occur in one or more contact zones under suitable contact conditions such as temperature, pressure, contact time, flow rates, etc. The contact zone can be disposed in a vessel (e.g. a storage tank, tote, container, mixing vessel, reactor, etc.), a length of pipe (e.g. a tee, inlet, injection port, or header for combining component feed lines into a common line), or any other suitable apparatus for bringing the components into contact. The processes can be carried out in a batch or continuous process as is suitable for a given embodiment, with physical parameters of the contact zone being specified accordingly.

In an embodiment, the olefin oligomerization can be a continuous process carried out in one or more reactors. In some embodiments, the continuous olefin oligomerization can comprise a loop reactor, a tubular reactor, a continuous stirred tank reactor (CSTR), or combinations thereof. In other embodiments, the continuous olefin oligomerization reactor can be a loop reactor; alternatively, a tubular reactor; or alternatively, a continuous stirred tank reactor (CSTR). In other embodiments, the continuous olefin oligomerization reactor can be employed in the form of different types of continuous reactors in combination, and in various arrangements.

Suitable oligomerization process conditions such as temperatures, pressures and times can be impacted by a number of factors such as the metal complex stability, metal complex activity, cocatalyst identity, cocatalyst activity, desired product distribution, and/or desired product purity among others. Provided the teachings of the present disclosure, one skilled in the art will recognize how to adjust the oligomerization process conditions to achieve the desired objectives.

The concentration of the diphosphino aminyl complexed metal compound (or metal compound) can be any concentration needed to produce the desired oligomerization product. In an embodiment, the concentration of the diphosphino aminyl complexed metal compound (or metal compound) can be greater than or equal to 5 x 10⁻⁶ equivalents/liter; alternatively, greater than or equal to 1 x 10⁻⁵ equivalents/liter; or alternatively, greater than or equal to 2.5 x 10⁻⁵ equivalents/liter. In other embodiments, the concentration of the diphosphino aminyl complexed metal compound can range from 5 x 10⁻⁶ to 5 x 10⁻³ equivalents/liter; alternatively, range from 1 x 10⁻⁵ to 1 x 10⁻⁴ equivalents/liter; or alternatively, range from 2.5 x 10⁻⁵ to 6 x 10⁻⁵ equivalents/liter.

The reaction pressure of the olefin oligomerization can be any reaction pressure required to produce the desired oligomerization product. In some embodiments, the olefin oligomerization gauge pressure can be greater than or equal to 1 psi (6.9 kPa); alternatively, greater than or equal to 50 psi (344 kPa); alternatively, greater than or equal to 100 psi (689 kPa); or alternatively, greater than or equal to 150 psi (1.0 MPa). In other embodiments, the oligomerization gauge pressure can range from 1 psi (6.9 kPa) to 5,000 psi (34.5 MPa); alternatively, 50 psi (344 kPa) to 4,000 psi (27.6 MPa); alternatively, 100 psi (689 kPa) to 3,000 psi (20.9 MPa); or alternatively, 150 psi (1.0 MPa) to 2,000 psi (13.8 MPa). In embodiments wherein the monomer is a gas (e.g. ethylene), the oligomerization can be carried out under a monomer gas pressure. When the olefin oligomerization reaction produces alpha olefins, the reaction pressure can be the monomer ethylene pressure. In some embodiments, the ethylene gauge pressure can be greater than or equal to 1 psi (6.9 kPa); alternatively, greater than or equal to 50 psi (344 kPa); alternatively, greater than or equal to 100 psi (689 kPa); or alternatively, greater than or equal to 150 psi (1.0 MPa). In other embodiments, the ethylene gauge pressure can range from 1 psi (6.9 kPa) to 5,000 psi (34.5 MPa); alternatively, 50 psi (344 kPa) to 4,000 psi (27.6 MPa); alternatively, 100 psi (689 kPa) to 3,000 psi (20.9 MPa); or alternatively, 150 psi (1.0 MPa) to 2,000 psi (13.8 MPa). In some cases when ethylene is the monomer, inert gases can form a portion of the total reaction pressure. In the cases where inert gases form a portion of the reaction pressure, the previously stated ethylene pressures can be the applicable ethylene partial pressures of the oligomerization reaction. In the situation where the monomer provides all or a portion of the oligomerization reaction pressure, the reaction system pressure can decrease as the gaseous monomer is consumed. In this situation, additional gaseous monomer and/or inert gas can be added to maintain a desired oligomerization reaction pressure. In an embodiment, additional gaseous monomer can be added to the oligomerization reaction at a set rate (e.g. for a continuous flow reactor), at different rates (e.g. to maintain a set system pressure in a batch reactor). In other embodiments, the oligomerization reaction pressure can be allowed to decrease without adding any additional gaseous monomer and/or inert gas.

In embodiments wherein hydrogen is contacted with the ethylene, and the catalyst system, hydrogen can be added in any amount that produces the desired effect described herein. In some embodiments, the hydrogen partial gauge pressure can be greater than or equal to 1 psi (kPa); alternatively, greater than or equal to 5 psi (34 kPa); alternatively, greater than or equal to 10 psi (69 kPa); or alternatively, greater than or equal to 15 psi (100 kPa). In other embodiments, the hydrogen partial gauge pressure can range from 1 psi (6.9 kPa) to 500 psi (3.5 MPa); alternatively, 5 psi (34 kPa) to 400 psi (2.8 MPa); alternatively, 10 psi (69 kPa) to 300 psi (2.1 MPa); or alternatively, 15 psi (100 kPa) to 200 psi (1.4 MPa).

In an embodiment, a condition to form an olefin product can include an oligomerization temperature. Generally, the oligomerization temperature can be any temperature which forms the desired olefin product or polymer product. In some embodiments, the reaction temperature for the oligomerization can range from -20 °C to 200 °C. In some embodiments, the oligomerization temperature ranges from 0 °C to 150 °C; alternatively, ranges from 10 °C to 150 °C; alternatively, ranges from 20 °C to 100 °C; or alternatively, ranges from 30 °C to 80 °C.

In an embodiment, a condition to form an olefin product can include an oligomerization time or polymerization time. Generally, the oligomerization time can be any time that produces the desired quantity of olefin product; or alternatively, provide a desired catalyst system productivity; or alternatively, provide a desired conversion of monomer. In some embodiments, the oligomerization reaction time can range from 1 minute to 8 hours; alternatively, from 5 minutes to 5 hours; alternatively, from 10 minutes to 2.5 hours; or alternatively, from 15 minutes to 2 hours.

The oligomerization process can comprise additional steps such as deactivating the catalyst and isolating the olefin oligomer.

In an embodiment, the olefin oligomerization can have a single pass olefin conversion of ethylene of at least 30 wt. % percent; alternatively, at least 35 wt. % percent; alternatively, at least 40 wt. %; percent; or alternatively, at least 45 wt. % percent. When the olefin is ethylene, the olefin conversion is ethylene conversion

In an embodiment, the olefin oligomerization process produces an olefin product comprising an olefin trimer, an olefin tetramer, or mixtures thereof. In some embodiments, when the olefin is ethylene the olefin oligomerization is an ethylene oligomerization process. In some embodiments, the olefin oligomerization produces an alpha olefin product having at least four carbon atoms. In an embodiment, the ethylene oligomerization process produces an olefin product comprising an ethylene trimer (e.g. hexene, or alternatively, 1-hexene), an ethylene tetramer (e.g. octene, or alternatively, 1-octene), or a combination thereof; alternatively, hexene; alternatively, octene; alternatively hexene and octene. In other embodiments, the ethylene oligomerization produces an olefin product comprising 1-hexene, 1-octene, or a combination thereof; alternatively, 1-hexene; alternatively, 1-octene; alternatively 1-hexene and 1-octene. In an embodiment, when the olefin is ethylene and the process produces an alpha olefin (e.g. 1-hexene, 1-octene, or a combination thereof) the olefin oligomerization process can be an alpha olefin production process.

In an embodiment, the ethylene oligomerization process can produce a liquid oligomer product mixture comprising at least 60 wt. % C₆ and C₈ olefins. In some embodiments, the liquid oligomer product mixture of the alpha olefin production process comprises greater than or equal to 70 wt. % C₆ and C₈ olefins; alternatively, greater than or equal to 75 wt. % C₆ and C₈ olefins; alternatively, greater than or equal to 80 wt. % C₆ and C₈ olefins; alternatively, greater than or equal to 85 wt. % C₆ and C₈ olefins; or alternatively, greater than or equal to 90 wt. % C₆ and C₈ olefins. In other embodiments, the liquid oligomer product mixture of the alpha olefin production process comprises from 60 to 99.5 wt. % C₆ and C₈ olefins; alternatively, from 70 to 99 wt. % C₆ and C₈ olefins; alternatively, from 75 to 97.5 wt. % C₆ and C₈ olefins; or alternatively, from 80 to 95 wt. % C₆ and C₈ olefins. Throughout this application, liquid oligomerized reactor effluent (or liquid oligomer product) refers to the oligomerized product having from 4 to 18 carbon atoms. The liquid oligomerized reactor effluent can also be referred to as a liquid oligomer poroduct or liquid oligomer product mixture.

In an embodiment, the liquid oligomerized reactor effluent (or liquid oligomer product, or liquid oligomerized product mixture) of the alpha olefin production process that comprises greater than or equal to 60 wt. % C₆ and C₈ olefins, or any other percent of C₆ and C₈ olefins described herein, comprises at least 30 wt. % C₈ olefins; alternatively, at least 40 wt. % C₈ olefins; alternatively, at least 45 wt. % C₈ olefins; alternatively, at least 50 wt. % C₈ olefins; or alternatively, at least 55 wt. % C₈ olefins. In other embodiments, the liquid oligomerized product mixture of the alpha olefin production process that comprises greater than or equal to 60 wt. % C₆ and C₈ olefins, or any other percent C₆ and C₈ olefins described herein, comprises from 30 to 80 wt % C₈ olefins; or alternatively, comprises from 40 to 70 wt % C₈ olefins. In yet other embodiments, the liquid oligomerized product mixture of the alpha olefin production process that comprises greater than or equal to 60 wt. % C₆ and C₈ olefins, or any other percent C₆ and C₈ olefins described herein, has a mass (or weight) ratio of C₈ olefins to o C₈ olefins ranging from 0.5 to 2.4; alternatively, from 0.7 to 2.2; or alternatively, from 0.9 to 2.0. The mass (or weight) ratio of C₈ olefins to C₆ olefins can also be referred to as an oligomerized product C₈:C₆ mass (or weight) ratio, or a C₈:C₆ oligomer product mass (or weight) ratio.

In an embodiment, the C₆ olefins comprises greater than or equal to 85 wt. % 1-hexene. In some embodiments, the C₆ olefins comprises greater or equal to 87.5 wt. % 1-hexene; alternatively, greater than or equal to 90 wt % 1-hexene; alternatively, greater than or equal to 91 wt. % 1-hexene; or alternatively, greater than or equal to 92 wt. % 1-hexene. In other embodiments, the C₆ olefins comprises from 85 to 99 wt % 1-hexene; alternatively, from 87.5 to 98 wt % 1-hexene; alternatively, from 90 to 97 wt % 1-hexene; alternatively, from 91 to 96 wt % 1-hexene. In an embodiment, the C₈ olefins comprises greater than or equal to 97 wt. % 1-octene. In some embodiments, the C₈ olefins comprises greater than or equal to 97.5 wt. % 1-octene; alternatively, greater than or equal to 98 wt % 1-octene; alternatively, greater than or equal to 98.5 wt. % 1-octene; or alternatively, greater than or equal to 99 wt. % 1-octene. In other embodiments, the C₈ olefins comprises from 97 to 99.8 wt % 1-octene; alternatively, from 97.5 to 99.6 wt % 1-octene; alternatively, from 98 to 99.7 wt % 1-octene; alternatively, from 98.5 to 99.5 wt % 1-octene. In an embodiment, the C₈ olefins comprises greater than or equal to 90 wt. % 1-hexene and the C₈ olefins comprises greater than or equal to 97 wt. % 1-octene. In some embodiments, the C₈ olefins comprises greater than or equal to 91 wt. % 1-hexene and the C₈ olefins comprises greater than or equal to 97.5 wt. % 1-octene. In some embodiments, the C₈ olefins comprises greater than or equal to 92 wt. % 1-hexene and the C₈ olefins comprises greater than or equal to 98 wt. % 1-octene. In other embodiments, the C₈ olefins comprises from 90 to 97 wt. % 1-hexene and C₈ olefins comprises from 98 to 99.7 wt. % wt. % 1-octene. In yet other embodiments, the C₈ olefins comprises from 91 to 96 wt. % 1-hexene and the C₈ olefins comprises from 98.5 to 99.5 wt. % 1-octene.

It has been discovered that, in some aspects and/or embodiments, aging the catalyst system before contacting the catalyst system with the olefin to be oligomerized can improve aspects of the olefin oligomerization. Firstly it has been observed that aging the catalyst system can decrease the amount of polymer produced in an olefin oligomerization process. Secondly and as a result of the reduction in polymer production, the temperature of the olefin oligomerization using a particular catalyst system can be increased (polymer production usually increases with increasing temperature).

The catalyst system aging impact(s) can be utilized to provide positive benefits to an olefin oligomerization and/or olefin polymerization process. For example, a decrease in polymer produced in an olefin oligomerization process upon aging the catalyst system can reduce the amount of polymer which could adhere to the oligomerization reactor walls or cooling apparatus. The reduction in polymer produced during the olefin oligomerization process can reduce the need to shut down a reactor in order to remove the polymer which can cause fouling. As a second example, increasing the oligomerization temperature can increase the wt. % 1-hexene found in the C₆ olefins produced in an ethylene oligomerization. Further the increase in reaction temperature can increase catalyst system reliability and reproducibility in an ethylene oligomerization.

In any aspect or embodiment wherein a mixture (or catalyst system mixture) comprising a diphosphino aminyl ligand, a metal compound, and a metal alkyl are contacted prior to contacting the olefin, the mixture (or catalyst system mixture) comprising the diphosphino aminyl ligand, a metal compound, and a metal alkyl can be allowed to age for a period of time prior to contacting he mixture (or catalyst system mixture) comprising the diphosphino aminyl ligand, a metal compound, and a metal alkyl with a mixture comprising the olefin. In some embodiments, the mixture (or catalyst system mixture) comprising the diphosphino aminyl ligand, a metal compound, and a metal alkyl can further comprise a solvent.

In any aspect or embodiment wherein a mixture (or catalyst system mixture) comprising a metal compound complexed to a diphosphino aminyl ligand and a metal alkyl are contacted prior to contacting the olefin, the mixture (or catalyst system mixture) comprising metal compound complexed to a diphosphino aminyl ligand and a metal alkyl can be allowed to age for a period of time prior to contacting the mixture (or catalyst system mixture) comprising the diphosphino aminyl ligand, a metal compound, and a metal alkyl with a mixture comprising the olefin. In some embodiments, the mixture (or catalyst system mixture) comprising the metal compound complexed to a diphosphino aminyl ligand and a metal alkyl can further comprise a solvent.

In a non-limiting embodiment, the olefin oligomerization process can comprise: a) preparing a catalyst system; b) allowing the catalyst system to age for a period of time; c) contacting the aged catalyst system with an olefin; and d) forming an olefin oligomer product. In some non-limiting embodiments, the olefin oligomerization process can comprise, a) preparing a catalyst system; b) allowing the catalyst system to age for a period of time; c) contacting the aged catalyst system with an olefin and hydrogen; and d) forming an olefin oligomer product. The catalyst system, olefin, and other features of the olefin oligomerization processt are independently described herein and can be utilized, without limitation to further describe the olefin oligomerization process. In some embodiments, the catalyst system can be prepared in a first solvent. In an embodiment, the olefin, aged catalyst system, and optionally hydrogen, can be contacted in a second solvent. Generally, a solvent in which the catalyst system can be prepared and the solvent in which the olefin and aged catalyst system can be contacted can be the same; or alternatively, can be different. The catalyst system, features of aging the catalyst system, features of the olefin oligomer, and features of the impacts of aging the catalysts system, among other features, are independently described herein and can be utilized, without limitation to further describe the olefin oligomerization. In some embodiments, the first and second solvent can be the same; or alternatively, the first and second solvent can be different. In some embodiments, the olefin oligomer product is formed under conditions suitable for forming an olefin oligomer product. In some embodiments, the benefits of aging the catalyst system can be made in comparison to a comparable system wherein the catalyst system is not aged prior to contact with the olefin. When the catalyst syetem is aged in the substantial absence of the olefin, the benefits of aging the catalyst system can made in comparison to a comparable system wherein the catalyst system is not aged in the substantial absence of the olefin.

In a non-limiting embodiment, the olefin oligomerization process can comprise: a) contacting i) a metal compound complexed to a diphosphino aminyl ligand and ii) metal alkyl to form a mixture; b) aging the mixture; c) contacting the aged mixture with an olefin; and d) forming an olefin oligomer product. In another non-limiting embodiment, the olefin oligomerization process can comprise: a) contacting i) a diphosphino aminyl ligand, ii) a metal compound, and iii) a metal alkyl; b) aging the mixture; c) contacting the aged mixture with an olefin; and c) forming an olefin oligomer product. In some embodiments the mixture can further comprise a solvent (e.g. a first solvent). In some embodiments, the mixture and the olefin can be contacted in a solvent (e.g. a second solvent). In yet another non-limiting embodiment, the olefin oligomerization process can comprise: a) contacting i) a metal compound complexed to a diphosphino aminyl ligand, ii) a metal alkyl, and iii) a first solvent to form a mixture; b) aging the mixture; c) contacting the aged mixture with an olefin and a second solvent; and c) forming an olefin oligomer product. In some embodiments, the mixture formed in step a) can comprise, or consist essentially of, i) a metal complex comprising a metal compound complexed to a diphosphino aminyl ligand, ii) a metal alkyl, and iii) a first solvent. In a further non-limiting embodiment, the olefin oligomerization process can comprise: a) contacting i) a diphosphino aminyl ligand, ii) a metal compound, iii) a metal alkyl, and iv) a first solvent to form a mixture; b) aging the mixture; c) contacting the aged mixture with an olefin and a second solvent; and d) forming an olefin oligomer product. In some embodiments, the mixture formed in step a) can comprise, or consist essentially of, i) a metal compound, ii) a diphosphino aminyl ligand, iii) a metal alkyl, and iv) a first solvent. In some embodiments, the olefin oligomer product is formed under conditions suitable for forming an olefin oligomer product.

In other non-limiting embodiments, the olefin oligomerization process can comprise: a) contacting i) Cr(acac)₃ complexed to a diphosphino aminyl ligand, and ii) an aluminoxane to form a mixture; b) aging the mixture; c) contacting the aged mixture with an olefin; and d) forming an olefin oligomer product. In still other embodiments, the olefin oligomerization process can comprise a) contacting i) CrCl₃ complexed to a diphosphino ligand and ii) an aluminoxane to form a mixture; b) aging the mixture; c) contacting the aged mixture with an olefin; and d) forming an olefin oligomer product. In other non-limiting embodiments, the olefin oligomerization process can comprise: a) contacting i) Cr(acac)₃, ii) a diphosphino aminyl ligand, and iii) an aluminoxane to form a mixture; b) aging the mixture; c) contacting the aged mixture with an olefin; and d) forming an olefin oligomer product. In still other embodiments, the olefin oligomerization process can comprise a) contacting i) CrCl₃, ii) a diphosphino ligand, and iii) an aluminoxane to form a mixture; b) aging the mixture; c) contacting the aged mixture with an olefin; and d) forming an olefin oligomer product. In some embodiments the mixture can further comprise a solvent (e.g. a first solvent). In some embodiments the mixture can further comprise a solvent (e.g. a first solvent). In some embodiments, the mixture formed in step a) can comprise, or consist essentially of, i) Cr(acac)₃ complexed to a diphosphino aminyl ligand, ii) an aluminoxane, and iii) a first solvent; alternatively, i) CrCl₃ complexed to a diphosphino aminyl ligand, ii) an aluminoxane, and iii) a first solvent; alternatively, i) Cr(acac)₃, ii) a diphosphino aminyl ligand, iii) an aluminoxane, and iv) a first solvent; or alternatively, i) CrCl₃, ii) a diphosphino aminyl ligand, iii) an aluminoxane, and iv) a first solvent. In some embodiments, the step of contacting the aged mixture with the olefin can be a step of contacting the aged mixture with an olefin and hydrogen. In an embodiment, the olefin, aged mixture, and optionally hydrogen, can be contacted in a second solvent. Generally, a solvent in which the mixture can be prepared and the solvent in which the olefin, aged mixtute, and optionally hydrogen can be contacted can be the same; or alternatively, can be different. The catalyst system, features of aging the catalyst system, features of the olefin oligomer, and features of the impacts of aging the catalysts system, among other features, are independently described herein and can be utilized, without limitation to further describe the olefin oligomerization. In some embodiments, the first and second solvent can be the same; or alternatively, the first and second solvent can be different. In some embodiments the mixture can be aged in the substantial absence of the olefin; or alternatively, in the absence of the olefin. In some embodiments, the olefin oligomer product is formed under conditions suitable for froming an olefin oligomer product.

In an embodiment, the contacting of the mixture (or catalyst system mixture) components and/or aging of the mixture (or catalyst system mixture) can be perfomed prior to contacting the the mixture (or catalyst system mixture) with the olefin to be oligomerized. In another embodiment, the contacting of the mixture (or catalyst system mixture) components and/or aging of the mixture (or catalyst system mixture) can be perfomed in the substantial absence of the olefin to be oligomerized. In some embodiments, the contacting of the mixture (or catalyst system mixture) components and/or aging of the mixture (or catalyst system mixture) can be perfomed in the absence of the olefin to be oligomerized. In a non-limiting embodiment, a substantial absence of olefin can be a molar ratio of olefin to metal complex or metal compound up to 5:1, 4:1, 3:1, 2:1, 1:1, 0.5:1, 0.25:1, or 0.1:1. In some non-limiting embodiments, when the olefin is a gas, a substantial absence of olefin can be an olefin partial pressure of less than 10 psig (69 kPa), 5 psig (34 kPa), 4 psig (28 kPa), 3 psig (21 kPa), 2 psig (14 kPa), 1 psig (7 kPa), or 0.5 psig (3.4 kPa).

In some embodiments, the step of contacting the aged catalyst system mixture with the olefin (and optionally a solvent - e.g. second solvent) can be a step of contacting the aged catalyst system mixture with an olefin and hydrogen. The diphosphino aminyl ligand, the metal compound, metal compound complexed to a diphosphino aminyl ligand, the metal alkyl, the olefin, solvents, features of aging the catalyst system, features of the olefin oligomer, and features of the impacts of aging the catalysts system, among other features, are independently described herein and can be utilized, without limitation to further describe the olefin oligomerization. In some embodiments, the first and second solvent can be the same; or alternatively, the first and second solvent can be different. In some embodiments, the metal alkyl can comprise an aluminoxane. Ratios for diphosphino aminyl ligand to metal salt and ratios for the metal of the metal alkyl to metal of the metal compound complexed to a diphosphino aminyl ligand, among other features, are independently described herein and can be utilized without limitation to further describe the olefin oligomerization.

In the various aspects and embodiments disclosed herein, aging can be carried out in one or more contact zones. A contact zone is a zone in which the components are commingled and/or combined, and thereby contacted. The contact zone can be disposed in a vessel, e.g. a storage tank, tote, container, mixing vessel, reactor, etc.; a length of pipe, e.g. a tee, inlet, injection port, or header for combining component feed lines into a common line, or any other suitable apparatus for bringing the components into contact. As used herein, the terms contacted and combined refer to any addition sequence, order, or concentration for contacting or combining two or more catalyst system components. In some embodiments, contacting of components can occur in one or more upstream contact zone(s) prior to further contacting with other catalyst component(s) in one or more downstream contact zone(s). Where a plurality of contact zones are employed, contacting can occur simultaneously across the contact zones, sequentially across the contact zones, or both, as is suitable for a given embodiment. Contacting can be carried out in a batch or continuous process, as is suitable for a given embodiment.

In an embodiment, aging of the components of the catalyst system is carried out under any conditions sufficient to thoroughly contact the components. For example, aging can be performed in an inert atmosphere, such as, for example, nitrogen, and/or argon.

In an embodiment, the catalyst system can be aged for up 14 days; alternatively, up to 10 days; alternatively, up to 8 days; alternatively, up to 6 days; alternatively, up to 4 days; alternatively, up to 3 days; alternatively, up to 48 hours; alternatively, up to 36 hours; alternatively, up to 24 hours; alternatively, up to 18 hours; alternatively, up to 10 hours; alternatively, up to 8 hours; alternatively, up to 6 hours; alternatively, up to 4 hours; or alternatively, up to 3 hours. The catalyst system can be aged for at least 20 minutes; alternatively, at least 30 minutes; alternatively, at least 40 minutes; or alternatively, at least 50 minutes. In an embodiment, the catalyst system can be aged for a time ranging from any catalyst system aging minimum time disclosed herein to any catalyst system aging maximum time disclosed herein. In some non-limiting embodiments, the catalyst system can be aged from 20 minutes to 4 days; alternatively, from 20 minutes to 3 days; alternatively, from 30 minutes to 48 hours; alternatively, from 30 minutes to 36 hours; alternatively, from 30 minutes to 24 hours; alternatively, from 30 minutes to 18 hours; alternatively, from 30 minutes to 10 hours ; alternatively, from 30 minutes to 8 hours; alternatively, from 30 minutes to 6 hours; alternatively, from 30 minutes to 4 hours; or alternatively, from 30 minutes to 3 hours.

In other embodiments, any catalyst system described herein can be aged at a temperature from 10 °C to 130 °C; alternatively, from 20 °C to 120 °C; or alternatively, from 35 °C to 110 °C. In some embodiments, any catalyst system described herein can be aged under an inert atmosphere. Generally, one will recognize that the temperature at which the catalyst system is aged can have an impact upon the aging time necessary to achieve a reduction in catalyst system polymer production. In any aspect or embodiment, the catalyst system can be aged at a combination of any catalyst system aging time described herein and any aging catalyst system aging temperature described herein.

In an embodiment, a calibration curve can be produced depicting the polymer production of an olefin oligomerization using any aged catalyst system described herein in response to one or more catalyst system aging variables (e.g. time, temperature, or time and temperature). In some embodiments the calibration curve can be depicted graphically as a function of a catalyst system aging variable(s) (e.g. time, temperature, or time and temperature); or alternatively, the calibration curve can be depicted as a predictive equation of a catalyst system aging variable(s) (e.g. time, temperature, or time and temperature). The graphical representation and/or predictive equation relating the polymer production of an olefin oligomerization in response to catalyst aging can be utilized to adjust one or more user and/or process parameters based upon the interpolation or extrapolation of the graphical representation or predictive equation. It is contemplated that in some aspects, the extent to which the polymer production of an olefin oligomerization with respect to catalyst system aging is reduced can fall outside the instantly disclosed ranges and can be larger than would be expected based on the presently disclosed values depending on conditions under which the catalyst system is aged. For example, the catalyst system can be subjected to aging for time periods that are longer than those presently recited and/or at temperatures greater than those presently recited. The effects of aging the catalyst system under such conditions can be subject to the herein mentioned analysis to provide predictive information that can lead one to conditions under which the catalyst system reduces the polymer production in the olefin oligomerization. It is contemplated that given the benefits of this disclosure and using routine experimentation one having ordinary skill in the art can modify the methodologies disclosed herein to reduce the amount of polymer produced in an olefin oligomerization process to a desired value or range. Such modifications fall within the scope of this disclosure.

In an embodiment, any aged catalyst system described herein (using any aging time period described herein and/or any aging temperature described herein) can provide a catalyst system which can produce a reduction in the percentage of polymer produced as compared to an otherwise similar olefin oligomerization utilizing a non-aged catalyst system (or catalyst system mixture). In some embodiments, aging of any catalyst system described herein can reduce (using any aging time period described herein and/or any aging temperature described herein) the amount of polymer produced in an olefin oligomerization process by at least 25 %; alternatively, at least 40 %; alternatively, at least 60 %; alternatively, at least 70 %; alternatively, at least 80 %; alternatively, at least 85 %; alternatively, at least 90 %; or alternatively, at least 95 %. Generally, the decrease in the catalyst system polymer production as a result of aging can be determined by comparing the polymer production of the aged catalyst system to the polymer production of a catalyst system that has been aged for less than 12 minutes.

It has also been discovered that the group attached to the aminyl nitrogen atom of the diphosphino aminyl ligand can impact the aging of the catalyst system. It has been discovered that as the steric bulk of the group attached to the aminyl nitrogen atom of the diphosphino aminyl ligand increases the time required to achieve an effect of catalyst system aging (reduction in the amount of polymer produced in an olefin oligomerization using an aged catalyst system) also increases.

In one embodiment, a bulky substituent(s) can be defined as one wherein the carbon atom attached to the aminyl nitrogen atom of the PNP ligand is a tertiary or quaternary carbon atom; or alternatively, as one wherein the carbon atom of adjacent to the carbon atom attached to the aminyl nitrogen atom of the PNP ligand is a tertiary or quaternary carbon atom. In another embodiment, the bulky substituent(s) can be defined as one wherein the carbon atom that is attached to the aminyl nitrogen atom of the PNP ligand is a tertiary carbon atom; or alternatively, as one wherein the carbon atom that is adjacent to the carbon atom attached to the aminyl nitrogen atom of the PNP ligand is a tertiary carbon atom. Yet, in another embodiment, the bulky substituent(s) can be defined as one wherein the carbon atom that is attached to the aminyl nitrogen atom of the PNP ligand is a quaternary carbon atom; or alternatively, as one wherein the carbon atom that is adjacent to the carbon atom attached to the central nitrogen atom of the PNP ligand is a quaternary carbon atom.

In another embodiment, the bulky substituent(s) can be defined as one wherein the carbon atom that is attached to the aminyl nitrogen atom of the PNP ligand is attached to 3 or 4 carbon atoms; or alternatively, as one wherein the carbon atom that is adjacent to the carbon atom attached to the aminyl nitrogen atom of the PNP ligand is attached to 3 or 4 carbon atoms. In another embodiment, the bulky substituent(s) can be defined as one wherein the carbon atom that is attached to the aminyl nitrogen atom of the PNP ligand is attached to 3 carbon atoms; or alternatively, as one wherein the carbon atom that is adjacent to the carbon atom attached to the aminyl nitrogen atom of the PNP ligand is attached to 3 carbon atoms. Yet, in another embodiment, the bulky substituent(s) can be defined as one wherein the carbon atom that is attached to the aminyl nitrogen atom of the PNP ligand is attached to 4 carbon atoms; or alternatively, as one wherein the carbon atom that is adjacent to the carbon atom attached to the aminyl nitrogen atom of the PNP ligand is attached to 4 carbon atoms.

In an embodiment, the bulky group can be an organyl group; alternatively, a organyl group consisting of inert functional groups; or alternatively a hycrocarbyl group. In some embodiment, the bulky group can be an alkyl group, a substituted alkyl group, a cycloalkyl group, a substituted cycloalkyl group, an aromatic group, a substituted aromatic group, an aryl group, a substituted aryl group, an aralkyl group, or a substituted aralkyl group; alternatively, an alkyl group, a substituted alkyl group, a cycloalkyl group, a substituted cycloalkyl group, an aryl group, a substituted aryl group, an aralkyl group, or a substituted aralkyl group; alternatively, an alkyl group or a substituted alkyl group; alternatively, a cycloalkyl group or a substituted cycloalkyl group; alternatively, an aryl group or a substituted aryl group; alternatively, an aralkyl group or a substituted aralkyl group; alternatively, an alkyl group; alternatively, a substituted alkyl group; alternatively, a cycloalkyl group; alternatively, a substituted cycloalkyl group; alternatively, an aryl group; alternatively, a substituted aryl group; alternatively, an aralkyl group; or alternatively, a substituted aralkyl group. These groups are generally and specifically described herein and these descriptions can be utilized to further describe the bulky substituent that is attached to the aminyl nitrogen atom of the PNP ligand which can be used in embodiments disclosed herein. One can readily discern, utilizing the present disclosure, whether or not a particular group attached to the aminyl nitrogen atom of a diphosphino aminyl ligand described herein is a bulkyl group.

In an aspect, the amount of polymer produced by the olefin oligomerization process can be reduced by providing and/or controlling the aging of two or more of the catalyst system components. Alternatively, the amount of polymer produced by the olefin oligomerization process can be reduced by contacting a catalyst system comprising i) a metal compound, ii) a diphosphino aminyl ligand and iii) a metal alkyl for a time period optimal to reduce polymer formation prior to introduction of a monomer. Alternatively, while not wishing to be bound by theory, it is also believed that the amount of polymer produced by olefin oligomerization process can be reduced by providing and/or controlling one or more olefin production (or oligomerization) parameters. In an embodiment, the amount of polymer produced by the olefin oligomerization process can be reduced by providing and/or controlling a concentration of the diphosphino aminyl complexed metal compound. In some embodiments, the amount of polymer produced by the olefin oligomerization process can be reduced by providing and/or controlling a metal of the metal alkyl to the catalyst metal molar ratio. In other embodiments, the amount of polymer produced by the olefin oligomerization process can be reduced by providing and/or controlling a diphosphino aminyl moiety to catalyst metal equivalent ratio. In yet other embodiments, the amount of polymer produced by the olefin oligomerization process can be reduced by providing and/or controlling a hydrogen partial pressure during the olefin oligomerization.

In an embodiment, while not wishing to be bound by theory, the amount of polymer produced by the olefin oligomerization process can be reduced by providing and/or controlling an olefin production process parameter selected from the group consisting of a) a concentration of diphosphino aminyl complexed metal compound, b) a metal of the metal alkyl to the metal of the metal complex or metal compound molar ratio, c) a diphosphino aminyl moiety to metal of the metal compound molar ratio, d) a hydrogen partial pressure during the olefin oligomerization, e) increased duration of aging of the catalyst system, or any combination of a, b, c, d, and e thereof. In some embodiments, a method for reducing an amount of polymer produced in an olefin production process comprises a) contacting an olefin, a diphosphino aminyl ligand metal complex, a metal alkyl, and hydrogen, b) providing and/or controlling an olefin production process parameter selected from the group consisting of i) a concentration of diphosphino aminyl complexed metal compound, ii) a metal of the metal alkyl to the metal of the metal complex or metal compound molar ratio, iii) a diphosphino aminyl moiety to metal of the metal compound molar ratio, iv) a hydrogen partial pressure, v) a catalyst system aging time, or any combination of i, ii, iii, iv , and v thereof, and forming an olefin oligomer product.

Various aspect and embodiments described herein refer non-hydrogen substituents such as halogen (or halo, halide), hydrocarbyl, hydrocarboxy, alkyl, and/or alkoxy substituents. The non-hydrogen substituents of any aspect or embodiment calling for a substituent can be a halide, a C₁ to C₁₅ hydrocarbyl group, or a C₁ to C₁₅ hydrocarboxy group; alternatively, a halide or a C₁ to C₁₅ hydrocarbyl group; alternatively, a halide or a C₁ to C₁₅ hydrocarboxy group; alternatively, a C₁ to C₁₅ hydrocarbyl group or a C₁ to C₁₅ hydrocarboxy group; alternatively, a halide; alternatively, a C₁ to C₁₅ hydrocarbyl group; or alternatively, a C₁ to C₁₅ hydrocarboxy group. In some embodiments, the non-hydrogen substituents of any aspect or embodiment calling for a substituent can be a halide, a C₁ to C₁₀ hydrocarbyl group, or a C₁ to C₁₀ hydrocarboxy group; alternatively, a halide or a C₁ to C₁₀ hydrocarbyl group; alternatively, a halide or a C₁ to C₁₀ hydrocarboxy group; alternatively, a C₁ to C₁₀ hydrocarbyl group or a C₁ to C₁₀ hydrocarboxy group; alternatively, a halide; alternatively, a C₁ to C₁₀ hydrocarbyl group; or alternatively, a C₁ to C₁₀ hydrocarboxy group. In other embodiments, the non-hydrogen substituents of any aspect or embodiment calling for a substituent can be a halide, a C₁ to C₅ hydrocarbyl group, or a C₁ to C₅ hydrocarboxy group; alternatively, a halide or a C₁ to C₅ hydrocarbyl group; alternatively, a halide or a C₁ to C₅ hydrocarboxy group; alternatively, a C₁ to C₅ hydrocarbyl group or a C₁ to C₅ hydrocarboxy group; alternatively, a halide; alternatively, a C₁ to C₅ hydrocarbyl group; or alternatively, a C₁ to C₅ hydrocarboxy group.

In an embodiment, any halide substituent of any aspect or embodiment calling for a substituent can be a fluoride, chloride, bromide, or iodide; alternatively, a fluoride or chloride. In some embodiments, any halide substituent of any aspect or embodiment calling for a substituent can be a fluoride; alternatively, a chloride; alternatively, a bromide; or alternatively, an iodide.

In an embodiment, any hydrocarbyl substituent can be an alkyl, an aryl, or an aralkyl group; alternatively, an alkyl group; alternatively, an aryl group; or alternatively, an aralkyl group. In an embodiment, any alkyl substituent of any aspect or embodiment calling for a substituent can be a methyl, an ethyl, an n-propyl, an isopropyl, an n-butyl, a sec-butyl, an isobutyl, a tert-butyl, an n-pentyl, a 2-pentyl, a 3-pentyl, a 2-methyl-1-butyl, a tert-pentyl, a 3-methyl-1-butyl, a 3-methyl-2-butyl, a neo-pentyl, or a n-hexyl group; alternatively, a methyl, ethyl, n-propyl, n-butyl, isobutyl, n-pentyl, 2-methyl-1-butyl, 3-methyl-1-butyl, neo-pentyl, or n-hexyl group; alternatively, a methyl, an ethyl, an isopropyl, a tert-butyl, or a neo-pentyl group; alternatively, a methyl group; alternatively, an ethyl group; alternatively, an isopropyl group; alternatively, a tert-butyl group; or alternatively, a neo-pentyl group. In an embodiment, any aryl substituent of any aspect or embodiment calling for a substituent can be phenyl, a tolyl, a xylyl, or a 2,4,6-trimethylphenyl group; alternatively, a phenyl group; alternatively, a tolyl group, alternatively, a xylyl group; or alternatively, a 2,4,6-trimethylphenyl group. In an embodiment, any aralkyl substituent of any aspect or embodiment calling for a substituent can be benzyl or an ethylphenyl group (2-phenyleth-1-yl or 1-phenyleth-1-yl); alternatively, a benzyl group; alternatively, an ethylphenyl group; alternatively a 2-phenyleth-1-yl group; or alternatively, a 1-phenyleth-1-yl group.

In an embodiment, any hydrocarboxy substituent of any aspect or embodiment calling for a substituent can be an alkoxy, an aryloxy, or an aralkoxy group; alternatively, an alkoxy group; alternatively, an aryloxy group, or an aralkoxy group. In an embodiment, any alkoxy substituent of any aspect or embodiment calling for a substituent can be a methoxy, an ethoxy, an n-propoxy, an isopropoxy, an n-butoxy, a sec-butoxy, an isobutoxy, a tert-butoxy, an n-pentoxy, a 2-pentoxy, a 3-pentoxy, a 2-methyl-1-butoxy, a tert-pentoxy, a 3-methyl-1-butoxy, a 3-methyl-2-butoxy, or a neo-pentoxy group; alternatively, a methoxy, an ethoxy, an isopropoxy, a tert-butoxy, or a neo-pentoxy group; alternatively, a methoxy group; alternatively, an ethoxy group; alternatively, an isopropoxy group; alternatively, a tert-butoxy group; or alternatively, a neo-pentoxy group. In an embodiment, any aryloxy substituent of any aspect or embodiment calling for a substituent can be phenoxy, a toloxy, a xyloxy, or a 2,4,6-trimethylphenoxy group; alternatively, a phenoxy group; alternatively, a toloxy group, alternatively, a xyloxy group; or alternatively, a 2,4,6-trimethylphenoxy group. In an embodiment, any aralkoxy substituent of any aspect or embodiment calling for a substituent can be benzoxy group.

The methods described herein can utilize one or more solvents. Solvents which can be utilized in aspects of the present disclosure include without limitation water, hydrocarbons, halogenated hydrocarbons, ethers, carbonates, esters, ketones, aldehydes, alcohols, nitriles and combinations thereof. In some embodiments, an aspect of the invention may call for a polar solvent. Polar solvents which can be utilized include without limitation water ethers, carbonates, esters, ketones, aldehydes, alcohols, nitriles, and mixtures thereof; alternatively, ethers, carbonates, esters, ketones, aldehydes, alcohols, nitriles, and mixtures thereof; alternatively, ethers, esters, ketones, alcohols, nitriles, and mixtures thereof; alternatively, ethers; alternatively, carbonates; alternatively, esters; alternatively, ketones; alternatively, aldehydes; alternatively, alcohols; or alternatively, nitriles. In some embodiments, an aspect of the invention may call for an aprotic polar solvent. Aprotic polar solvents which can be utilized include without limitation ethers, esters, ketones, aldehydes, nitriles, and mixtures thereof; alternatively, ethers, nitriles and mixtures thereof; alternatively, esters, ketones, aldehydes and mixtures thereof; alternatively, ethers; alternatively, esters; alternatively, ketones; alternatively, aldehydes; or alternatively, nitriles. In other embodiments, an aspect of the disclosure may call for a non-polar solvent. Non-polar solvents include without limitation hydrocarbons, halogenated hydrocarbons, or mixtures thereof; alternatively, a hydrocarbon; or alternatively, a halogenated hydrocarbon. In another embodiment, an aspect of the present disclosure may call for a solvent that is substantially unreactive with a metal alkyl. Solvents which are unreactive with a metal alkyl include without limitation ethers, hydrocarbons, and mixtures thereof; alternatively, ethers; or alternatively, hydrocarbons.

Hydrocarbons and halogenated hydrocarbon can include, for example, aliphatic hydrocarbons, aromatic hydrocarbons, petroleum distillates, halogenated aliphatic hydrocarbons, halogenated aromatic hydrocarbons, or combinations thereof; alternatively aliphatic hydrocarbons, aromatic hydrocarbons, halogenated aliphatic hydrocarbons, halogenated aromatic hydrocarbons, and combinations thereof; alternatively, aliphatic hydrocarbons; alternatively, aromatic hydrocarbons; alternatively, halogenated aliphatic hydrocarbons; or alternatively, halogenated aromatic hydrocarbons.. Aliphatic hydrocarbons which can be useful as a solvent include C₃ to C₂₀ aliphatic hydrocarbons; alternatively C₄ to C₁₅ aliphatic hydrocarbons; or alternatively, C₅ to C₁₀ aliphatic hydrocarbons. The aliphatic hydrocarbons can be cyclic or acyclic and/or can be linear or branched, unless otherwise specified. Non-limiting examples of suitable acyclic aliphatic hydrocarbon solvents that can be utilized singly or in any combination include propane, iso-butane, n-butane, butane (n-butane or a mixture of linear and branched C₄ acyclic aliphatic hydrocarbons), pentane (n-pentane or a mixture of linear and branched C₅ acyclic aliphatic hydrocarbons), hexane (n-hexane or mixture of linear and branched C₆ acyclic aliphatic hydrocarbons), heptane (n-heptane or mixture of linear and branched C₇ acyclic aliphatic hydrocarbons), octane (n-octane or a mixture of linear and branched C₈ acyclic aliphatic hydrocarbons), and combinations thereof; alternatively, iso-butane, n-butane, butane (n-butane or a mixture of linear and branched C₄ acyclic aliphatic hydrocarbons), pentane (n-pentane or a mixture of linear and branched C₅ acyclic aliphatic hydrocarbons), hexane (n-hexane or mixture of linear and branched C₆ acyclic aliphatic hydrocarbons), heptane (n-heptane or mixture of linear and branched C₇ acyclic aliphatic hydrocarbons), octane (n-octane or a mixture of linear and branched C₈ acyclic aliphatic hydrocarbons), and combinations thereof; alternatively, iso-butane, n-butane, butane (n-butane or a mixture of linear and branched C₄ acyclic aliphatic hydrocarbons), pentane (n-pentane or a mixture of linear and branched C₅ acyclic aliphatic hydrocarbons), heptane (n-heptane or mixture of linear and branched C₇ acyclic aliphatic hydrocarbons), octane (n-octane or a mixture of linear and branched C₈ acyclic aliphatic hydrocarbons), and combinations thereof; alternatively, propane; alternatively, iso-butane; alternatively, n-butane; alternatively, butane (n-butane or a mixture of linear and branched C₄ acyclic aliphatic hydrocarbons) ; alternatively, pentane (n-pentane or a mixture of linear and branched C₅ acyclic aliphatic hydrocarbons) ; alternatively, hexane (n-hexane or mixture of linear and branched C₆ acyclic aliphatic hydrocarbons) ; alternatively, heptane (n-heptane or mixture of linear and branched C₇ acyclic aliphatic hydrocarbons) ; or alternatively, octane (n-octane or a mixture of linear and branched C₈ acyclic aliphatic hydrocarbons). Non-limiting examples of suitable cyclic aliphatic hydrocarbon solvents include cyclohexane, methyl cyclohexane; alternatively cyclohexane; or alternatively, methylcyclohexane. Aromatic hydrocarbons which can be useful as a solvent include C₆ to C₂₀ aromatic hydrocarbons; or alternatively, C₆ to C₁₀ aromatic hydrocarbons. Non-limiting examples of suitable aromatic hydrocarbons that can be utilized singly or in any combination include benzene, toluene, xylene (including ortho-xylene, meta-xylene, para-xylene, or mixtures thereof), and ethylbenzene, or combinations thereof; alternatively, benzene; alternatively, toluene; alternatively, xylene (including ortho-xylene, meta-xylene, para-xylene or mixtures thereof); or alternatively, ethylbenzene.

Halogenated aliphatic hydrocarbons which can be useful as a solvent include C₁ to C₁₅ halogenated aliphatic hydrocarbons; alternatively, C₁ to C₁₀ halogenated aliphatic hydrocarbons; or alternatively, C₁ to C₅ halogenated aliphatic hydrocarbons. The halogenated aliphatic hydrocarbons can be cyclic or acyclic and/or can be linear or branched, unless otherwise specified. Non-limiting examples of suitable halogenated aliphatic hydrocarbons which can be utilized include methylene chloride, chloroform, carbon tetrachloride, dichloroethane, trichloroethane, and combinations thereof; alternatively, methylene chloride, chloroform, dichloroethane, trichloroethane, and combinations thereof; alternatively, methylene chloride; alternatively, chloroform; alternatively, carbon tetrachloride; alternatively, dichloroethane; or alternatively, trichloroethane. Halogenated aromatic hydrocarbons which can be useful as a solvent include C₆ to C₂₀ halogenated aromatic hydrocarbons; or alternatively, C₆ to C₁₀ halogenated aromatic hydrocarbons. Non-limiting examples of suitable halogenated aromatic hydrocarbons include chlorobenzene, dichlorobenzene, and combinations thereof; alternatively chlorobenzene and dichlorobenzene.

Ethers, carbonates, esters, ketones, aldehydes, or alcohols which can be useful as a solvent include C₂ to C₂₀ ethers, carbonates, esters, ketones, aldehydes, or alcohols; alternatively, C₂ to C₁₀ ethers, carbonates, esters, ketones, aldehydes, or alcohols; or alternatively, C₂ to C₅ ethers, carbonates, esters, ketones, aldehydes, or alcohols. Suitable ether solvents can be cyclic or acyclic. Non-limiting examples of suitable ethers which can be useful as a solvent include dimethyl ether, diethyl ether, methyl ethyl ether, monoethers or diethers of glycols (e.g., dimethyl glycol ether), furans, substituted furans, dihydrofuran, substituted dihydrofurans, tetrahydrofuran (THF), substituted tetrahydrofurans, tetrahydropyrans, substituted tetrahydropyrans, 1,3-dioxanes, substituted 1,3-dioxanes, 1,4-dioxanes, substituted 1,4-dioxanes, or mixtures thereof. In an embodiment, each substituent of a substituted furan, substituted dihydrofuran, substituted tetrahydrofuran, substituted tetrahydropyran, substituted 1,3-dioxane, or substituted 1,4-dioxane, can be a C₁ to C₅ alkyl group. C₁ to C₅ alkyl substituent group are disclosed herein and can be utilized without limitation of further describe the substituted tetrahydrofuran, dihydrofuran, furan, 1,3-dioxane, or 1,4 dioxane solvents. Non-limiting examples of suitable carbonates which can be utilized as a solvent include ethylene carbonate, propylene carbonate, diethyl carbonate, diethyl carbonate, glycerol carbonate, and combinations thereof. Non-limiting examples of suitable esters which can be utilized as a solvent include ethyl acetate, propyl acetate, butyl acetate, isobutyl isobutyrate, methyl lactate, ethyl lactate, and combinations thereof. Non-limiting examples of suitable ketones which can be utilized as a solvent include acetone, ethyl methyl ketone, methyl isobutyl ketone, and combinations thereof. Non-limiting examples of suitable alcohols which can be utilized as a solvent include methanol, ethanol, propanol, isopropanol, n-butanol, isobutanol, pentanol, hexanol, heptanol, octanol, benzyl alcohol, phenol, cyclohexanol, and the like, or combinations thereof.

In an embodiment, the amount of polymer produced in an olefin oligomerization process can be reduced by adjusting any of the parameters of the aging process. In an embodiment, the amount of polymer produced can be reduced by a) increasing the duration of the aging b) increasing the temperature at which the aging process is carried out; c) adjusting the concentrations of one or more of the catalyst system components involved in the aging process or any combination of a, b, and c.

### EXAMPLES

The invention having been generally described, the following examples are given as particular embodiments of the invention and to demonstrate the practice and advantages thereof. It is understood that the examples are given by way of illustration and are not intended to limit the specification of the claims in any manner.

All PNP-ligands shown below and the corresponding metal complexes were synthesized using established literature methods. For the following experiments the PNP ligands were designated as L1, L2, L3, L4, or L5 and Table 2 provides the structure for each of these ligands.

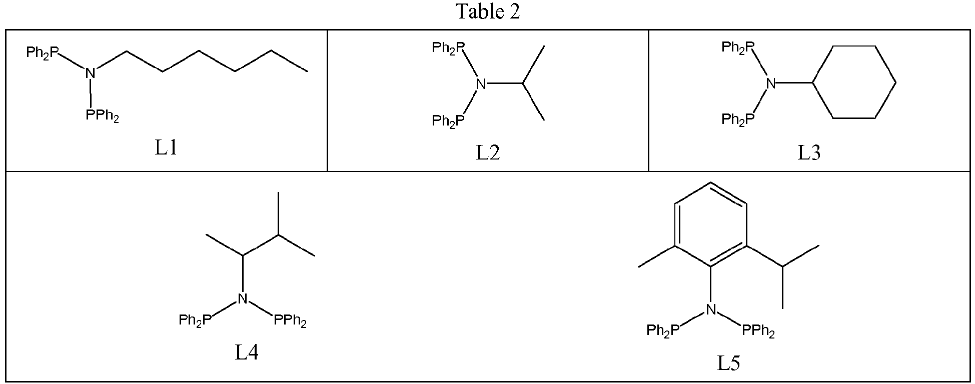

A total of 25 catalyst systems were prepared and subjected to aging under the following conditions:

### Runs 1-13

Catalyst System Peparation: All experiments were performed under an inert atmosphere in a drybox using solvents dried over molecular sieves. A known amount of metal compound, [LCrCl₃]₂, was suspended in ethylbenzene (1.00 g) in a small glass vial. An internal standard (n-nonane, 0.50 g), metal alkyl (MMAO-3A in heptanes, 7.6 wt. % Al) and stir bar were added to the vial. The mixture was stirred for the time and at the temperature indicated in Table 3. The solution was then added to a glass charger containing 400 mL cyclohexane.

### Runs 14-15

Catalyst System Peparation: All experiments were performed under an inert atmosphere in a drybox using solvents dried over molecular sieves. A chromium source, Cr(acac)₃, and PNP-ligand were suspended in ethylbenzene (1.00 g) in a small glass vial. An internal standard (n-nonane, 0.50 g), co-catalyst (MMAO-3A in heptanes, 7.6 wt. % Al) and stir bar were added to the vial. The mixture was stirred for the time and at the temperature indicated in Table 3. The solution was then added to a glass charger containing 400 mL cyclohexane.

### Runs 16-25

Catalyst System Preparation: All experiments were performed under an inert atmosphere in a drybox using solvents dried over molecular sieves. A chromium source, Cr(acac)₃, and PNP-ligand were suspended in ethylbenzene (1.00 g, except for runs 16+17) and cyclohexane in an appropriately sized glass vial. An internal standard (n-nonane, 0.50 g), co-catalyst (MMAO-3A in heptanes, 7.6 wt. % Al) and stir bar were added to the vial. The mixture was stirred for the time and at the temperature indicated in Table 3. The solution was then added to a glass charger containing 400 mL cyclohexane.

All olefin oligomerizations were conducted as follows: The glass charger containing the catalyst system solution and cyclohexane was removed from the drybox and charged into a dry 1L Zipperclave™ reactor heated to 55° C (the reactor was dried by heating under dynamic vacuum for at least 6 h at 110° C). The reactor was then heated to 10° C below the reaction temperature, charged with 172 kPa or 345 kPa (25 psi or 50 psi) hydrogen and the stir impeller was activated. The oligomerization was initiated by slow addition of ethylene over 5 minutes to an operating gauge pressure of 6205 kPa (900 psi) and continuously fed on-demand. The reaction temperature was maintained by the appropriate use of an external heating mantle and internal cooling coils. Upon reaction completion, the reactor was cooled to ambient temperature, depressurized, and the product isolated and analyzed by gas-chromatograph/flame ionization detection (GC-FID).

A summary of the components, parameters, and product analysis for all of the experimental runs is presented in Table 12.

**Table 3**

| Run # | 1 | 2 | 3 | 4 | 5 | 6 | 7 | 8 | 9 |
|---|---|---|---|---|---|---|---|---|---|
| **Catalyst System Composition and Preparation** | | | | | | | | | |
| Ligand | 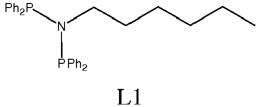 | | | 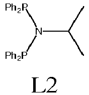 | 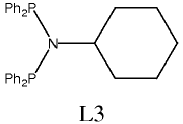 | | 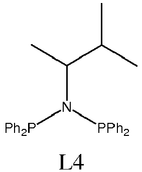 | | |
| Metal Compound | [LCrCl₃]₂ | [LCrCl₃]₂ | [LCrCl₃]₂ | [LCrCl₃]₂ | [LCrCl₃]₂ | [LCrCl₃]₂ | [LCrCl₃]₂ | [LCrCl₃]₂ | [LCrCl₃]₂ |
| Metal Compound (mg) | 6.0 | 7.0 | 6.0 | 7.0 | 9.0 | 4.0 | 5.2 | 5.0 | 5.2 |
| Metal Compound (mmol) | 0.0086 | 0.0100 | 0.0086 | 0.0119 | 0.0154 | 0.0068 | 0.0085 | 0.0081 | 0.0085 |
| Disphosphino aminyl moiety (mg) | - | - | - | - | - | - | - | - | - |
| Co-catalyst | MMAO | MMAO | MMAO | MMAO | MMAO | MMAO | MMAO | MMAO | MMAO |
| Al:Cr molar ratio | 600 | 500 | 600 | 500 | 500 | 500 | 600 | 600 | 600 |
| g EB in pre-activation | 1.00 | 1.00 | 1.00 | 1.00 | 1.00 | 1.00 | 1.00 | 1.00 | 1.00 |
| mL cyclohexane in pre-activation | 0 | 0 | 0 | 0 | 0 | 0 | 0 | 0 | 0 |
| Aging Temperature (°C) | 25 | 25 | 25 | 25 | 25 | 25 | 40 | 25 | 100 |
| Aging Time (hr) | 1 | 1 | 1 | 1 | 0.02 | 0.02 | 0.25 | 1 | 1 |

| **Olefin Oligomerization Conditions** | | | | | | | | | |
|---|---|---|---|---|---|---|---|---|---|
| Bulk Solvent (mL cyclohexane) | 400 | 400 | 400 | 400 | 400 | 400 | 400 | 400 | 400 |
| H₂ gauge Pressure in psi (in kPa) | 50 (344) | 50(344) | 50 (344) | 50 (344) | 50 (344) | 50 (344) | 50 (344) | 50 (344) | 50 (344) |
| Ethylene gauge Pressure in psi (in MPa)) | 850 (5.9) | 850 (5.9) | 850 (5.9) | 850 (5.9) | 850 (5.9) | 850 (5.9) | 850 (5.9) | 850 (5.9) | 850 (5.9) |
| Reaction Temperature (°C) | 70 | 90 | 110 | 70 | 70 | 70 | 70 | 70 | 70 |

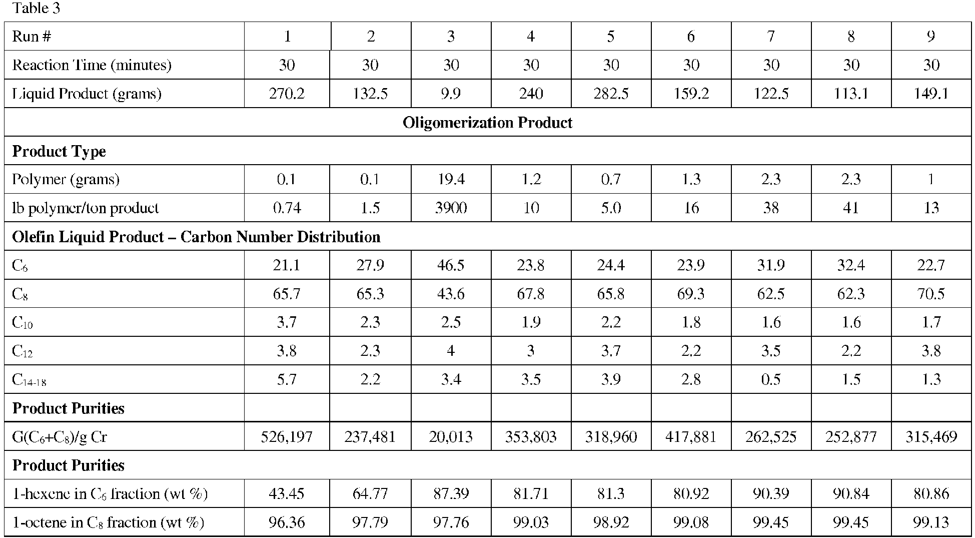

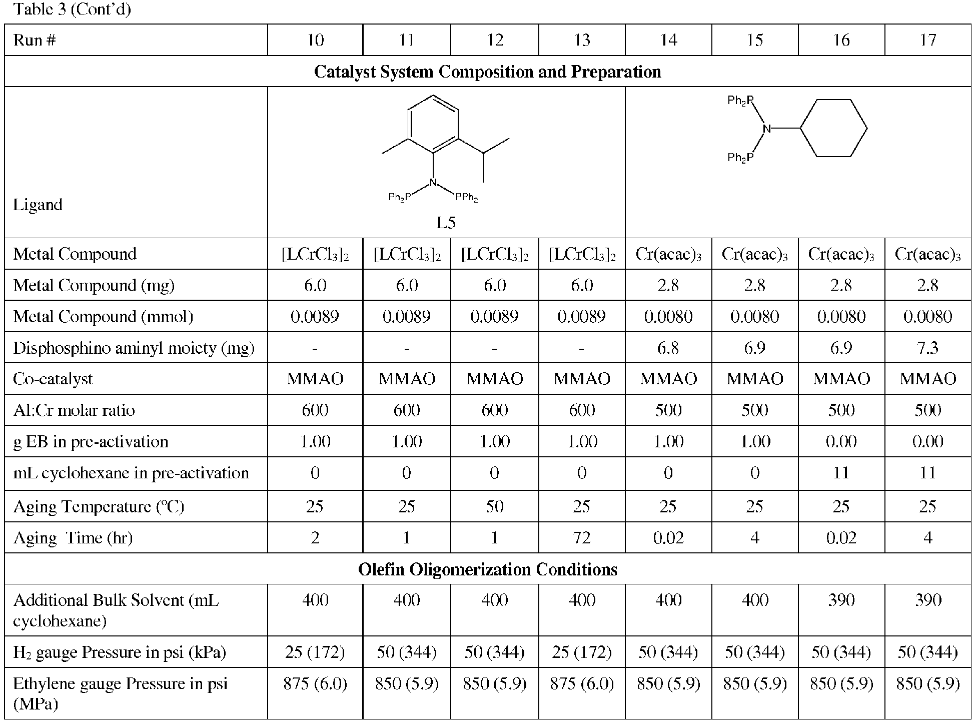

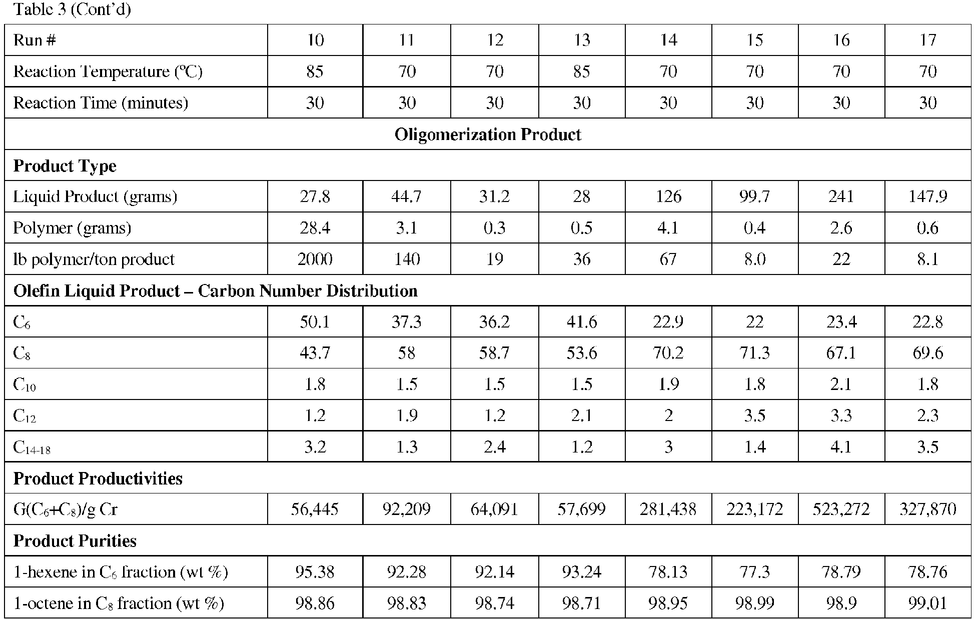

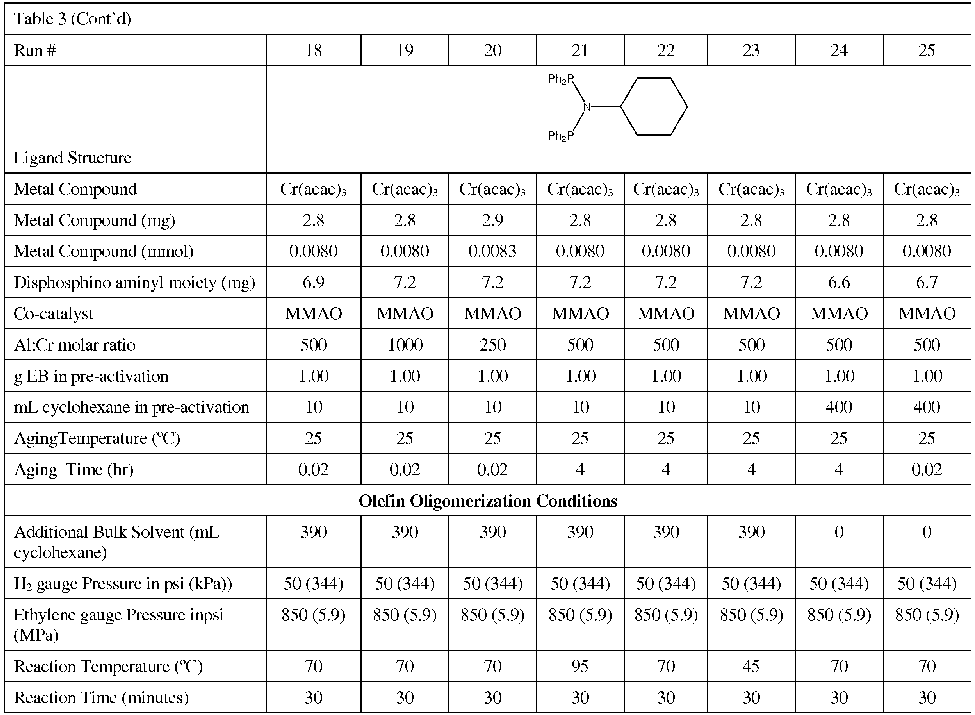

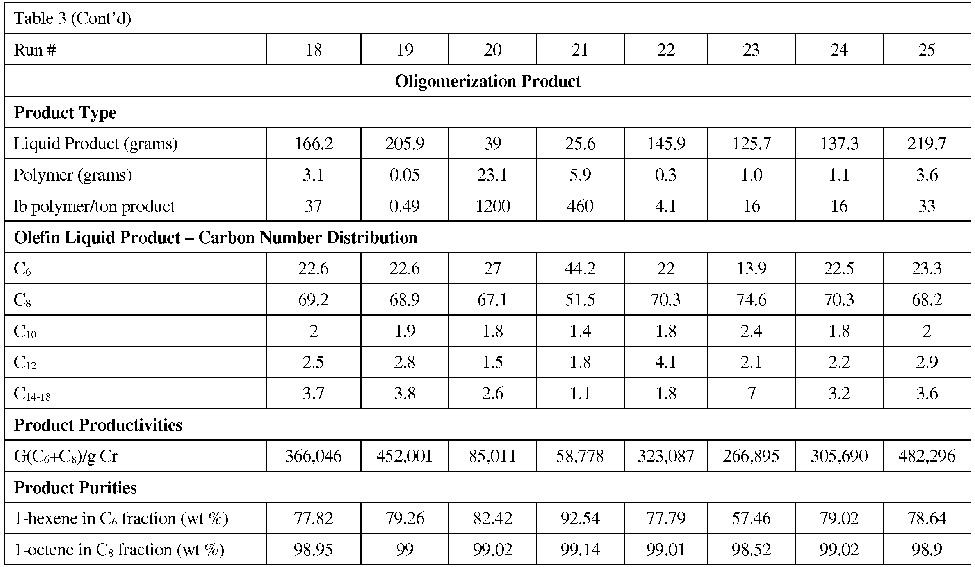

The effect of the nature of the metal compound on the catalyst system was investigated. Specifically, an oligomerization catalyst system was prepared using either Cr(acac)₃ and PNP ligand L3 (Run # 14) or LCrCl₃ where L was L4 as the metal compound (Run #6). The aged catalyst system components were utilized in the ethylene oligomerization. Selected results relating to the ethylene oligomerization product are presented in Table 4.

**Table 4**

| Chromium Source | Cr(acac)₃ | [LCrCl₃]₂ |
|---|---|---|
| 1-Hexene in the C₆ Fraction | 78.13 | 80.92 |
| 1-octene in the C₈ Fraction | 98.95 | 99.08 |
| g(C₆+C₈)/g Cr | 281,438 | 417,881 |
| %C₆ | 22.9 | 23.9 |
| %C₈ | 70.2 | 69.3 |
| lb polymer/ton liquid | 67 | 16 |

The data of Table 4 indicates subtle differences between the catalyst systems investigated. Particularly, the catalyst system using CrCl₃ (Run #6) has higher productivties and produces less polymer than the catalyst system using Cr(acac)₃ (Run #14). Since the diphosphino aminyl ligand is the same, the results appear to indicate that the the catalyst system using CrCl₃ may activate more rapidly than the catalyst system using Cr(acac)₃ (Run #14).

The effect of temperature on the aging process was investigated. Catalyst systems utilizing CrCl₃ as the metal compound and the diphosphino aminyl ligand L4 or L5 were prepared as described. The central nitrogen atom of the diphosphino aminyl ligand was substituted with CHMeⁱPr or 2-Me-6ⁱPrPh and corresponding to ligands L4 and L5 respectively. Table 5 provides the amount polymer produced (lb polymer/ton liquid), the catalyst system productivity (gC₆+C₈) )/g Cr, and the 1-hexene purity (1-C₆ purity) for each of the catalyst systems utilized in the oligomerization reaction for the catalyst system comprising ligand L4 (Run # 7-9) or the catalyst system comprising ligand L5 (run # 11-12). The results demonstrate the catalyst productivity can increase as the aging temperature increases for the catalyst system comprising ligand L4 with a concomitant reduction in polymer formation. Additionally, 1-hexene purity decreased significantly at the highest temperature (100°C). For the catalyst system comprising ligand L5, doubling the aging temperature resulted in a marked reduction in polymer formation with little change in 1-hexene purity. However, there was a decrease in catalyst productivity observed with the increased aging temperature. These results demonstrate that catalyst system aging times can be shortened by increasing the catalyst system aging temperature.

**Table 5**

| Run No. | 7 | 8 | 9 | 11 | 12 |
|---|---|---|---|---|---|
| Aging time (h) | 0.25 | 1 | 1 | 1 | 1 |
| Aging temperature | 40 | 25 | 100 | 25 | 50 |
| lb polymer/ton liquid | 38 | 41 | 13 | 140 | 19 |
| g(C₆+Cg)/g Cr | 262,525 | 252,877 | 315,469 | 92,209 | 64,091 |
| 1-Hexene in the C₆ fraction | 90.39 | 90.84 | 80.86 | 92.28 | 92.14 |

The effect of increasing the steric bulk on the central nitrogen atom of the PNP ligand on the aging process was investigated. Catalyst systems utilizing CrCl₃ as the metal compound and a diphosphino aminyl ligands L1, L3, L4 and L5 were prepared as described. The central nitrogen atom of the diphosphino aminyl ligand was substituted with n-hexyl, iPr, CHMeⁱPr or 2-Me-6ⁱPrPh corresponding to ligands L1, L3, L4 and L5 respectively. Table 6 provides the amount of polymer produced (lb polymer/ton liquid), catalyst system productivity (gC₆+C₈) )/g Cr, percentage of hexene and octene produced (%C₆, %C₈), 1-hexene purity (1-C₆ purity), and 1-octene purity (1-C₈ purity) for each the oligomerization reaction runs.

**Table 6**

| Run # | 1 | 4 | 8 | 11 |
|---|---|---|---|---|
| lb polymer/ton liquid | 0.74 | 10 | 41 | 140 |
| g(C₆+C₈)/g Cr | 526,197 | 353,803 | 252,877 | 92,209 |
| %C₆ | 21.1 | 23.8 | 32.4 | 37.3 |
| %C₈ | 65.7 | 67.8 | 62.3 | 58.0 |
| 1-Hexene in the C₆ Fraction | 43.45 | 81.71 | 90.84 | 92.28 |
| 1-octene in the C₈ Fraction | 96.36 | 99.03 | 99.45 | 98.83 |
| Oligomerization Temp (°C) | 70 | 70 | 70 | 70 |

The results demonstrate that catalyst systems comprising bulkier groups attached to the aminyl nitrogen atom of the phosphino aminyl ligand when subjected to aging produced 1-hexene and 1-octene products of increased purity. For example, 1-hexene purity was increased from 43.5% to 92.3% when going from a catalyst system comprising a PNP ligand substituted with n-hexyl (Run #1) to one substituted with the bulkier 2-Me-6-ⁱPrPh group (Run # 11). The results also demonstrate that as the substitutents on the central nitrogen of the diphosphino aminyl ligand increased in steric bulk, the catalyst systems comprising the ligand displayed a concomitant decrease in the productivity and increase in polymer formation.

The effect of the ratio of components on the aging process was investigated. Catalyst systems utilizing the metal compound [Cr(acac)₃]₂ and the phosphino aminyl ligand L3 were prepared as described. Table 7 provides the amount of polymer produced (lb polymer/ton liquid), catalyst system productivity (gC₆+C₈)/g Cr, percentage of hexene and octene produced (%C₆, %C₈), 1-hexene purity (1-C₆ purity), and 1-octene purity (1-C₈ purity) for each of the oligomerization reaction runs. The results demonstrate a decrease in polymer production and increased catalyst system productivity as the Al/Cr ratio in the catalyst system was increased. The %C6, %C8, 1-octene and 1-hxene purity appeared similar at all Al/Cr ratios employed.

**Table 7**

| Run # | 20 | 18 | 19 |
|---|---|---|---|
| Al/Cr | 250 | 500 | 1000 |
| lb polymer/ton liquid | 1,299 | 37 | 0.49 |
| g(C₆+C₈)/g Cr | 85,011 | 366,046 | 452,001 |
| %C₆ | 27.0 | 22.6 | 22.6 |
| %C₈ | 67.1 | 69.2 | 68.9 |
| 1-Hexene in the C₆ Fraction | 82.42 | 77.82 | 79.26 |
| 1-octene in the C₈ Fraction | 99.02 | 98.95 | 99 |

The effect of the amount of metal compound utilized in aging the catalyst system was investigated. Catalyst systems utilizing the metal compound CrCl₃ and the diphosphino aminyl ligand L3 were prepared as described. Run# 6 contained 0.4 mg of Cr while Run# 5 contained 0.8 mg of Cr. Table 8 provides the amount of polymer produced (lb polymer/ton liquid), catalyst system productivity (gC₆+C₈)/g Cr, percentage of hexene and octene produced (%C₆, %C₈), 1-hexene purity (1-C₆ purity), and 1-octene purity (1-C₈ purity) for each of the oligomerization reaction runs. Refering to Table 8, it was observed that increasing the amount of metal compound present during the aging decreased the amount of polymer formed. However, the increase in the amount of metal compound present during aging also resulted in a decrease in the catalyst system productivity. The results demonstrate that the chromium loading can be optimized to balance high catalyst productivity with low polymer production.

The effect of the amount of solvent present in the catalys system aging process was investigated. Catalyst systems utilizing the metal compound CrCl₃ and the diphosphino aminyl ligand L3 were prepared as described. Table 9 provides the amount of polymer produced (lb polymer/ton liquid), catalyst system productivity (gC₆+C₈)/g Cr, percentage of hexene and octene produced (%C₆, %Cg), 1-hexene purity (1-C₆ purity), and 1-octene purity (1-C₈ purity) for each of the ratio of components utilized in the oligomerization reaction. EB stands for ethylbenzene. The results demonstrate a general increase in catalyst system productivity with increasing amounts of cyclohexane and a reduction in polymer production. The absence of ethylbenezene produced the largest increase in catalyst system productivity.

**Table 8**

| Run # | 6 | 5 |
|---|---|---|
| lb polymer/ton liquid | 16 | 5.0 |
| g(C₆+C₈)/g Cr | 417,881 | 318,960 |
| %C₆ | 23.9 | 24.4 |
| %C₈ | 69.3 | 65.8 |
| 1-Hexene in the C₆ Fraction | 80.92 | 81.3 |
| 1-octene in the C₈ Fraction | 99.08 | 98.92 |

**Table 9**

| Run# | 14 | 18 | 16 | 25 |
|---|---|---|---|---|
| Cyclohexane dilution (mL) | 0 | 10 | 11 | 400 |
| EB(g) | 1 | 1 | 0 | 1 |
| lb polymer/ton liquid | 67 | 37 | 22 | 33 |
| g(C₆+C₈)/g Cr | 281,438 | 366,046 | 523,272 | 482,296 |

The experiment was repeated with the exception that the catalyst system aging time was extended to 4 hours. The results of these experiments carried out using an extended aging time are presented in Table 10. Table 10 provides the amount of polymer produced (lb polymer/ton liquid), catalyst system productivity (gC₆+C₈)/g Cr, percentage of hexene and octene produced (%C₆, %C₈), 1-hexene purity (1-C₆ purity), and 1-octene purity (1-C₈ purity) for each of the oligomerization reaction runs. The results demonstrate that when the aging time is increased from 1 minute to 4 hours, the addition of cyclohexane again results in an increase in catalyst system productivity. However; in contrast to the results observed when using an aging time of 1 minute, polymer production was observed to increase. Further, the presence of EB appears to have little effect on the parameters investigated.

**Table 10**

| Run# | 15 | 22 | 17 | 24 |
|---|---|---|---|---|
| Cyclohexane dilution (mL) | 0 | 10 | 11 | 400 |
| EB | 1 | 1 | 0 | 1 |
| Lb polymer/ton liquid | 8.0 | 4.1 | 8.1 | 16 |
| G(C₆+C₈)/g Cr | 223,172 | 323,087 | 327,870 | 305,690 |

The effect of oligomerization temperature on 1-hexene purity was investigated. Catalyst systems comprising the metal compounds a diphosphino aminyl ligands as indicated in Table 11 were prepared as described. Table 11 provides the amount of polymer produced (lb polymer/ton liquid), catalyst system productivity (gC₆+C₈)/g Cr, percentage of hexene and octene produced (%C₆, %C₈), 1-hexene purity (1-C₆ purity), and 1-octene purity (1-C₈ purity) for each of the oligomerization reaction runs. As shown in Table 9, L3, increasing the oligomerization temperature from 45° C to 70° C improved 1-hexene purity by 35%; the catalyst system utilizing L1 as the diphosphino aminyl ligand saw an increase in 1-hexene purity of 43.9% when the temperature was changed from 70° C to 110° C. The catalyst system utilizing L5 as the diphosphino aminyl ligand saw an increase in 1-hexene purity of 1.1% when the temperature was changed from 70° C to 85° C. The 1-hexene purity as a function of diphosphino aminyl ligand is presented in Figure 1.

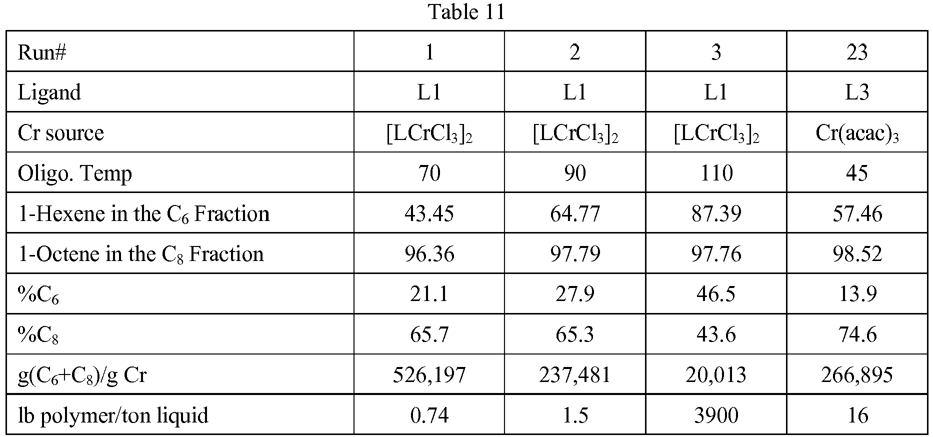

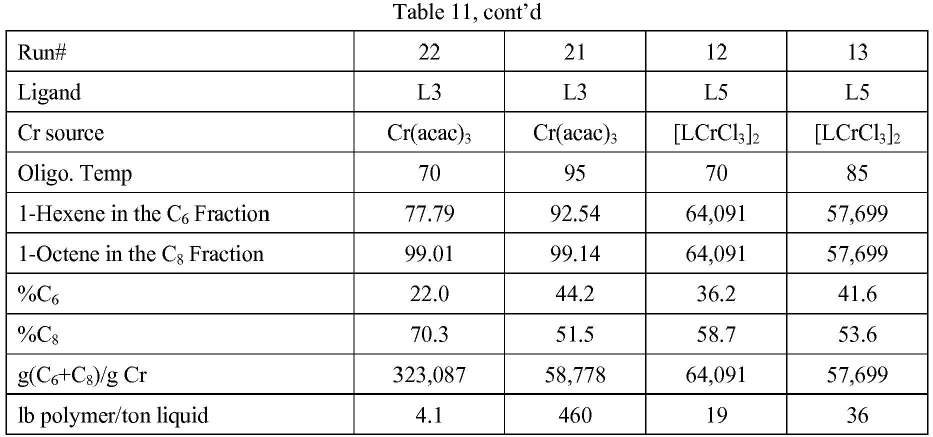

The effect of the duration of catalyst system aging on catalyst system olefin oligomeriation performance was investigated. Catalyst systems utilizing the metal compound and the diphosphino aminyl ligand, as indicated in Table 3 were prepared as described. Table 12 provides the amount of polymer produced (lb polymer/ton liquid), catalyst system productivity (gC₆+C₈)g Cr, percentage of hexene and octene produced (%C₆, %C₈), 1-hexene purity (1-C₆ purity), and 1-octene purity (1-C₈ purity) for each of the ratio of components utilized in the oligomerization reaction.

**Table 12**

| Run # | 14 | 15 | 10 | 13 |
|---|---|---|---|---|
| Cr source | Cr(acac)3 | Cr(acac)3 | [LCrCl3]2 | [LCrCl3]2 |
| Aging Time (h) | 0.02 | 4 | 2 | 72 |
| lb polymer/ ton liquid | 67 | 8.0 | 2000 | 36 |
| G(C6+C8)/g Cr | 281,438 | 223,172 | 56,445 | 57,699 |
| Oligomerization Temperature (°C) | 70 | 70 | 85 | 85 |

The results demonstrate increasing the duration of catalyst system aging resulted in a dramatic decrease in the amount of polymer formed. The results for the catalyst system utilizing the L5 ligand are particularly striking as the increase in aging duration from 2 hours to 72 hours resulted in a 98% decrease in polymer formation.

Without further elaboration, it is believed that one skilled in the art can, using the description herein, utilize the present invention to its fullest extent. While preferred embodiments of the invention have been shown and described, modifications thereof can be made by one skilled in the art without departing from the scope of the claims. The embodiments and examples described herein are exemplary only, and are not intended to be limiting. Many variations and modifications of the invention disclosed herein are possible and are within the scope of the invention. Where numerical ranges or limitations are expressly stated, such express ranges or limitations should be understood to include iterative ranges or limitations of like magnitude falling within the expressly stated ranges or limitations (e.g., from about 1 to about 10 includes, 2, 3, 4, etc.; greater than 0.10 includes 0.11, 0.12, 0.13, etc.). Use of the term "optionally" with respect to any element of a claim is intended to mean that the subject element is required, or alternatively, is not required. Both alternatives are intended to be within the scope of the claim. Use of broader terms such as comprises, includes, having, etc. should be understood to provide support for narrower terms such as consisting of, consisting essentially of, comprised substantially of, etc.

Accordingly, the scope of protection is not limited by the description set out above but is only limited by the claims which follow. Each and every claim is incorporated into the specification as an embodiment of the present invention. Thus, the claims are a further description and are an addition to the preferred embodiments of the present invention.

## Claims

1. An olefin oligomerization process comprising: a) contacting i) a metal compound, ii) a diphosphino aminyl ligand, and iii) a metal alkyl comprising an aluminoxane to form a mixture; b) aging the mixture for at least 20 minutes in the substantial absence of an olefin monomer to form an aged mixture; c) contacting the aged mixture with an olefin monomer; and d) forming an olefin oligomer product under conditions suitable for forming an olefin oligomer product, wherein "substantial absence of an olefin monomer" means a molar ratio of olefin monomer to the metal compound up to 5:1, and wherein the mixture consists essentially of the metal compound, the diphosphino aminyl ligand, the metal alkyl comprising an aluminoxane and optionally a hydrocarbon solvent.

2. The process of claim 1, wherein the mixture is aged for at least 30 minutes, or for at least 40 minutes.

3. The process of claim 1, wherein the conditions suitable for forming an olefin oligomer product comprise a temperature that is increased when compared to an otherwise similar process wherein the mixture is aged in the presence of an olefin monomer.

4. The process of claim 1, wherein the olefin oligomer product has a polymer amount that is reduced when compared to an otherwise similar process wherein the mixture is aged in the presence of an olefin monomer.

5. The process of claim 1, wherein the diphosphino aminyl ligand comprises one diphosphino aminyl moiety.

6. The process of claim 1, wherein the the metal compound is complexed to the diphosphino aminyl ligand comprising at least two diphosphino aminyl moieties and a linking group linking each aminyl nitrogen atom of the diphosphino aminyl moieties.

7. The process of claim 6, wherein the linking group comprises at least one cyclic group, and the aminyl nitrogen atoms of the two diphosphino aminyl moieties are attached to a ring carbon of the linking group.

8. A process for reducing an amount of polymer produced in an olefin oligomerization process comprising:
a) contacting a metal compound, a diphosphino aminyl ligand, and a metal alkyl comprising an aluminoxane to form a mixture;
b) aging the mixture in the substantial absence of an olefin monomer for at least 20 minutes to form a aged mixture;
c) contacting the aged mixture with an olefin monomer; and
d) forming an olefin oligomer product wherein the olefin oligomer product has a reduced polymer formation when compared to an otherwise similar process wherein the mixture is aged in the presence of the olefin monomer;
wherein "substantial absence of an olefin monomer" means a molar ratio of olefin monomer to the metal compound up to 5:1; and
wherein the mixture consists essentially of the metal compound, the diphosphino aminyl ligand, the metal alkyl comprising an aluminoxane and optionally a hydrocarbon solvent.

9. The process of claim 1 or 8, wherein the mixture is aged at a temperature from 10 °C to 130 °C.

10. The process of claim 1 or 8, wherein the metal compound comprises chromium.

11. The process of claim 1 or 8, wherein the metal compound is a chromium(III) chloride or chromium(III) acetylacetonate.

12. The process of claim 1 or 8, wherein the metal alkyl and metal compound are present in a molar ratio amount of from about 50 to about 1000.

13. The process of claim 1 or 8, wherein the olefin comprises ethylene and a liquid olefin oligomer product comprises comprises at least 60 wt. % C₆ and C₈ olefins.

14. The process of claim 13, wherein the C₆ olefins comprises greater than or equal to 90 wt. % 1-hexene and the C₈ olefins comprises greater than or equal to 97 wt. % 1-octene.

15. The process of claim 1 or 8, wherein the process produces a C₆ olefin fraction containing more 1-hexene than when compared to an otherwise similar process wherein the mixture is aged in the presence of the olefin monomer.

16. A process for preparing a catalyst system comprising: a) forming a catalyst system mixture consisting essentially of i) a metal compound, ii) a diphosphino aminyl ligand, iii) a metal alkyl comprising an aluminoxane, and optionally (iv) a hydrocarbon solvent; b) aging the catalyst system mixture for at least 20 minutes in the substantial absence of an olefin monomer, wherein "substantial absence of an olefin monomer" means a molar ratio of olefin monomer to the metal compound up to 5:1.

17. The process of claim 16, wherein the catalyst system mixture is aged at a temperature from 10 °C to 130 °C.

18. The use of a catalyst system prepared in accordance with claim 16 or 17, consisting essentially of
i) a metal compound, ii) a diphosphino aminyl ligand, iii) a metal alkyl comprising an aluminoxane, and optionally iv) a hydrocarbon solvent, as an olefin oligomerization catalyst.

## Patentansprüche

1. Olefinoligomerisierungsvorgang, Folgendes umfassend: a) Inberührungbringen i) einer Metallverbindung, ii) eines Diphosphinoaminylliganden und iii) eines ein Aluminoxan umfassenden Metallalkyls, um ein Gemisch auszubilden; b) Altern des Gemischs über wenigstens 20 Minuten bei wesentlicher Abwesenheit eines Olefinmonomers, um ein gealtertes Gemisch auszubilden; c) Inberührungbringen des gealterten Gemischs mit einem Olefinmonomer und d) Ausbilden eines Oligomerprodukts unter Bedingungen, die geeignet sind, ein Olefinoligomerprodukt auszubilden, wobei "wesentliche Abwesenheit eines Olefinmonomers" ein Molverhältnis zwischen Olefinmolomer und Metallverbindung von bis zu 5:1 beschreibt und wobei das Gemisch im Wesentlichen aus der Metallverbindung, dem Diphosphinoaminylliganden, dem ein Aluminoxan umfassenden Metallalkyl und optional einem Kohlenwasserstofflösungsmittel besteht.

2. Vorgang nach Anspruch 1, wobei das Gemisch über wenigstens 30 Minuten oder über wenigstens 40 Minuten gealtert wird.

3. Vorgang nach Anspruch 1, wobei die Bedingungen, die zum Ausbilden eines Olefinoligomerprodukts geeignet sind, eine Temperatur umfassen, die im Vergleich zu einem ansonsten ähnlichen Vorgang, bei dem das Gemisch in der Gegenwart eines Olefinmonomers gealtert wird, höher ist.

4. Vorgang nach Anspruch 1, wobei das Olefinoligomerprodukt eine Polymermenge umfasst, die im Vergleich zu einem ansonsten ähnlichen Vorgang, bei dem das Gemisch in der Gegenwart eines Olefinmonomers gealtert wird, geringer ist.

5. Vorgang nach Anspruch 1, wobei der Diphosphinoaminylligand eine Diphosphinoaminylgruppe umfasst.

6. Vorgang nach Anspruch 1, wobei die Metallverbindung an den Diphosphinoaminylliganden, der wenigstens zwei Diphosphinoaminylgruppen und eine Bindungsgruppe umfasst, die jedes Aminylstickstoffatom der Diphosphinoaminylgruppen verbindet, komplexiert ist.

7. Vorgang nach Anspruch 6, wobei die Bindungsgruppe wenigstens eine zyklische Gruppe umfasst und die Aminylstickstoffatome der zwei Diphosphinoaminylgruppen an ein Ringkohlenstoffatom der Bindungsgruppe gebunden sind.

8. Vorgang zum Verringern einer Menge von in einem Olefinoligomerisierungsvorgang hergestelltem Polymer, Folgendes umfassend:
a) Inberührungbringen einer Metallverbindung, eines Diphosphinoaminylliganden und eines ein Aluminoxan umfassenden Metallalkyls, um ein Gemisch auszubilden;
b) Altern des Gemischs bei wesentlicher Abwesenheit eines Olefinmonomers über wenigstens 20 Minuten, um ein gealtertes Gemisch auszubilden;
c) Inberührungbringen des gealterten Gemischs mit einem Olefinmonomer und
d) Ausbilden eines Olefinoligomerprodukts, wobei das Olefinoligomerprodukt im Vergleich zu einem ansonsten ähnlichen Vorgang, bei dem das Gemisch in Gegenwart des Olefinmonomers gealtert wird, eine verringerte Polymerbildung aufweist;
wobei "wesentliche Abwesenheit eines Olefinmonomers" ein Molverhältnis zwischen Olefinmolomer und Metallverbindung von bis zu 5:1 beschreibt und
wobei das Gemisch im Wesentlichen aus der Metallverbindung, dem Diphosphinoaminylliganden, dem ein Aluminoxan umfassenden Metallalkyl und optional einem Kohlenwasserstofflösungsmittel besteht.

9. Vorgang nach Anspruch 1 oder 8, wobei das Gemisch bei einer Temperatur von 10 °C bis 130 °C gealtert wird.

10. Vorgang nach Anspruch 1 oder 8, wobei die Metallverbindung Chrom umfasst.

11. Vorgang nach Anspruch 1 oder 8, wobei die Metallverbindung Chrom(III)chlorid oder Chrom(III)acetylacetonat ist.

12. Vorgang nach Anspruch 1 oder 8, wobei das Metallalkyl und die Metallverbindung in einem Molverhältnis von etwa 50 bis etwa 1000 vorliegen.

13. Vorgang nach Anspruch 1 oder 8, wobei das Olefin Ethylen umfasst und ein flüssiges Olefinoligomerprodukt wenigstens 60 Gew.-% C₆- und C₈-Olefine umfasst.

14. Vorgang nach Anspruch 13, wobei die C₆-Olefine wenigstens 90 Gew.-% 1-Hexen umfassen und die C₈-Olefin wenigstens 97 Gew.-% 1-Octen umfassen.

15. Vorgang nach Anspruch 1 oder 8, wobei der Vorgang eine C₆-Olefinfraktion erzeugt, die im Vergleich zu einem ansonsten ähnlichen Vorgang, bei dem das Gemisch in Anwesenheit des Olefinmonomers gealtert wird, mehr 1-Hexen enthält.

16. Vorgang zum Herstellen eines Katalysatorsystems, Folgendes umfassend: a) Ausbilden eines Katalysatorsystemgemischs, das im Wesentlichen aus i) einer Metallverbindung, ii) einem Diphosphinoaminylliganden, iii) einem ein Aluminoxan umfassenden Metallalkyl und optional iv) einem Kohlenwasserstofflösungsmittel besteht; b) Altern des Katalysatorsystemgemischs über wenigstens 20 Minuten bei wesentlicher Abwesenheit eines Olefinmonomers, wobei "wesentliche Abwesenheit eines Olefinmonomers" ein Molverhältnis zwischen Olefinmolomer und Metallverbindung von bis zu 5:1 beschreibt.

17. Vorgang nach Anspruch 16, wobei das Katalysatorsystemgemisch bei einer Temperatur von 10 °C bis 130 °C gealtert wird.

18. Gebrauch eines Katalysatorsystems, hergestellt nach Anspruch 16 oder 17, im Wesentlichen bestehend aus i) einer Metallverbindung, ii) einem Diphosphinoaminylliganden, iii) einem ein Aluminoxan umfassenden Metallalkyl und optional iv) einem Kohlenwasserstofflösungsmittel als ein Olefinoligomerisierungskatalysator.

## Revendications

1. Procédé d'oligomérisation d'oléfine comprenant :
a) la mise en contact
i) d'un sel métallique,
ii) d'un ligand diphosphino aminyle, et
iii) d'un alkylmétal comprenant un aluminoxane pour former un mélange ;
b) le vieillissement du mélange pendant au moins 20 minutes essentiellement en l'absence de monomère oléfine pour former un mélange vieilli ;
c) la mise en contact du mélange vieilli avec un monomère oléfine ; et
d) la formation d'un produit oligomère d'oléfine dans des conditions adéquates pour former un produit oligomère d'oléfine, où "essentiellement en l'absence de monomère oléfine" signifie un rapport molaire du monomère oléfine au sel métallique de jusqu'à 5:1, et où le mélange consiste essentiellement en le sel métallique, le ligand diphosphino aminyle, l'alkylmétal comprenant un aluminoxane et facultativement un solvant hydrocarbure.

2. Procédé selon la revendication 1, dans lequel le mélange est vieilli pendant au moins 30 minutes, ou pendant au moins 40 minutes.

3. Procédé selon la revendication 1, dans lequel les conditions adéquates pour former un produit oligomère d'oléfine comprennent une température augmentée par rapport à un procédé autrement similaire, dans lequel le mélange est vieilli en présence d'un monomère oléfine.

4. Procédé selon la revendication 1, dans lequel le produit oligomère d'oléfine a une quantité de polymère réduite par rapport à un procédé autrement similaire, dans lequel le mélange est vieilli en présence d'un monomère oléfine.

5. Procédé selon la revendication 1, dans lequel le ligand diphosphino aminyle comprend une entité diphosphino aminyle.

6. Procédé selon la revendication 1, dans lequel le sel métallique est complexé au ligand diphosphino aminyle comprenant au moins deux entités diphosphino aminyles et un groupe de liaison liant chaque atome d'azote des aminyles aux entités diphosphino aminyles.

7. Procédé selon la revendication 6, dans lequel le groupe de liaison comprend au moins groupe cyclique, et les atomes d'azote du groupe aminyle des deux entités diphosphino aminyles sont attachés à un carbone du cycle du groupe de liaison.

8. Procédé de réduction d'une quantité de polymère produite dans un procédé d'oligomérisation d'oléfine, comprenant :
a) la mise en contact d'un sel métallique, d'un ligand diphosphino aminyle et un alkylmétal comprenant un aluminoxane pour former un mélange ;
b) le vieillissement du mélange essentiellement en l'absence de monomère oléfine pendant au moins 20 minutes pour former un mélange vieilli ;
c) la mise en contact du mélange vieilli avec un monomère oléfine ; et
d) la formation d'un produit oligomère oléfine, le produit oligomère oléfine ayant une formation réduite de polymère par rapport à un procédé similaire dans lequel le mélange est vieilli en présence du monomère oléfine ;
où "essentiellement en l'absence de monomère oléfine" signifie un rapport molaire du monomère oléfine au sel métallique de jusqu'à 5:1 ;
où le mélange consiste essentiellement en le sel métallique, le ligand diphosphino aminyle, l'alkylmétal comprenant un aluminoxane et facultativement un solvant hydrocarbure.

9. Procédé selon la revendication 1 ou 8, dans lequel le mélange est vieilli à une température entre 10°C et 130°C.

10. Procédé selon la revendication 1 ou 8, dans lequel le sel métallique comprend du chrome.

11. Procédé selon la revendication 1 ou 8, dans lequel le sel métallique est un chlorure de chrome (III) ou un acétylacétonate de chrome (III).

12. Procédé selon la revendication 1 ou 8, dans lequel l'alkylmétal et le sel métallique sont présents dans un rapport molaire d'environ 50 à environ 1000.

13. Procédé selon la revendication 1 ou 8, dans lequel l'oléfine comprend de l'éthylène et un produit oligomère oléfine liquide comprend au moins 60% en poids d'oléfines en C₆ et C₈.

14. Procédé selon la revendication 13, dans lequel les oléfines en C₆ comprennent au moins 90% en poids d'hex-1-ène et les oléfines en C₈ comprennent au moins 97% en poids d'oct-1-ène.

15. Procédé selon la revendication 1 ou 8, dans lequel le procédé produit une fraction d'oléfines en C₆ contenant davantage d'hex-1-ène que par rapport à d'autres procédés similaires dans lesquels le mélange est vieilli en présence du monomère oléfine.

16. Procédé de préparation d'un système catalytique comprenant :
a) la formation d'un mélange système catalytique consistant essentiellement en
i) un sel métallique,
ii) un ligand diphosphino aminyle,
iii) un alkylmétal comprenant un aluminoxane, et facultativement (iv) un solvant hydrocarbure ;
b) le vieillissement du mélange pendant au moins 20 minutes essentiellement en l'absence de monomère oléfine, où "essentiellement en l'absence de monomère oléfine" signifie un rapport molaire du monomère oléfine au sel métallique de jusqu'à 5:1.

17. Procédé selon la revendication 16, dans lequel le mélange système catalytique est vieilli à une température entre 10°C et 130°C.

18. Utilisation d'un système catalytique préparé selon la revendication 16 ou 17, consistant essentiellement en
i) un sel métallique,
ii) un ligand diphosphino aminyle,
iii) un alkylmétal comprenant un aluminoxane, et facultativement
iv) un solvant hydrocarbure,
comme catalyseur d'oligomérisation d'oléfines.
